# EUROPEAN PATENT SPECIFICATION

(11) **EP 0 986 401 B1**
(45) Date of publication and mention of the grant of the patent: **25.02.2004**
(21) Application number: 98937941.7
(22) Date of filing: 29.05.1998
(51) Int. Cl.: A61K 41/00, A61K 47/48, A61K 48/00, A61K 51/04, A61K 51/06, A61K 51/10

(54) **P-SELECTIN TRANSLOCATION TO VASCULAR EPITHELIAL LUMEN BY IONIZING RADIATION**
P-SELECTIN TRANSLOKATION INS VASKULARE EPITHELIALE LUMEN DURCH IONISIERENDE STRAHLUNG
TRANSLOCATION DE P-SELECTINE DANS LA LUMIERE VASCULAIRE EPITHELIALE PAR RAYONNEMENT IONISANT

(30) Priority: 30.05.1997 US 48141 P
(43) Date of publication of application: 22.03.2000
(73) Proprietor: ARCH DEVELOPMENT CORPORATION, Chicago, Illinois 60637 (US)
(72) Inventor: HALLAHAN, Dennis, E., Nashville, TN 37215 (US); VIRUDACHALAM, Subbulakshmi, Granite Bay, CA 95746 (US)
(74) Representative: Gowshall, Jonathan Vallance
(86) International application number: PCT/US1998/010913
(87) International publication number: WO 1998/053852

(56) References cited:
- WO-A-96/25947
- BARKER J N: "Adhesion molecules in cutaneous inflammation." CIBA FOUNDATION SYMPOSIUM, (1995) 189 91-101;DISCUSSION 101-6. REF: 38 JOURNAL CODE: D7X. ISSN: 0300-5208., XP002079448 Netherlands
- DULKANCHAINUN T S ET AL: "Reduction of hepatic ischemia/reperfusion injury by a soluble P - selectin glycoprotein ligand-1." ANNALS OF SURGERY, (1998 JUN) 227 (6) 832-40. JOURNAL CODE: 67S. ISSN: 0003-4932., XP002079449 United States
- BERG E L ET AL: "Antibodies cross-reactive with E-and P-selectin block both E-and P-selectin functions" BLOOD, vol. 85, no. 1, 1 January 1995, pages 31-37, XP000601489
- SLUITER ET AL.: "Leukocyte adhesion molecules on the vascular endothelium: their role in the pathogenesis of cardiovascular disease and the mechanisms underlying their expression" J. CARDIOVASC. PHARMACOL., vol. 22, no. 4, 1993, pages S37-S44, XP002079450
- MCEVER R P: "GMP - 140: a receptor for neutrophils and monocytes on activated platelets and endothelium." JOURNAL OF CELLULAR BIOCHEMISTRY, (1991 FEB) 45 (2) 156-61. REF: 36 JOURNAL CODE: HNF. ISSN: 0730-2312., XP002079451 United States

## Description

### BACKGROUND OF THE INVENTION

### 1. Field of the Invention

The present invention relates generally to the fields of pulmonary pathology and to radiation biology. More particularly, it provides a variety of compositions and methods for use in reducing radiation injury in lung tissue, particularly relating to the expression of P-selectin and the induction of inflammatory cell response to radiation.

### 2. Description of the Related Art

Although significant effort continues to be applied to the development of effective anti-cancer strategies, many prevalent forms of human cancer still resist effective chemotherapeutic intervention. A considerable underlying problem that must be addressed in any treatment regimen is the concept of "total cell kill." This is based on the fact that in order to have an effective treatment regimen, whether it be a surgical or chemotherapeutic approach, or both, all of the so-called "clonogenic" malignant cells must be killed to prevent regrowth of the tumor mass.

Due to the need to develop therapeutic agents and regimens capable of achieving such total cell kill, certain types of tumors have been more amenable than others to therapy. For example, lymphomas, and tumors of the blood and blood-forming organs, *e.g*., leukemias, have generally been more responsive to chemotherapeutic therapy, while solid tumors, such as carcinomas, generally prove more resistant to such therapies.

One underlying reason for this phenomenon is that blood-based tumors are physically more accessible to the chemotherapeutic agents, whereas it is often difficult for most chemotherapeutic agents to reach all of the cells of a solid tumor. Increasing the dose of chemotherapeutic agents, rather than achieving the desired total cell kill, most often results in toxic side effects that limit the effectiveness the chemotherapy.

Even immunotoxins, that are directed to selected cancer cell antigens, have proven to be of limited use in the treatment of solid tumors (Weiner *et al.,* 1989; Byers *et al.*, 1989). One reason for this is that solid tumors are generally impermeable to antibody-sized molecules, often exhibiting specific uptake values of less than 0.001% of the injected dose/g of tumor in human studies (Sands *et al.,* 1988; Epenetos *et al.,* 1986).

Further significant problems that can apply to any conventional chemotherapeutic include the formation of mutants that escape cell killing and regrow; the dense packing of cells within the tumor that creates a physical barrier to macromolecular transport; the absence of lymphatic drainage, creating an elevated interstitial pressure that reduces extravasation and fluid convection; the heterogeneous distribution of blood vessels that leaves certain tumor cells at a considerable diffusion distance; and the adsorption of agents in the perivascular tumor cells.

Radiation treatments address many of the shortcomings of chemotherapy. Radiation suffers from a lack of target specificity and from serious side effects. One side effect results from inflammatory reactions to radiation injury. The mechanisms of the inflammatory response include the induction of genes in the vascular endothelium that mediate the inflammatory response (Hallahan *et al.*, 1995; Hallahan *et al.*, 1996; Hallahan and Virudachalam, 1997). These radiation-inducible inflammatory mediators include the cell adhesion molecules (CAMs) E-selectin and ICAM-1, which are present on the vascular endothelium and provide molecular signals that regulate leukocyte adhesion and emigration. Leukocyte adhesion to blood vessels occurs soon after irradiation (Fliss and Menard, 1994; Panes *et al*., 1995). Infiltration of leukocytes into inflamed tissue is a primary component in inflammation mediated tissue injury (Collins, 1995; Springer, 1994; Fantone & Ward, 1985). The circulatory and migratory properties of neutrophils allow rapid accumulation of these inflammatory cells at sites of injury and infection. Neutrophils extravasate from the circulation in response to changes on the vascular endothelium that signal injury or infection.

CAM's that are induced within irradiated endothelial cells include the intercellular adhesion molecule-1 (ICAM-1) and the endothelial leukocyte adhesion molecule-1 (ELAM-1, E-selectin) (Hallahan *et al.,* 1995, 1996, 1997a, 1997b). The role of these cell adhesion molecules in the pathogenesis of tissue injury has been implicated in rodent lung, liver and kidney models (Lo *et al.,* 1994). Elimination of leukocyte binding to the selectins or ICAM-1 attenuates the inflammatory response (Sligh *et al.,* 1993; Labow *et al.,* 1994). The selectins E-selectin and P-selectin progressively reduces the velocity of leukocyte movement over the endothelium (Collins, 1995; Springer, 1994; Lawrence & Springer, 1991). Following the slowing of leukocytes within blood vessels, these inflammatory cells extravasate and migrate into the inflamed tissue (Springer, 1994; Fantone & Ward, 1985).

ICAM-1 functions in the pathogenesis of radiation-induced inflammatory injury in the lung. ICAM-1 is a proteoglycan /in the immunoglobulin superfamily that mediates leukocyte emigration from the circulation (Luscinskas *et al*., 1991; Smith *et al*., 1988). Homozygosity for a null ICAM-1 mutation (as found in the ICAM-1 -knockout mouse) eliminates the propagation of radiation-induced inflammation (Hallahan and Virudachalam, 1997).

Weibel Palade organelles (WPBs) (Weibel and Palada, 1964) are membranous organelles that are localized to the cytoplasm of endothelial cells Eyden, 1993). WPB have a single limiting membrane that encloses a dense matrix. von Willebrand factor (vWF) is compartmentalized to WPBs in endothelial cells and is released into the vascular lumen during exocytosis of WPB (Pinsky *et al.,* 1996). vWF is a large (360 kD) polypeptide that is capable of forming multimers within its storage granules in the vascular endothelium (Suzuki *et al.,* 1996). Ionizing radiation induces vWF expression (Jahroudi *et al.,* 1996) and subsequent platelet activation (Verheij *et al.,* 1994). The biological significance of radiation-induced platelet aggregates is that they may be associated with organ injury following bone marrow transplantation (Zeigler *et al.,* 1996). Platelet aggregation has been proposed to contribute to the pathogenesis of tissue injury (Senaldi and Piguet, 1997; Verheij *et al.,* 1994).

P-selectin (GMP 140, CD63P), an adhesion receptor for leukocytes, is an activation-dependent surface protein found within alpha granules of platelets and in the WPBs of endothelial cells. Studies of the ultrastructure of the vascular endothelium within neoplasms have demonstrated that WPBs in tumors are translocated to the cell membrane following stimulation (Eyden, 1993). P-selectin is translocated to the blood-tissue interface of the endothelium, and is not released from storage reservoirs, but remains tethered to the endothelial cell membrane (Johnston, 1989). P-selectin regulates several biological responses, including adhesion of circulating leukocytes to the vascular lumen, platelet aggregation, and activation of inflammatory cells (Malik and Lo, 1996). Platelets have recently been shown to roll along the vascular endothelium, which is dependent upon P-selected translocation to the luminal surface of blood vessels (Frenette *et al.,* 1995; Boukerche, 1996). P-selectin binds to its counterreceptors, carbohydrate ligands, and PSGL-1 on leukocytes, to activate signal transduction within the inflammatory cells and inflammatory cell activation (Haller *et al.,* 1997, Pouyani and Seed, 1995; Weyrich *et al.,* 1995; Celi *et al.,* 1994). P-selectin-mediated monocyte production of MCP-1, tissue factor, or TNF can be attenuated by P-selectin blocking antibody (Weyrich *et al.,* 1995; Celi *et al.,* 1994). P-selectin is required for the interaction between leukocytes and activated platelets (Lehr, *et al.*, 1994). Platelet aggregation is attenuated by antibodies to P-selectin and is markedly delayed in P-selectin -/- mice (Subramaniam *et al.,* Boukerche, 1996). The P-selectin knockout mouse has an attenuated inflammatory response (Mayadas *et al.,* 1993).

WO-A-96/25947 discloses methods and compositions for use in specifically targeting tumor vasculature endothelial cells.

Barker JN, Ciba Foundation Symposium (1995) 189 91-101 discloses studies on expression of adhesion molecules in inflammatory skin diseases.

Dulkanchainu T S *et al.* Annals of Surgery, (1998 Jun) 227 No. 6 832-840 discloses a study on the reduction of hepatic Ischemia/Reperfusion Injury by a Soluble P-Selectin Glycoprotein Ligand-1.

Berg E. L. *et al.,* Blood Vol. 85, No. 1., 1 January 1995 p31-37 discloses antibodies that are cross-reactive with E-and P-selectin and that block both E-and P-selectin functions.

Sluiter *et al.,* J. Cardiovascular Pharmacology Vol. 22, No. 4, 1993, p.537-543 discloses the role and mechanisms for expression of Leukocyte adhesion molecules on the vascular endothelium.

McEver R P., Journal of Cellular Biochemistry 45: 156-161 (1991) discloses GMP-140, a membrane glycoprotein that is translocated to the plasma membrane of a cell that is activated by agonists.

Taken together, these findings implicate the role of cell adhesion molecules in the radiation-mediated inflammatory response. It is therefore clear that a significant need exists for the development of novel strategies for the alleviation of radiation related damage to normal tissue in the treatment of solid tumors.

### SUMMARY OF THE INVENTION

The present invention seeks to overcome these and other drawbacks inherent in the prior art by providing improved compositions for use in the manufacture of medicaments for preventing damage to tissues, in particular to prevent damage to lung tissues, during radiation therapy. The present invention is based on the observation that P-selectin is translocated, shortly after radiation of pulmonary endothelium, to vascular lumen. The presence of P-selectin in this area is believed to facilitate leukocyte signaling and induction, which in turn causes inflammatory responses and tissue injury. In this aspect, the invention involves the blocking of P-selectin activation of leukocytes following the translocation of the molecule P-selectin to the lumen of tumor vasculature endothelial cells, caused by ionizing radiation.

Thus, in a first embodiment, the present disclosure concerns the use of compositions for manufacture of medicaments for preventing or treating radiation damage in an animal or human patient undergoing radiotherapy, or one that is accidentally exposed to ionizing radiation. These methods generally comprise administering to the irradiated animal or patient a pharmaceutically acceptable composition comprising a P-selectin binding agent alone, or with a selected second agent. The pharmaceutically acceptable composition may be administered to the animal in a topical, oral or parenteral formulation, depending on the damaged or protectable area. Binding to P-selectin, the interaction of this ligand with leukocyte and other inflammatory cell receptors in inhibited, and the inflammatory response should be reduced or blocked.

The translocation of P-selectin occurs rapidly - within about 30 minutes. Thus, targeting of the P-selectin to prevent leukocyte activation should occur within about 15 minutes of the radiation. The treatment may constitute a single dose of the blocking agent or multiple doses over a longer period - *e.g.*, about 30 minutes, about one hour, about two hours or later. The treatment may constitute a continuous provision of the blocking agent over the same extended periods: about 15-30 min., about 15-60 min., about 15-120 min., *etc*.

The P-selectin binding agent of the composition may be an antibody, preferably, a monoclonal antibody, or a fragment thereof, such as an scFv, Fv, Fab', Fab or F(ab')₂ fragment of an antibody. Preferred antibodies are monoclonal antibodies (MAbs), as may be obtained from a variety of commercial sources, or that may be generated using standard MAb technology.

Other effective P-selectin binding agents are oligosaccharides, polysaccharides, glycolipids, and even glycoproteins; oligosaccharides, polysaccharides or glycolipids formulated into liposome preparations; and oligosaccharides, polysaccharides or glycolipids conjugated to a protein or polypeptide carrier, such as albumin. Currently preferred liposome preparations are cationic liposomes, DOTMA/DOPE, DOTMA and DORIE.

Suitable oligosaccharides include PGSL, glycyrrhizin, carminic acid, cylexin, sialyl Lewis X/A oligosaccharides and sialyl Lewis X/A mimics. Examples of sialyl Lewis X/A oligosaccharides include sialyl Lewis X (sialyl Lewis^{x}, sLe^{x}, Neu5Acα2-3Galβ1-4 [Fucα1-3]GlcNAc); sialyl Lewis A (sialyl Lewis^{a}, sLe^{a}, Neu5Acα2-3Galβ1-3 [Fucα1-4]GlcNAc); sialyl Lewis X/A (sLe^{xa}); sialyl Lewis pentasaccharides; sialyl Lewis tetrasaccharides; sulfated Le penta- and tetrasaccharides; and dimeric sialyl Lewis compounds. Currently preferred oligosaccharides are glycyrrhizin and sialyl Lewis X/A-based oligosaccharides, particularly sialyl Lewis pentasaccharides and tetrasaccharides; sulfated Le penta-and tetrasaccharides; and amino substituted sLe^{a}, as described by Nelson *et al.* (1993). Further useful P-selectin binding agents include structural analogues of the above oligosaccharides, including those identified in the pharmacophore search by Narasinga Rao *et al.* (1994).

Suitable P-selectin-binding polysaccharides include polylactosamine. A glycoprotein contemplated for use as an P-selectin targeting component is Protein C (Grinnell *et al.,* 1994); and the 150 kD, 230 kd and 130 kd glycoproteins described by Lenter *et al.* (1994) may also be employed. As used herein, the 150 kD, 230 kd and 130 kd glycoproteins described by Lenter *et al.* (1994) are also referred to the "150 kD", the "230 kD", and the "130 kD", repectively.

Still other effective P-selectin targeting components are cells, such as T lymphocytes or leukocytes, PMN's, eosinophils, NK cells, and the like.

In a second embodiment, the observation that P-selectin is translocated to vascular lumen raises the possibility of using P-selectin targeting agents to deliver agents to the vasculature of a tumor or an otherwise diseased vasculature. In this aspect, the disclosure requires the translocation of the molecule P-selectin to the lumen of tumor vasculature endothelial cells by ionizing radiation, which then allows P-selectin to be targeted using specific binding compositions and selected agents.

The disclosure therefore provides the use of compositions for manufacture of medicaments for delivering a selected agent to the vasculature of an animal or human subject comprising, generally, inducing P-selectin translocation to the lumen of vascular endothelial cells and administering to the animal a composition comprising a P-selectin targeting component operatively associated with or attached to a selected agent.

Again, given the relatively rapid translocation of P-selectin to the lumen, the administration of the targeting agent should occur within about 15-120 minutes, preferably about 30 minutes, from the radiation. The administration may be a single dose, multiple dose, or continuous infusion.

Also provided are the use of compositions for the manufacture of medicaments for delivering a selected agent to the tumor-associated vasculature of an animal or human patient having a vascularized tumor, which methods generally comprise translocating P-selectin to the tumor-associated vascular endothelial lumen and administering to the animal a composition comprising a P-selectin targeting compound operatively associated with or attached to a selected agent.

Further provided are the use of compositions for manufacture of medicaments for delivering a selected agent to the disease-associated vasculature of an animal or human subject with a disease that has a vascular component, *i.e.,* a disease that is connected in some manner with the aberrant function, or number, of blood vessels. These methods generally comprise inducing translocation of P-selectin to the lumen of disease-associated vascular endothelial cells and administering to the animal or patient a composition comprising an P-selectin targeting compound operatively associated with a selected agent.

The use of compositions for manufacture of medicaments for treating malignant and benign diseases that have a dysfunction of the vasculature as one causal or contributory factor also are provided. Such diseases include various solid tumors, diabetic retinopathy, vascular restenosis, arteriovenous malformation (AVM) and meningioma. These treatment methods generally include inducing the translocation of P-selectin to the lumen of disease-associated vascular endothelial cells of an animal or patient having a vascularized tumor, or a vascular component-associated benign disease, and administering to the animal or patient a therapeutically effective amount of a pharmaceutical composition comprising a P-selectin targeting component operatively associated with or attached to a selected agent.

In all such methods, the P-selectin translocation to the lumen of vascular endothelial cells may be induced by ionizing radiation, *i.e.,* by exposing the cells to ionizing radiation at a dose sufficient to result in translocation. P-selectin translocation may be induced by γ-irradiation, or preferably, by using by x-rays. Where cancer or other disease are to be treated, P-selectin translocation may be induced by specific x-ray irradiation of the tumor or disease site. Translocation of P-selectin also may be achieved using heat or oxidants, such as H₂O₂ or O₂. This may also be controlled and directed to a specific area of the body.

Following induction of P-selectin translocation, a composition of one or more selectin-second agent components is administered to the animal or patient, generally in a pharmaceutically acceptable formulation. This may be achieved by parenteral administration, by injection or instillation into the disease site or vascularized tumor site, *e.g*., using any one of a variety of catheters.

The P-selectin targeting component of the composition may be an antibody, preferably, a monoclonal antibody, or a fragment thereof, such as an scFv, Fv, Fab', Fab or F(ab')₂ fragment of an antibody. Preferred antibodies are monoclonal antibodies (MAbs), as may be obtained from a variety of commercial sources, or that may be generated using standard MAb technology.

Other effective P-selectin targeting components are oligosaccharides, polysaccharides, glycolipids, and even glycoproteins; oligosaccharides, polysaccharides or glycolipids formulated into liposome preparations; and oligosaccharides, polysaccharides or glycolipids conjugated to a protein or polypeptide carrier, such as albumin. Currently preferred liposome preparations are cationic liposomes, DOTMA/DOPE, DOTMA and DORIE.

Suitable oligosaccharides include PGSL, glycyrrhizin, carminic acid, cylexin, sialyl Lewis X/A oligosaccharides and sialyl Lewis X/A mimics. Examples of sialyl Lewis X/A oligosaccharides include sialyl Lewis X (sialyl Lewis^{x}, sLe^{x}, Neu5Acα2-3Galβ1-4 [Fucα1-3]GlcNAc); sialyl Lewis A (sialyl Lewis^{a}, sLe^{a}, Neu5Acα2-3Galβ1-3 [Fucα1-4]GlcNAc); sialyl Lewis X/A (sLe^{xa}); sialyl Lewis pentasaccharides; sialyl Lewis tetrasaccharides; sulfated Le penta- and tetrasaccharides; and dimeric sialyl Lewis compounds. Currently preferred oligosaccharides are glycyrrhizin and sialyl Lewis X/A-based oligosaccharides, particularly sialyl Lewis pentasaccharides and tetrasaccharides; sulfated Le penta-and tetrasaccharides; and amino substituted sLe^{a}, as described by Nelson *et al.* (1993). Further useful P-selectin binding agents include structural analogues of the above oligosaccharides, including those identified in the pharmacophore search by Narasinga Rao *et al.* (1994).

Suitable P-selectin-targeting polysaccharides include polylactosamine. A glycoprotein contemplated for use as an P-selectin targeting component is Protein C (Grinnell *et al.,* 1994); and the 150 kD, 230 kd and 130 kd glycoproteins described by Lenter *et al.* (1994) may also be employed.

Still further effective P-selectin selectin targeting components are cells, such as T lymphocytes or leukocytes, helper T cells, polymorphonuclear neutrophils, eosinophils, NK cells, and the like, that are known to bind to P-selectin physiologically. Tumor-infiltrating lymphocytes (TILs) may be obtained from the animal to be treated and re-administered in conjunction with a selected agent or transfected with the ELAM ligand fucosyltransferase (ELFT) gene. The selected agent may be a recombinant vector that is inserted into the TIL, so that the vector expresses a protein following uptake into the vascular endothelial cells. Ligands isolated from leukocytes or T cells or from polymorphonuclear neutrophils, or recombinant versions of such ligands, may also be used as P-selectin targeting components. One such example is the HECA-452 antigen from lymphocytes (De Boer *et al.,* 1994).

Antibodies and oligosaccharide-containing compounds already administered to humans in the treatment of distinct diseases conditions may be initially preferred for use in this invention. For example, glycyrrhizin is a natural product that is used in Chinese herbal medicines (Davis and Morris, 1991). Many antibodies have been tested in animal models in studies directed to areas of diagnosis or treatment other than those connected with radiation (Keelan *et al.,* 1994a; 1994b; Chapman *et al.,* 1994; Ulich *et al.,* 1994; Silber *et al.,* 1994; Gosset *et al.,* 1995), and as such have demonstrated acceptable safety levels. Diagnosis and therapy using antibody-based compounds is particularly based upon Roeske *et al.* (1990) and Leichner *et al.* (1993).

In the disclosed methods, after P-selectin translocation is induced to the lumen of vascular endothelial cells, a composition comprising a selectin targeting component operatively associated with, or attached to, a selected agent, is administered to an animal or patient.

Appropriate selected agents include therapeutic agents, such as thrombolytic agents, and also, anticellular agents that kill or suppress the growth or cell division of disease-associated endothelial cells. Examples of effective thrombolytic agents are streptokinase and urokinase.

Effective anticellular agents include classical chemotherapeutic agents, such as steroids, antimetabolites, anthracycline, vinca alkaloids, antibiotics, alkylating agents, epipodophyllotoxin and anti-tumor agents such as neocarzinostatin (NCS), adriamycin and dideoxycytidine; mammalian cell cytotoxins, such as interferon-α (IFN-α), interferon-βγ (IFN-βγ), interleukin-12 (IL-12) and tumor necrosis factor-α (TNF-α); plant-, fungus- and bacteria-derived toxins, such as ribosome inactivating protein, gelonin, α-sarcin, aspergillin, restrictocin, ribonucleases, diphtheria toxin, *Pseudomonas* exotoxin, bacterial endotoxins, the lipid A moiety of a bacterial endotoxin, ricin A chain, deglycosylated ricin A chain and recombinant ricin A chain; as well as radioisotopes.

Diagnostic agents will generally be a fluorogenic, paramagnetic or radioactive ion that is detectable upon imaging. Examples of paramagnetic ions include chromium (III), manganese (II), iron (III), iron (II), cobalt (II), nickel (II), copper (II), neodymium (III), samarium (III), ytterbium (III), gadolinium (III), vanadium (II), terbium (III), dysprosium (III), holmium (III) and erbium (III) ions.

Examples of radioactive ions include iodine¹²³, technicium^{99m}, indium¹¹¹, rhenium¹⁸⁸, rhenium¹⁸⁶, copper⁶⁷, iodine¹³¹, yttrium⁹⁰, iodine¹²⁵, astatine²¹¹, gallium⁶⁷, iridium¹⁹², cobalt⁶⁰, radium²²⁶, gold¹⁹⁸, cesium¹³⁷ and phosphorus³² ions. Examples of fluorogenic agents include gadolinium and renographin.

In operatively attaching a fluorogenic, paramagnetic or radioactive ion to an oligosaccharide, polysaccharide or glycolipid, one may wish to first conjugate the oligosaccharide, polysaccharide or glycolipid to a protein or polypeptide carrier, such as albumin, and then link the fluorogenic, paramagnetic or radioactive ion to the protein or polypeptide carrier, using methods commonly known in the art.

In certain embodiments, the selected agent will be a recombinant vector, or other gene-expressing unit, that comprises a promoter operatively linked to a protein expression region. The vector will then direct the expression of the encoded therapeutic protein or polypeptide following uptake into vascular endothelial cells.

The recombinant vectors may comprise an ionizing radiation-inducible promoter, such as a CArG domain of an *Egr-1* promoter, a *los* promoter, a c-jun promoter or TNF-α promoter, operatively linked to a protein expression region. Alternatively, the vector may have a vascular endothelial cell specific promoter operatively, such as an *Egr-1* gene promoter, an ICAM-1 gene promoter or an E-selectin gene promoter, linked to a protein expression region. Certain ionizing radiation-inducible promoters are also vascular endothelial cell specific promoters.

The protein expression region will often be one that expresses an anticellular agent capable of killing or suppressing the growth or cell division of disease-associated endothelial cells. Such agents are exemplified by tumor suppressor proteins, such as p53, p16, the retinoblastoma gene product and the Wilms' tumor gene product (WT1); chemotherapeutic agents, such as IFN-α, IFN-βγ, IL-12 and TNF-α; mammalian cell-, plant-, fungus- and bacteria-derived toxins, such as TNF-α, diphtheria toxin, *Pseudomonas* exotoxin, ricin A chain and deglycosylated ricin A chain; and agents that suppresses neovascularization.

The protein expression unit, gene or vector may also ultimately effect cell killing or growth suppression by expressing an enzyme capable of converting a non-toxic pro-drug into a cytotoxic drug. Effective examples include the herpes simplex virus (HSV) thymidine kinase (tk) enzyme and the cytosine deaminase enzyme.

The recombinant vector selected agents may be housed within cells, liposomes or viruses, such as a retrovirus, AAV, HSV-1, HPV, vaccinia, adeno-associated virus, or adenovirus, and may be targeted to selectin-expressing cells via other means, such as by linking to a bispecific antibody.

The present disclosure further provides the use of compositions for manufacture of medicaments for delivering agents to cells, and for treating benign and malignant diseases. These methods generally comprise administering to an animal or patient in which lumenal vascular endothelial cell P-selectin translocation has been induced, a pharmaceutical composition comprising a P-selectin targeting component operatively associated with a recombinant vector comprising an ionizing radiation-inducible promoter operatively linked to a protein expression region, and wherein the method further comprises inducing expression of the encoded protein by subsequently exposing the disease or tumor site to an additional effective dose of ionizing radiation.

The disclosure still further provides compositions for use a manufacture of medicaments for determining the radiation exposure of an animal or patient, which methods generally comprise determining the level of P-selectin translocation to the lumen of vascular endothelial cells of an irradiated site of the animal or patient, wherein an increase in the P-selectin level, in comparison to the level in normal or non-irradiated animals or patients, is indicative of an increase in radiation exposure.

The level of P-selectin translocation may be determined by means of administering to the animal or patient an effective amount of composition comprising a P-selectin targeting component operatively associated with a detectable marker and exposing the animal or patient to a detection device to identify the detectable marker.

The detection methods are exemplified by administering to the animal or patient glycyrrhizin, or an antibody that specifically binds to P-selectin, operatively associated with a nuclear magnetic spin-resonance isotope or a radioactive substance.

Compositions and kits of the present invention include all the above described combinations of P-selectin binding agents and targeting components operatively associated with or attached to all the above described selected agents, and such compositions dispersed in pharmacologically acceptable media. Currently preferred P-selectin targeting and binding components are antibodies, glycyrrhizin, carminic acid, cylexin, sialyl Lewis X, sialyl Lewis A, sialyl Lewis X/A and sialyl Lewis X/A mimics. Currently preferred selected agents are anticellular agent capable of killing or suppressing the growth or cell division of tumor-associated endothelial cells and fluorogenic, paramagnetic and radioactive ions that are detectable upon imaging.

Other objects, features and advantages of the present invention will become apparent from the following detailed description. It should be understood, however, that the detailed description and the specific examples, while indicating preferred embodiments of the invention, are given by way of illustration only, since various changes and modifications within the invention as defined in the claims will become apparent to those skilled in the art from this detailed description.

As used herein, the terms "binding" and "targeting" may be used interchangably. As used herein, the articles "a", "an" or "the" may be used to denote one or more than one.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein:

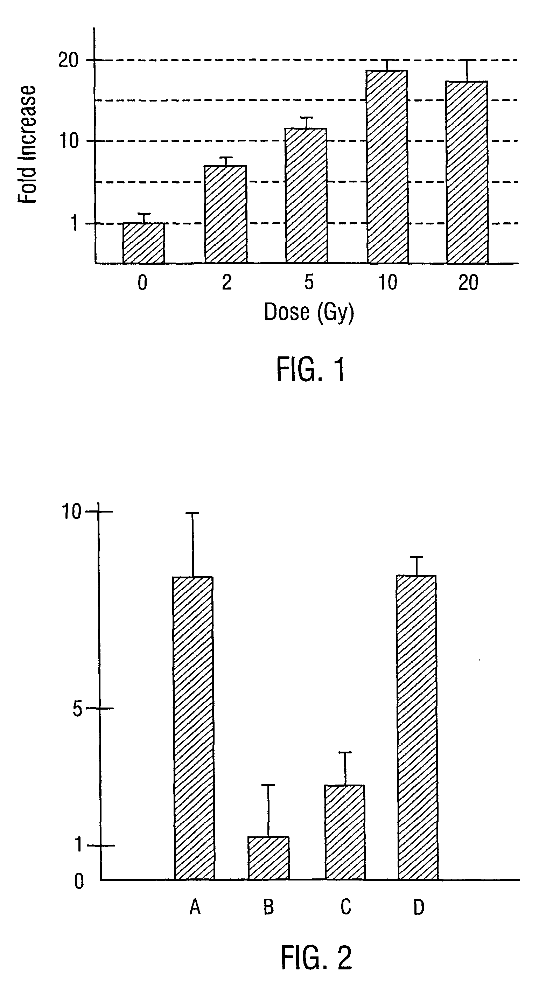

FIG. 1. Dose-dependent increase in E-selectin expression in the lungs from C3H mice. Intensity of E-selectin immunofluoresence at 6 hours after exposure to the indicated dose of thoracic irradiation. Shown are the mean and SEM of three experiments.
FIG. 2. Cell surface staining of P-selectin on x-irradiated (2 Gy) HUVEC. Cells were pretreated with A) buffer alone, B) Colcemid, C) nocodozol, D) cytochalasin-B. Confocal microscopy was used to measure immunofluorescence on the cell surface, which was quantified by NIH Image software. Shown are the mean and standard error of 10 cells measured in each of three experiments.

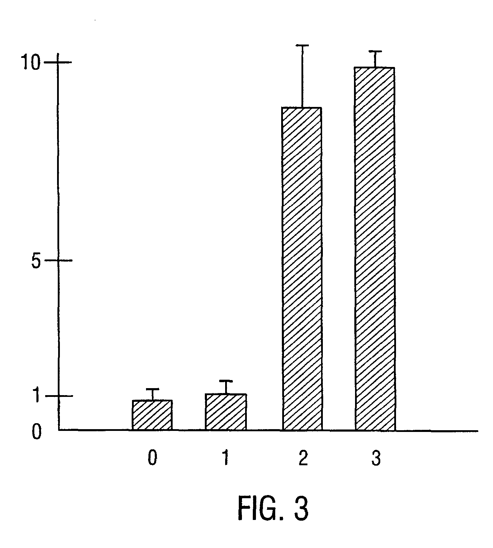

FIG. 3 Dose-dependent P-selectin translocation in irradiated HUVEC. HUVEC were x-irradiated with the indicated doses and fixed at 60 minutes. Confocal microscopy was used to measure immunofluorescence on the cell surface, which was quantified by NIH Image software. Bar 0, no irradiation, bar 1, 1 Gy, bar 2, 2 Gy, and bar 3, 5 Gy. Shown are the mean and standard error of 10 cells measured in each of three experiments.

### DETAILED DESCRIPTION OF THE PREFERRED EMBODIMENTS

### I. Present Invention

Persistent disease or local recurrence is a primary mode of failure in cancer patients, especially those with advanced disease (Haagense, 1971). For example, when breast tumors greater than 5 cm are treated with 80 to 90 Gy, 44% fail within the field of irradiation (Fletcher and Shukovsky, 1975). Furthermore, 50% of inflammatory breast carcinoma patients have local recurrences when the treated with daily irradiation (Barker *et al.,* 1980), while twice daily irradiation combined with chemotherapy reduces the rate of local recurrence to 20 to 27% (Fastenberg, 1985).

Thus, improved local control has been achieved by aggressive chemotherapy and radiotherapy, but this is limited by both local and systemic toxicity. Systemic toxicities may be reduced and efficacy may be increased by localizing therapy to the site of disease. One approach to localizing anticancer pharmaceuticals to cancers is to use cytotoxic agents with an affinity for tumor vasculature (Bicknell, 1994). The vasculature is required for neoplasia and destruction of the vasculature can result in tumor necrosis (Malik, 1992). Carcinomas have abundant vasculature that may serve as a target for site specific anticancer pharmaceuticals (Harris *et al.,* 1994).

Accumulation of vascular smooth muscle cells as a consequence of arterial injury is another major feature of vascular proliferative disorders. Molecular approaches to the inhibition of smooth muscle cell proliferation in these settings could potentially limit intimal expansion. This problem has been approached by introducing adenoviral vectors encoding the herpes virus thymidine kinase (tk) into porcine arteries that had been injured by a balloon on a catheter (Ohno *et al.,* 1994). These smooth muscle cells were shown to be infectable with adenoviral vectors, and introduction of the tk gene rendered them sensitive to the nucleoside analog ganciclovir. When this vector was introduced into porcine arteries immediately after a balloon injury, intimal hyperplasia decreased after a course of ganciclovir treatment. These data suggest that transient expression of an enzyme that catalyzes the formation of a cytotoxic drug locally may limit smooth muscle cell proliferation in response to balloon injury. Nonetheless, effective targeting of vascular tissue remains a challenge.

Endothelial cells line the lumen of blood vessels and cell adhesion-molecules (CAMs) expressed on their surface represent a potential target for "site-directed" pharmaceuticals. CAMs influence neutrophil binding following stimulation with cytokines or oxidants (Read *et al.,* 1994; Bevilacqua *et al.,* 1989). CAMs can also be induced by viral infection (Etingin *et al.,* 1991). As CAMs bind to specific carbohydrates, glycoproteins, cells, viruses and antibodies, they represent a potential target for therapeutics.

The inventors contemplated that an improved means of localizing CAM-based pharmaceuticals is to induce expression of a CAM within the tumor volume. This would require a CAM that is readily inducible, but has no basal expression in unirradiated tissues. The inventors selected low dose ionizing radiation as the inducing agent to promote endothelial cell surface expression of candidate molecules, with the aim of using radioisotopes and stereotactic radiotherapy to induce expression of CAMs in the vasculature of advanced cancers.

The findings that acute and subacute clinical manifestations of ionizing radiation may in part mimic the inflammatory response to a number of stimuli (Slauson *et al.,* 1976; Narayan and Cliff, 1982; Dunn *et al.,* 1986) prompted the inventors to investigate this area. Neutrophil margination of the vasculature and infiltration of the perivascular region occurs rapidly following irradiation (Reinhold *et al.,* 1990; Hopewell *et al.,* 1993; Dunn *et al.,* 1986; Matzner *et al.,* 1988). One of the components of acute inflammation is enhanced adherence of leukocytes to the endothelium before extravasation (Cliff, 1966). During the inflammatory reaction, endothelial cells rapidly and transiently produce a number of glycoproteins that influence neutrophil binding (Pober and Cotran, 1990). Unfortunately, this limits the doses of radiation that may be employed. Thus, inhibiting the binding and activation of leukocytes in response to radiation is another method for improving this therapeutic endeavor.

To study the dose dependence of x-ray-mediated adhesion molecule expression, HUVEC cells were irradiated with a dose range from 0.5 to 50 Gy and cells were scraped at 4 or 24 h. E-selectin expression increased at 4 h following exposure to 0.5 Gy and increased in a dose dependent manner until 20 Gy after which plateau was reached. When cells were scraped and incubated with antibody 24 h following irradiation, only cells treated with 20 Gy or greater had a persistent increase in E-selectin expression as compared to those treated with lower doses that returned to baseline. In contrast, ICAM was not expressed at x-ray doses below 5 Gy, but showed an increase at 24 h when treated with higher doses. These data indicate that E-selectin is induced transiently following low doses of irradiation, while ICAM induction requires high radiation doses and expression is more prolonged and PCAM and VCAM are not increased by irradiation. The inventors also found that E-selectin induction in irradiated endothelial cells is PKC independent but requires the NFκB binding sequence and Raf-1 kinase activity.

After x-irradiation, the endothelial leukocyte adhesion molecule-1 (ELAM-1, E-selectin) was primarily expressed in the pulmonary endothelium of larger vessels and minimally in the microvascular endothelium. Conversely, the intercellular adhesion molecule-1 (CD54; ICAM-1) was expressed in the pulmonary capillary endothelium, and minimally in the endothelium of larger vessels. Radiation-mediated E-selectin expression was first observed at 6 hours, whereas ICAM-1 expression initially increased at 24 h after irradiation. ICAM-1 and E-selectin expression persisted for several days.

P-selectin is constitutively expressed in Weibel-Palade bodies in the endothelium, which moved to the vascular lumen within 30 minutes after irradiation. P-selectin the entered the vascular lumen, but was not detected in the pulmonary endothelium at 6 hours after irradiation. The radiation dose required for increased P-selectin expression within the pulmonary vascular endothelium was 2 Gy, and expression increased in a dose-dependent manner. These data demonstrate that P-selectin is a viable target for tumor-directed therapy.

Several classes of molecules bind to selectin including carbohydrates such as glycyrrhizin and sialyl Lewis X/A, monoclonal antibodies to selectins, and polymorphonuclear neutrophils. Conjugation of pharmaceuticals to molecules that bind selectins is now contemplated to allow localization of many classes of pharmaceuticals (Kanoka *et al.,* 1990). These include chemotherapeutic agents, radiopharmaceuticals, and gene therapy delivery systems such as liposomes, and lysosomes. Moreover, results of gene therapy in the treatment of cancer have had encouraging results (Gutierrez *et al.,* 1992), and can now be improved using selectin-targeting agents.

This invention therefore provides P-selectin conjugated pharmaceuticals that are directed to locally advanced cancers following radiation-induced translocation of P-selectin. Included are treatments of cancers such as brain, lung, liver, spleen, kidney, lymph node, small intestine, pancreas, blood cells, colon, stomach, breast, endometrium, prostate, testicle, ovary, skin, head and neck, esophagus, bone marrow and blood tumors. The invention is also intended for use in benign neoplasms, including meningiomas, arteriovenous malformations, hemangiomas and the like. Additionally, the invention may be employed in the treatment of other vascular diseases, such as diabetic retinopathy, because these treatments will destroy the aberrant vasculature - as the presently used laser treatment does.

P-selectin though to play a role in inflammatory cell attachment and activation in the vascular endothelium. Thus, in a second embodiment, there are provided methods for the reduction of radiation induced inflammation in tissues by the blocking of P-selectin binding and activation of leukocytes and other inflammatory response cells. P-selectin binding agents, formulated as pharmaceuticals, are provided to irradiation vasculature, for example, lung vasculature, for the purpose of inhibiting the interaction of P-selectin with inflammatory cells.

### II. Generating Antibodies Reactive With P-Selectin

In one aspect, the present invention contemplates an antibody that is immunoreactive with a P-selectin molecule of the present invention, or any portion thereof. An antibody can be a polyclonal or a monoclonal antibody. In a preferred embodiment, an antibody is a monoclonal antibody. Means for preparing and characterizing antibodies are well known in the art (see, *e.g*., Howell and Lane, 1988).

Briefly, a polyclonal antibody is prepared by immunizing an animal with an immunogen comprising a polypeptide of the present invention and collecting antisera from that immunized animal. A wide range of animal species can be used for the production of antisera. Typically an animal used for production of anti-antisera is a non-human animal including rabbits, mice, rats, hamsters, pigs or horses. Because of the relatively large blood volume of rabbits, a rabbit is a preferred choice for production of polyclonal antibodies.

Antibodies, both polyclonal and monoclonal, specific for isoforms of antigen may be prepared using conventional immunization techniques, as will be generally known to those of skill in the art. A composition containing antigenic epitopes of the compounds of the present invention can be used to immunize one or more experimental animals, such as a rabbit or mouse, which will then proceed to produce specific antibodies against the compounds of the present invention. Polyclonal antisera may be obtained, after allowing time for antibody generation, simply by bleeding the animal and preparing serum samples from the whole blood.

It is proposed that the monoclonal antibodies of the present invention will find useful application in standard immunochemical procedures, such as ELISA and Western blot methods and in immunohistochemical procedures such as tissue staining, as well as in other procedures which may utilize antibodies specific to P-selectin-related antigen epitopes. Additionally, it is proposed that monoclonal antitibodies specific to the particular P-selectin of different species may be utilized in other useful applications.

In general, both polyclonal and monoclonal antibodies against P-selectin may be used in a variety of embodiments. For example, they may be employed in antibody cloning protocols to obtain cDNAs or genes encoding other P-selectin. They may also be used in inhibition studies to analyze the effects of P-selectin related peptides in cells or animals. Anti-P-selectin antibodies will also be useful in immunolocalization studies to analyze the distribution of P-selectin during various cellular events, for example, to determine the cellular or tissue-specific distribution of P-selectin polypeptides under different points in the cell cycle. A particularly useful application of such antibodies is in purifying native or recombinant P-selectin, for example, using an antibody affinity column. The operation of all such immunological techniques will be known to those of skill in the art in light of the present disclosure.

Means for preparing and characterizing antibodies are well known in the art (see, *e.g*., Harlow and Lane, 1988. More specific examples of monoclonal antibody preparation are give in the examples below.

As is well known in the art, a given composition may vary in its immunogenicity. It is often necessary therefore to boost the host immune system, as may be achieved by coupling a peptide or polypeptide immunogen to a carrier. Exemplary and preferred carriers are keyhole limpet hemocyanin (KLH) and bovine serum albumin (BSA). Other albumins such as ovalbumin, mouse serum albumin or rabbit serum albumin can also be used as carriers. Means for conjugating a polypeptide to a carrier protein are well known in the art and include glutaraldehyde, *m*-maleimidobencoyl-N-hydroxysuccinimide ester, carbodiimide and bis-biazotized benzidine.

As also is well known in the art, the immunogenicity of a particular immunogen composition can be enhanced by the use of non-specific stimulators of the immune response, known as adjuvants. Exemplary and preferred adjuvants include complete Freund's adjuvant (a non-specific stimulator of the immune response containing killed *Mycobacterium tuberculosis),* incomplete Freund's adjuvants and aluminum hydroxide adjuvant.

The amount of immunogen composition used in the production of polyclonal antibodies varies upon the nature of the immunogen as well as the animal used for immunization. A variety of routes can be used to administer the immunogen (subcutaneous, intramuscular, intradermal, intravenous and intraperitoneal). The production of polyclonal antibodies may be monitored by sampling blood of the immunized animal at various points following immunization. A second, booster, injection may also be given. The process of boosting and titering is repeated until a suitable titer is achieved. When a desired level of immunogenicity is obtained, the immunized animal can be bled and the serum isolated and stored, and/or the animal can be used to generate mAbs.

MAbs may be readily prepared through use of well-known techniques, such as those exemplified in U.S. Patent 4,196,265. Typically, this technique involves immunizing a suitable animal with a selected immunogen composition, *e.g.*, a purified or partially purified P-selectin protein, polypeptide or peptide or cell expressing high levels of P-selectin. The immunizing composition is administered in a manner effective to stimulate antibody producing cells. Rodents such as mice and rats are preferred animals, however, the use of rabbit, sheep frog cells is also possible. The use of rats may provide certain advantages (Goding, 1986), but mice are preferred, with the BALB/c mouse being most preferred as this is most routinely used and generally gives a higher percentage of stable fusions.

Following immunization, somatic cells with the potential for producing antibodies, specifically B-lymphocytes (B-cells), are selected for use in the mAb generating protocol. These cells may be obtained from biopsied spleens, tonsils or lymph nodes, or from a peripheral blood sample. Spleen cells and peripheral blood cells are preferred, the former because they are a rich source of antibody-producing cells that are in the dividing plasmablast stage, and the latter because peripheral blood is easily accessible. Often, a panel of animals will have been immunized and the spleen of animal with the highest antibody titer will be removed and the spleen lymphocytes obtained by homogenizing the spleen with a syringe. Typically, a spleen from an immunized mouse contains approximately 5 x 10⁷ to 2 x 10⁸ lymphocytes.

The antibody-producing B lymphocytes from the immunized animal are then fused with cells of an immortal myeloma cell, generally one of the same species as the animal that was immunized. Myeloma cell lines suited for use in hybridoma-producing fusion procedures preferably are non-antibody-producing, have high fusion efficiency, and enzyme deficiencies that render then incapable of growing in certain selective media which support the growth of only the desired fused cells (hybridomas).

Any one of a number of myeloma cells may be used, as are known to those of skill in the art (Goding, 1986). For example, where the immunized animal is a mouse, one may use P3-X63/Ag8, P3-X63-Ag8.653, NS1/1.Ag 4 1, Sp210-Ag14, FO, NSO/U, MPC-11, MPC11-X45-GTG 1.7 and S194/5XX0 Bul; for rats, one may use R210.RCY3, Y3-Ag 1.2.3, IR983F and 4B210; and U-266, GM1500-GRG2, LICR-LON-HMy2 and UC729-6 are all useful in connection with cell fusions.

Methods for generating hybrids of antibody-producing spleen or lymph node cells and myeloma cells usually comprise mixing somatic cells with myeloma cells in a 2:1 ratio, though the ratio may vary from about 20:1 to about 1:1, respectively, in the presence of an agent or agents (chemical or electrical) that promote the fusion of cell membranes. Fusion methods using Sendai virus have been described (Kohler and Milstein, 1975; 1976), and those using polyethylene glycol (PEG), such as 37% (v/v) PEG, by Gefter *et al.,* (1977). The use of electrically induced fusion methods is also appropriate (Goding, 1986).

Fusion procedures usually produce viable hybrids at low frequencies, around 1 x 10⁻⁶ to 1 x 10⁻⁸. However, this does not pose a problem, as the viable, fused hybrids are differentiated from the parental, unfused cells (particularly the unfused myeloma cells that would normally continue to divide indefinitely) by culturing in a selective medium. The selective medium is generally one that contains an agent that blocks the *de novo* synthesis of nucleotides in the tissue culture media. Exemplary and preferred agents are aminopterin, methotrexate, and azaserine. Aminopterin and methotrexate block *de novo* synthesis of both purines and pyrimidines, whereas azaserine blocks only purine synthesis. Where aminopterin or methotrexate is used, the media is supplemented with hypoxanthine and thymidine as a source of nucleotides (HAT medium). Where azaserine is used, the media is supplemented with hypoxanthine.

The preferred selection medium is HAT. Only cells capable of operating nucleotide salvage pathways are able to survive in HAT medium. The myeloma cells are defective in key enzymes of the salvage pathway, *e.g.*, hypoxanthine phosphoribosyl transferase (HPRT), and they cannot survive. The B-cells can operate this pathway, but they have a limited life span in culture and generally die within about two weeks. Therefore, the only cells that can survive in the selective media are those hybrids formed from myeloma and B-cells.

This culturing provides a population of hybridomas from which specific hybridomas are selected. Typically, selection of hybridomas is performed by culturing the cells by single-clone dilution in microtiter plates, followed by testing the individual clonal supernatants (after about two to three weeks) for the desired reactivity. The assay should be sensitive, simple and rapid, such as radioimmunoassays, enzyme immunoassays, cytotoxicity assays, plaque assays, dot immunobinding assays, and the like.

The selected hybridomas would then be serially diluted and cloned into individual antibody-producing cell lines, which clones can then be propagated indefinitely to provide mAbs. The cell lines may be exploited for mAb production in two basic ways. A sample of the hybridoma can be injected (often into the peritoneal cavity) into a histocompatible animal of the type that was used to provide the somatic and myeloma cells for the original fusion. The injected animal develops tumors secreting the specific monoclonal antibody produced by the fused cell hybrid. The body fluids of the animal, such as serum or ascites fluid, can then be tapped to provide mAbs in high concentration. The individual cell lines could also be cultured *in vitro,* where the mAbs are naturally secreted into the culture medium from which they can be readily obtained in high concentrations. mAbs produced by either means may be further purified, if desired, using filtration, centrifugation and various chromatographic methods such as HPLC or affinity chromatography.

The individual cell lines could also be cultured *in vitro,* where the MAbs are naturally secreted into the culture medium from which they can be readily obtained in high concentrations.

MAbs produced by either means may be further purified, if desired, using filtration, centrifugation and various chromatographic methods such as HPLC or affinity chromatography. Fragments of the monoclonal antibodies of the invention can be obtained from the purified monoclonal antibodies by methods which include digestion with enzymes, such as pepsin or papain, and/or by cleavage of disulfide bonds by chemical reduction. Alternatively, monoclonal antibody fragments encompassed by the present invention can be synthesized using an automated peptide synthesizer.

It also is contemplated that a molecular cloning approach may be used to generate monoclonals. For this, combinatorial immunoglobulin phagemid libraries are prepared from RNA isolated from the spleen of the immunized animal, and phagemids expressing appropriate antibodies are selected by panning using cells expressing the antigen and control cells *e.g.*, normal-versus-tumor cells. The advantages of this approach over conventional hybridoma techniques are that approximately 10⁴ times as many antibodies can be produced and screened in a single round, and that new specificities are generated by H and L chain combination which further increases the chance of finding appropriate antibodies.

Humanized monoclonal antibodies are antibodies of animal origin that have been modified using genetic engineering techniques to replace constant region and/or variable region framework sequences with human sequences, while retaining the original antigen specificity. Such antibodies are commonly derived from rodent antibodies with specificity against human antigens. such antibodies are generally useful for *in vivo* therapeutic applications. This strategy reduces the host response to the foreign antibody and allows selection of the human effector functions.

The techniques for producing humanized immunoglobulins are well known to those of skill in the art. For example U.S. Patent No. 5,693,762 discloses methods for producing, and compositions of, humanized immunoglobulins having one or more complementarity determining regions (CDR's). When combined into an intact antibody, the humanized immunoglobulins are substantially non-immunogenic in humans and retain substantially the same affinity as the donor immunoglobulin to the antigen, such as a protein or other compound containing an epitope.

Other U.S. patents that teach the production of antibodies useful in the present invention include U.S. Patent No 5,565,332, which describes the production of chimeric antibodies using a combinatorial approach; 4,816,567 which describes recombinant immunoglobin preparations and 4,867,973 which describes antibody-therapeutic agent conjugates.

U.S. Patent 5,565,332 describes methods for the production of antibodies, or antibody fragments, which have the same binding specificity as a parent antibody but which have increased human characteristics. Humanized antibodies may be obtained by chain shuffling, perhaps using phage display technology. Human antibodies may also be produced by transforming B cells with EBV and subsequent cloning of secretors as described by Hoon *et al.,* (1993).

Antibody conjugates in which a P-selectin antibody is linked to a detectable label or a cytotoxic agent form further aspects of the invention. Diagnostic antibody conjugates may be used both in *vitro* diagnostics, as in a variety of immunoassays, and in vivo diagnostics, such as in imaging technology.

Certain antibody conjugates include those intended primarily for use *in vitro,* where the antibody is linked to a secondary binding ligand or to an enzyme (an enzyme tag) that will generate a colored product upon contact with a chromogenic substrate. Examples of suitable enzymes include urease, alkaline phosphatase, (horseradish) hydrogen peroxidase and glucose oxidase. Preferred secondary binding ligands are biotin and avidin or streptavidin compounds. The use of such labels is well known to those of skill in the art in light and is described, for example, in U.S. Patents 3,817,837; 3,850,752; 3,939,350; 3,996,345; 4,277,437; 4,275,149 and 4,366,241.

Radioactively labeled monoclonal antibodies of the present invention may be produced according to well-known methods in the art. For instance, monoclonal antibodies can be iodinated by contact with sodium or potassium iodide and a chemical oxidizing agent such as sodium hypochlorite, or an enzymatic oxidizing agent, such as lactoperoxidase. Monoclonal antibodies according to the invention may be labeled with radioisotopes by ligand exchange process, for example, by reducing pertechnate with stannous solution, chelating the reduced technetium onto a Sephadex column and applying the antibody to this column or by direct labeling techniques, *e.g.*, by incubating pertechnate, a reducing agent such as SNCl₂, a buffer solution such as sodium-potassium phthalate solution, and the antibody.

Intermediary functional groups which are often used to bind radioisotopes which exist as metallic ions to antibody are diethylenetriaminepentaacetic acid (DTPA) and ethylene diaminetetracetic acid (EDTA). Fluorescent labels include rhodamine, fluorescein isothiocyanate and renographin.

### III. Toxins and Radio- and Chemotherapeutics

Agents for delivery to cells include proteins such as toxins, enzymes, interferons, interleukins, hormones or cytokines, paramagnetic ions, radiotherapeutics and chemotherapeutics. For any polypeptide, a nucleic acid encoding that polypeptide may be delivered instead (see below).

Tumor suppressors of interest include p53, p16, RB, APC, DCC, NF-1, NF-2, WT-1, MEN-I, MEN-II, zac1, p73, VHL, MMAC1, FCC and MCC. Inducers of apoptosis, such as Bax, Bak, Bcl-Xₛ, Bik, Bid, Harakiri, Ad E1B, Bad and ICE-CED3 proteases, similarly could find use according to the present invention.

Various enzyme are of interest according to the present invention. Such enzymes include cytosine deaminase, hypoxanthine-guanine phosphoribosyltransferase, galactose-1-phosphate uridyltransferase, phenylalanine hydroxylase, glucocerbrosidase, sphingomyelinase, α-L-iduronidase, glucose-6-phosphate dehydrogenase, HSV thymidine kinase and human thymidine kinase.

Hormones are another group of polypeptides that may be delivered to cells as described herein. Included are growth hormone, prolactin, placental lactogen, luteinizing hormone, follicle-stimulating hormone, chorionic gonadotropin, thyroid-stimulating hormone, leptin, adrenocorticotropin (ACTH), angiotensin I and II, β-endorphin, β-melanocyte stimulating hormone (β-MSH), cholecystokinin, endothelin I, galanin, gastric inhibitory peptide (GIP), glucagon, insulin, lipotropins, neurophysins, somatostatin, calcitonin, calcitonin gene related peptide (CGRP), β-calcitonin gene related peptide, hypercalcemia of malignancy factor (1-40), parathyroid hormone-related protein (107-139) (PTH-rP), parathyroid hormone-related protein (107-111) (PTH-rP), glucagon-like peptide (GLP-1), pancreastatin, pancreatic peptide, peptide YY, PHM, secretin, vasoactive intestinal peptide (VIP), oxytocin, vasopressin (AVP), vasotocin, enkephalinamide, metorphinamide, alpha melanocyte stimulating hormone (alpha-MSH), atrial natriuretic factor (5-28) (ANF), amylin, amyloid P component (SAP-1), corticotropin releasing hormone (CRH), growth hormone releasing factor (GHRH), luteinizing hormone-releasing hormone (LHRH), neuropeptide Y, substance K (neurokinin A ), substance P and thyrotropin releasing hormone (TRH).

Other classes of polypeptides that are contemplated for the present invention include interferons, interleukins and cytokines. Inteferon-α, interferon-βγ, interleukin 1 (IL-1), IL-2, IL-3, IL-4, IL-5, IL-6, IL-7, IL-8, IL-9, IL-10, IL-11 IL-12, GM-CSF and G-CSF.

Other therapeutics polypeptides include antigens such as viral antigens, bacterial antigens, fungal antigens or parasitic antigens. Viruses include picornavirus, coronavirus, togavirus, flavirviru, rhabdovirus, paramyxovirus, orthomyxovirus, bunyavirus, arenvirus, reovirus, retrovirus, papovavirus, parvovirus, herpesvirus, poxvirus, hepadnavirus, and spongiform virus. Preferred viral targets include influenza, herpes simplex virus 1 and 2, measles, small pox, polio or HIV. Pathogens include trypanosomes, tapeworms, roundworms, helminths,. Also, tumor markers, such as fetal antigen or prostate specific antigen, may be targeted in this manner. Preferred examples include HIV env proteins and hepatitis B surface antigen. Administration of a vector according to the present invention for vaccination purposes would require that the vector-associated antigens be sufficiently non-immunogenic to enable long term expression of the transgene, for which a strong immune response would be desired. Preferably, vaccination of an individual would only be required infrequently, such as yearly or_ biennially, and provide long term immunologic protection against the infectious agent.

Many radioactive agents are known in the art; for both diagnostic and therapeutic purposes, as are methods for their attachment to antibodies (see, *e.g*., U.S. patents 5,021,236 and 4,472,509). Certain attachment methods involve the use of a metal chelate complex employing, for example, an organic chelating agent such a DTPA attached to the antibody (U.S. Patent 4,472,509). Monoclonal antibodies also may be reacted with an enzyme in the presence of a coupling agent such as glutaraldehyde or periodate. Conjugates with fluorescein markers are prepared in the presence of these coupling agents or by reaction with an isothiocyanate.

In the case of paramagnetic ions, one might mention by way of example ions such as chromium (III), manganese (II), iron (III), iron (II), cobalt (II), nickel (II), copper (II), neodymium (III), samarium (III), ytterbium (III), gadolinium (III), vanadium (II), terbium (III), dysprosium (III), holmium (III) and erbium (III), with gadolinium being particularly preferred.

Ions useful in other contexts, such as X-ray imaging, include but are not limited to lanthanum (III), gold (III), lead (II), and especially bismuth (III).

In the case of radioactive isotopes for therapeutic and/or diagnostic application, one might mention astatine²¹¹, ¹⁴carbon, ⁵¹chromium, ³⁶chlorine, ⁵⁷cobalt, ⁵⁸cobalt, copper⁶⁷, ¹⁵²Eu, gallium⁶⁷, ³hydrogen, iodine¹²³, iodine¹²⁵, iodine¹³¹, indium¹¹¹, ⁵⁹iron, ³²phosphorus, rhenium¹⁸⁶, rhenium¹⁸⁸, ⁷⁵selenium, ³⁵sulphur, technicium^{99m} and yttrium⁹⁰. ¹²⁵I is often being preferred for use in certain embodiments, and technicium^{99m} and indium¹¹¹ are also often preferred due to their low energy and suitability for long range detection.

Radioactively labeled monoclonal antibodies of the present invention may be produced according to well-known methods in the art. For instance, monoclonal antibodies can be iodinated by contact with sodium or potassium iodide and a chemical oxidizing agent such as sodium hypochlorite, or an enzymatic oxidizing agent, such as lactoperoxidase. Monoclonal antibodies according to the invention may be labeled with technetium-^{99m} by ligand exchange process, for example, by reducing pertechnate with stannous solution, chelating the reduced technetium onto a Sephadex column and applying the antibody to this column or by direct labeling techniques, *e.g*., by incubating pertechnate, a reducing agent such as SNCl₂, a buffer solution such as sodium-potassium phthalate solution, and the antibody.

Intermediary functional groups which are often used to bind radioisotopes which exist as metallic ions to antibody are diethylenetriaminepentaacetic acid (DTPA) and ethylene diaminetetracetic acid (EDTA).

The agent also may a chemotherapeutic agent. Examples of such agents are adriamycin (doxorubicin), 5-fluorouracil, etoposide, camptothecin, actinomycin D, mitomycin C, or cisplatin. Agents that directly cross-link nucleic acids, specifically DNA, are envisaged to facilitate DNA damage. Agents such as cisplatin, and other DNA alkylating agents may be used. Cisplatin has been widely used to treat cancer, with efficacious doses used in clinical applications of 20 mg/m² for 5 days every three weeks for a total of three courses. Cisplatin is not absorbed orally and must therefore be delivered via injection intravenously, subcutaneously, intratumorally or intraperitoneally.

Agents that damage DNA also include compounds that interfere with DNA replication, mitosis and chromosomal segregation. Such chemotherapeutic compounds include adriamycin, also known as doxorubicin, etoposide, verapamil, podophyllotoxin, and the like. Widely used in a clinical setting for the treatment of neoplasms, these compounds are administered through bolus injections intravenously at doses ranging from 25-75 mg/m² at 21 day intervals for adriamycin, to 35-50 mg/m² for etoposide intravenously or double the intravenous dose orally.

Agents that disrupt the synthesis and fidelity of nucleic acid precursors and subunits also lead to DNA damage. As such a number of nucleic acid precursors have been developed. Particularly useful are agents that have undergone extensive testing and are readily available. As such, agents such as 5-fluorouracil (5-FU), are preferentially used by neoplastic tissue, making this agent particularly useful for targeting to neoplastic cells. Although quite toxic, 5-FU, is applicable in a wide range of carriers, including topical, however intravenous administration with doses ranging from 3 to 15 mg/kg/day being commonly used.

Other exemplary chemotherapeutic agents include hormones such as steroids; antimetabolites such as cytosine arabinoside, methotrexate or aminopterin; anthracycline; mitomycin C; vinca alkaloids; demecolcine; mithramycin; or alkylating agents such as chlorambucil or melphalan.

Preferred immunotoxins often include a plant-, fungal- or bacterial-derived toxin, such as an A chain toxin, a ribosome inactivating protein, α-sarcin, aspergillin, restirictocin, a ribonuclease, diphtheria toxin or *pseudomonas* exotoxin, to mention just a few examples. The use of toxin-antibody constructs is well known in the art of immunotoxins, as is their attachment to antibodies. Of course, combinations of the various toxins could also be coupled to one antibody molecule, thereby accommodating variable or even enhanced cytotoxicity.

One type of toxin for attachment to antibodies is ricin, with deglycosylated ricin A chain being particularly preferred. As used herein, the term "ricin" is intended to refer to ricin prepared from both natural sources and by recombinant means. Various 'recombinant' or 'genetically engineered' forms of the ricin molecule are known to those of skill in the art, all of which may be employed in accordance with the present invention.

Deglycosylated ricin A chain (dgA) is preferred because of its extreme potency, longer half-life, and because it is economically feasible to manufacture it a clinical grade and scale (available commercially from Inland Laboratories, Austin, TX.). Truncated ricin A chain, from which the 30 N-terminal amino acids have been removed by Nagarase (Sigma), also may be employed.

### IV. Linking of Agents to P-Selectin Targeting Components

Linking or coupling one or more agents to an a targeting component may be achieved by a variety of mechanisms, for example, covalent binding, affinity binding, intercalation, coordinate binding and complexation. Preferred binding methods are those involving covalent binding, such as using chemical cross-linkers, natural peptides or disulfide bonds.

The covalent binding can be achieved either by direct condensation of existing side chains or by the incorporation of external bridging molecules. Many bivalent or polyvalent agents are useful in coupling protein molecules to other proteins, peptides or amine functions. Examples of coupling agents are carbodiimides, diisocyanates, glutaraldehyde, diazobenzenes, and hexamethylene diamines. This list is not intended to be exhaustive of the various coupling agents known in the art but, rather, is exemplary of the more common coupling agents that may be used.

In preferred embodiments, it is contemplated that one may wish to first derivatize the component, and then attach the agent to the derivatized product. As used herein, the term "derivatize" is used to describe the chemical modification of the antibody substrate with a suitable cross-linking agent. Examples of cross-linking agents for use in this manner include the disulfide-bond containing linkers SPDP (*N*-succinimidyl-3-(2-pyridyldithio)propionate) and SMPT (4-succinimidyl-oxycarbonyl-α-methyl-α(2-pyridyldithio)toluene).

Biologically releasable bonds are particularly important to the realization of a clinically active moieties, which should be capable of being released from the targeting component once it has entered the target cell. Numerous types of linking constructs are known, including simply direct disulfide bond formation between sulfhydryl groups contained on amino acids such as cysteine, or otherwise introduced into respective protein structures, and disulfide linkages using available or designed linker moieties.

Numerous types of disulfide-bond containing linkers are known which can successfully be employed to conjugate agents to targeting components, however, certain linkers are generally preferred, such as, for example, sterically hindered disulfide bond linkers are preferred due to their greater stability *in vivo,* thus preventing release of the toxin moiety prior to binding at the site of action. A particularly preferred cross-linking reagent is SMPT, although other linkers such as SATA, SPDP and 2-iminothiolane also may be employed. Once conjugated, it will be important to purify the conjugate so as to remove contaminants.

In general, one technique will incorporate the use of Blue-Sepharose with a gel filtration or gel permeation step. Blue-Sepharose is a column matrix composed of Cibacron Blue 3GA and agarose, which has been found to be useful in the purification of immunoconjugates. The use of Blue-Sepharose combines the properties of ion exchange with A chain binding to provide good separation of conjugated from unconjugated binding. The Blue-Sepharose allows the elimination of the free (non-conjugated antibody) targeting component from the conjugate preparation. To eliminate the free (unconjugated) agent (*e.g*., dgA) a molecular exclusion chromatography step may be used using either conventional gel filtration procedure or high performance liquid chromatography.

After a sufficiently purified conjugate has been prepared, one will generally desire to prepare it into a pharmaceutical composition that may be administered parenterally. This is done by using for the last purification step a medium with a suitable pharmaceutical composition. Such formulations will typically include pharmaceutical buffers, along with excipients, stabilizing agents and such like. The pharmaceutically acceptable compositions will be sterile, non-immunogenic and non-pyrogenic. Details of their preparation are well known in the art and are further described herein. It will be appreciated that endotoxin contamination should be kept minimally at a safe level, for example, less that 0.5 ng/mg protein.

Suitable pharmaceutical compositions in accordance with the invention will generally comprise from about 10 to about 100 mg of the desired conjugate admixed with an acceptable pharmaceutical diluent or excipient, such as a sterile aqueous solution, to give a final concentration of about 0.25 to about 2.5 mg/ml with respect to the conjugate.

In analyzing the variety of chemotherapeutic and pharmacologic agents available for conjugating to a targeting component, one may wish to particularly consider those that have been previously shown to be successfully conjugated to antibodies and to function pharmacologically. Exemplary antineoplastic agents that have been used include doxorubicin, daunomycin, methotrexate, vinblastine. Moreover, the attachment of other agents such as neocarzinostatin, macromycin, trenimon and α-amanitin has also been described. The lists of suitable agents presented herein are, of course, merely exemplary in that the technology for attaching pharmaceutical agents to antibodies for specific delivery to tissues is well established.

Thus, it is generally believed to be possible to conjugate to antibodies any pharmacologic agent that has a primary or secondary amine group, hydrazide or hydrazine group, carboxyl alcohol, phosphate, or alkylating group available for binding or cross-linking to the amino acids or carbohydrate groups of the antibody. In the case of protein structures, this is most readily achieved by means of a cross linking agent, as described above for the immunotoxins. Attachment also may be achieved by means of an acid labile acyl hydrazone or *cis* aconityl linkage between the drug and the antibody, or by using a peptide spacer such as L-Leu-L-Ala-L-Leu-L-Ala, between the γ-carboxyl group of the drug and an amino acid of the antibody.

### V. Pharmaceuticals and Routes of Administration

Where clinical applications and *in vivo* diagnositic applications are contemplated, it will be necessary to prepare pharmaceutical compositions in a form appropriate for the intended application. Generally, this will entail preparing compositions that are essentially free of pyrogens, as well as other impurities that could be harmful to humans or animals.

One will generally desire to employ appropriate salts and buffers to render delivery vectors stable and allow for uptake by target cells. Buffers also will be employed when recombinant cells are introduced into a patient. Aqueous compositions of the present invention comprise an effective amount of the vector to cells, dissolved or dispersed in a pharmaceutically acceptable carrier or aqueous medium. Such compositions also are referred to as inocula. The phrase "pharmaceutically or pharmacologically acceptable" refer to molecular entities and compositions that do not produce adverse, allergic, or other untoward reactions when administered to an animal or a human. As used herein, "pharmaceutically acceptable carrier" includes any and all solvents, dispersion media, coatings, antibacterial and antifungal agents, isotonic and absorption delaying agents and the like. The use of such media and agents for pharmaceutically active substances is well know in the art. Except insofar as any conventional media or agent is incompatible with the vectors or cells of the present invention, its use in therapeutic compositions is contemplated. Supplementary active ingredients also can be incorporated into the compositions.

The active compositions of the present invention may include classic pharmaceutical preparations. Administration of these compositions according to the present invention will be via any common route so long as the target tissue is available via that route. This includes oral, nasal, buccal, rectal, vaginal or topical. Alternatively, administration may be by orthotopic, intradermal, subcutaneous, intramuscular, intraperitoneal or intravenous injection. Such compositions would normally be administered as pharmaceutically acceptable compositions, injected intravenously in a direct, local or regional fashion with respect to the target site.

The active compounds may also be administered parenterally or intraperitoneally. Solutions of the active compounds as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating action of microorganisms, such as bacteria and fungi. The carrier can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion and by the use of surfactants. The prevention of the action of microorganisms can be brought about by various antibacterial an antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

### VII. Transgenic Animals/Knockout Animals

In one embodiment of the invention, transgenic animals are produced which lack a functional P-selectin gene. Such transgenic animals and transgenic embryos may be useful in methods as described in the examples, for example, in determining inflammatory dose responses to radiation. Alternatively, one may wish to introduce a P-selectin gene into a cell such that its regulation'is not typical of that found in nature.

Methods for producing transgenic animals are generally described by Wagner and Hoppe (U.S. Patent No. 4,873,191), Brinster *et al.* 1985 and in "Manipulating the Mouse Embryo; A Laboratory Manual" 2nd edition (eds., Hogan, Beddington, Costantimi and Long, Cold Spring Harbor Laboratory Press, 1994).

### VIII. Expression Constructs

The present invention also provides, in another embodiment, delivery of expression constructs. Expression constructs of the present invention will be used to deliver genes, under the control of appropriate eukaryotic regulatory machinery, such the genes are expressed and affect the target cell into which they have been delivered.

### A. Therapeutic Genes

The present invention contemplates the use of a variety of different genes. For example, genes encoding enzymes, hormones, cytokines, oncogenes, receptors, tumor suppressors, transcription factors, drug selectable markers, toxins and various antigens are contemplated as suitable genes for use according to the present invention. In addition, antisense constructs derived from oncogenes are other "genes" of interest according to the present invention.

Tumor suppressors include p53, p16, RB, APC, DCC, NF-1, NF-2, WT-1, MEN-I, MEN-II, zac1, p73, VHL, MMAC1, FCC and MCC. Inducers of apoptosis, such as Bax, Bak, Bcl-Xₛ, Bik, Bid, Harakiri, Ad E1B, Bad and ICE-CED3 proteases, similarly could find use according to the present invention.

Various enzyme genes are of interest according to the present invention. Such enzymes include cytosine deaminase, hypoxanthine-guanine phosphoribosyltransferase, galactose-1-phosphate uridyltransferase, phenylalanine hydroxylase, glucocerbrosidase, sphingomyelinase, α-L-iduronidase, glucose-6-phosphate dehydrogenase, HSV thymidine kinase and human thymidine kinase.

Hormones are another group of gene that may be used in the vectors described herein. Included are growth hormone, prolactin, placental lactogen, luteinizing hormone, follicle-stimulating hormone, chorionic gonadotropin, thyroid-stimulating hormone, leptin, adrenocorticotropin (ACTH), angiotensin I and II, β-endorphin, β-melanocyte stimulating hormone (β-MSH), cholecystokinin, endothelin I, galanin, gastric inhibitory peptide (GIP), glucagon, insulin, lipotropins, neurophysins, somatostatin, calcitonin, calcitonin gene related peptide (CORP), β-calcitonin gene related peptide, hypercalcemia of malignancy factor (1-40), parathyroid hormone-related protein (107-139) (PTH-rP), parathyroid hormone-related protein (107-111) (PTH-rP), glucagon-like peptide (GLP-1), pancreastatin, pancreatic peptide, peptide YY, PHM, secretin, vasoactive intestinal peptide (VIP), oxytocin, vasopressin (AVP), vasotocin, enkephalinamide, metorphinamide, alpha melanocyte stimulating hormone (alpha-MSH), atrial natriuretic factor (5-28) (ANF), amylin, amyloid P component (SAP-1), corticotropin releasing hormone (CRH), growth hormone releasing factor (GHRH), luteinizing hormone-releasing hormone (LHRH), neuropeptide Y, substance K (neurokinin A ), substance P and thyrotropin releasing hormone (TRH).

Other classes of genes that are contemplated to be inserted into the vectors of the present invention include interferons, interleukins and cytokines. Inteferon-α, interferon-βγ, interleukin 1 (IL-1), IL-2, IL-3, IL-4, IL-5, IL-6, IL-7, IL-8, IL-9, IL-10, IL-11 IL-12, GM-CSF and G-CSF.

Other therapeutics genes might include genes encoding antigens such as viral antigens, bacterial antigens, fungal antigens or parasitic antigens. Viruses include picornavirus, coronavirus, togavirus, flavirviru, rhabdovirus, paramyxovirus, orthomyxovirus, bunyavirus, arenvirus, reovirus, retrovirus, papovavirus, parvovirus, herpesvirus, poxvirus, hepadnavirus, and spongiform virus. Preferred viral targets include influenza, herpes simplex virus 1 and 2, measles, small pox, polio or HIV. Pathogens include trypanosomes, tapeworms, roundworms, helminths, . Also, tumor markers, such as fetal antigen or prostate specific antigen, may be targeted in this manner. Preferred examples include HIV env proteins and hepatitis B surface antigen. Administration of a vector according to the present invention for vaccination purposes would require that the vector-associated antigens be sufficiently non-immunogenic to enable long term expression of the transgene, for which a strong immune response would be desired. Preferably, vaccination of an individual would only be required infrequently, such as yearly or biennially, and provide long term immunologic protection against the infectious agent.

In yet another embodiment, the heterologous gene may include a single-chain antibody. Methods for the production of single-chain antibodies are well known to those of skill in the art. The skilled artisan is referred to U.S. Patent No. 5,359,046, for such methods. A single chain antibody is created by fusing together the variable domains of the heavy and light chains using a short peptide linker, thereby reconstituting an antigen binding site on a single molecule.

Single-chain antibody variable fragments (Fvs) in which the C-terminus of one variable domain is tethered to the N-terminus of the other via a 15 to 25 amino acid peptide or linker, have been developed without significantly disrupting antigen binding or specificity of the binding (Bedzyk *et al.,* 1990; Chaudhary *et al.,* 1990). These Fvs lack the constant regions (Fc) present in the heavy and light chains of the native antibody.

### B. Antisense Constructs

Antisense technology also may be used to "knock-out" the function of negative effector genes. Antisense methodology takes advantage of the fact that nucleic acids tend to pair with "complementary" sequences. By complementary, it is meant that polynucleotides are those which are capable of base-pairing according to the standard Watson-Crick complementarity rules. That is, the larger purines will base pair with the smaller pyrimidines to form combinations of guanine paired with cytosine (G:C) and adenine paired with either thymine (A:T) in the case of DNA, or adenine paired with uracil (A:U) in the case of RNA. Inclusion of less common bases such as inosine, 5-methylcytosine, 6-methyladenine, hypoxanthine and others in hybridizing sequences does not interfere with pairing.

Targeting double-stranded (ds) DNA with polynucleotides leads to triple-helix formation; targeting RNA will lead to double-helix formation. Antisense polynucleotides, when introduced into a target cell, specifically bind to their target polynucleotide and interfere with transcription, RNA processing, transport, translation and/or stability. Antisense RNA constructs, or DNA encoding such antisense RNA's, may be employed to inhibit gene transcription or translation or both within a host cell, either in vitro or in vivo, such as within a host animal, including a human subject.

Antisense constructs may be designed to bind to the promoter and other control regions, exons, introns or even exon-intron boundaries of a gene. It is contemplated that the most effective antisense constructs will include regions complementary to intron/exon splice junctions. Thus, it is proposed that a preferred embodiment includes an antisense construct with complementarity to regions within 50-200 bases of an intron-exon splice junction. It has been observed that some exon sequences can be included in the construct without seriously affecting the target selectivity thereof. The amount of exonic material included will vary depending on the particular exon and intron sequences used. One can readily test whether too much exon DNA is included simply by testing the constructs *in vitro* to determine whether normal cellular function is affected or whether the expression of related genes having complementary sequences is affected.

"Complementary" or "antisense" means polynucleotide sequences that are substantially complementary over their entire length and have very few base mismatches. For example, sequences of fifteen bases in length may be termed complementary when they have complementary nucleotides at thirteen or fourteen positions. Naturally, sequences which are completely complementary will be sequences which are entirely complementary throughout their entire length and have no base mismatches. Other sequences with lower degrees of homology also are contemplated. For example, an antisense construct which has limited regions of high homology, but also contains a non-homologous region (*e.g*., ribozyme; see below) could be designed. These molecules, though having less than 50% homology, would bind to target sequences under appropriate conditions.

It may be advantageous to combine portions of genomic DNA with cDNA or synthetic sequences to generate specific constructs. For example, where an intron is desired in the ultimate construct, a genomic clone will need to be used. The cDNA or a synthesized polynucleotide may provide more convenient restriction sites for the remaining portion of the construct and, therefore, would be used for the rest of the sequence.

### C. Ribozymes

Another approach for addressing the "dominant negative" mutants is through the use of ribozymes. Although proteins traditionally have been used for catalysis of nucleic acids, another class of macromolecules has emerged as useful in this endeavor. Ribozymes are RNA-protein complexes that cleave nucleic acids in a site-specific fashion. Ribozymes have specific catalytic domains that possess endonuclease activity (Kim and Cook, 1987; Gerlach *et al.,* 1987; Forster and Symons, 1987). For example, a large number of ribozymes accelerate phosphoester transfer reactions with a high degree of specificity, often cleaving only one of several phosphoesters in an oligonucleotide substrate (Cook *et al.,* 1981; Michel and Westhof, 1990; Reinhold-Hurek and Shub, 1992). This specificity has been attributed to the requirement that the substrate bind via specific base-pairing interactions to the internal guide sequence ("IGS") of the ribozyme prior to chemical reaction.

Ribozyme catalysis has primarily been observed as part of sequence-specific cleavage/ligation reactions involving nucleic acids (Cook *et al.,* 1981). For example, U.S. Patent No. 5,354,855 reports that certain ribozymes can act as endonucleases with a sequence specificity greater than that of known ribonucleases and approaching that of the DNA restriction enzymes. Thus, sequence-specific ribozyme-mediated inhibition of gene expression may be particularly suited to therapeutic applications (Scanlon *et al.,* 1991; Sarver *et al.,* 1990). Recently, it was reported that ribozymes elicited genetic changes in some cells lines to which they were applied; the altered genes included the oncogenes H-ras, c-fos and genes of HIV. Most of this work involved the modification of a target mRNA, based on a specific mutant codon that is cleaved by a specific ribozyme.

### D. Vectors for Cloning, Gene Transfer and Expression

In certain embodiments, expression vectors are used in gene therapy. Expression requires that appropriate signals be provided in the vectors, and which include various regulatory elements, such as enhancers/promoters from both viral and mammalian sources that drive expression of the genes of interest in host cells. Elements designed to optimize messenger RNA stability and translatability in host cells also are defined. The conditions for the use of a number of dominant drug selection markers for establishing permanent, stable cell clones expressing the products are also provided, as is an element that links expression of the drug selection markers to expression of the polypeptide.

### (i) Regulatory Elements

***Promoters.*** Throughout this application, the term "expression construct" is meant to include any type of genetic construct containing a nucleic acid coding for gene products in which part or all of the nucleic acid encoding sequence is capable of being transcribed. The transcript may be translated into a protein, but it need not be. In certain embodiments, expression includes both transcription of a gene and translation of mRNA into a gene product. In other embodiments, expression only includes transcription of the nucleic acid encoding genes of interest.

The nucleic acid encoding a gene product is under transcriptional control of a promoter. A "promoter" refers to a DNA sequence recognized by the synthetic machinery of the cell, or introduced synthetic machinery, required to initiate the specific transcription of a gene. The phrase "under transcriptional control" means that the promoter is in the correct location and orientation in relation to the nucleic acid to control RNA polymerase initiation and expression of the gene.

The term promoter will be used here to refer to a group of transcriptional control modules that are clustered around the initiation site for RNA polymerase II. Much of the thinking about how promoters are organized derives from analyses of several viral promoters, including those for the HSV thymidine kinase (*tk*) and SV40 early transcription units. These studies, augmented by more recent work, have shown that promoters are composed of discrete functional modules, each consisting of approximately 7-20 bp of DNA, and containing one or more recognition sites for transcriptional activator or repressor proteins.

At least one module in each promoter functions to position the start site for RNA synthesis. The best known example of this is the TATA box, but in some promoters lacking a TATA box, such as the promoter for the mammalian terminal deoxynucleotidyl transferase gene and the promoter for the SV40 late genes, a discrete element overlying the start site itself helps to fix the place of initiation.

Additional promoter elements regulate the frequency of transcriptional initiation. Typically, these are located in the region 30-110 bp upstream of the start site, although a number of promoters have recently been shown to contain functional elements downstream of the start site as well. The spacing between promoter elements frequently is flexible, so that promoter function is preserved when elements are inverted or moved relative to one another. In the tk promoter, the spacing between promoter elements can be increased to 50 bp apart before activity begins to decline. Depending on the promoter, it appears that individual elements can function either co-operatively or independently to activate transcription.

The particular promoter employed to control the expression of a nucleic acid sequence of interest is not believed to be important, so long as it is capable of directing the expression of the nucleic acid in the targeted cell. Thus, where a human cell is targeted, it is preferable to position the nucleic acid coding region adjacent to and under the control of a promoter that is capable of being expressed in a human cell. Generally speaking, such a promoter might include either a human or viral promoter.

In various embodiments, the human cytomegalovirus (CMV) immediate early gene promoter, the SV40 early promoter, the Rous sarcoma virus long terminal repeat, β-actin, rat insulin promoter and glyceraldehyde-3-phosphate dehydrogenase can be used to obtain high-level expression of the coding sequence of interest. The use of other viral or mammalian cellular or bacterial phage promoters which are well-known in the art to achieve expression of a coding sequence of interest is contemplated as well, provided that the levels of expression are sufficient for a given purpose. By employing a promoter with well-known properties, the level and pattern of expression of the protein of interest following transfection or transformation can be optimized.

Selection of a promoter that is regulated in response to specific physiologic or synthetic signals can permit inducible expression of the gene product. For example in the case where expression of a transgene, or transgenes when a multicistronic vector is utilized, is toxic to the cells in which the vector is produced in, it may be desirable to prohibit or reduce expression of one or more of the transgenes. Examples of transgenes that may be toxic to the producer cell line are pro-apoptotic and cytokine genes. Several inducible promoter systems are available for production of viral vectors where the transgene product may be toxic.

The ecdysone system (Invitrogen, Carlsbad, CA) is one such system. This system is designed to allow regulated expression of a gene of interest in mammalian cells. It consists of a tightly regulated expression mechanism that allows virtually no basal level expression of the transgene, but over 200-fold inducibility. The system is based on the heterodimeric ecdysone receptor of Drosophila, and when ecdysone or an analog such as muristerone A binds to the receptor, the receptor activates a promoter to turn on expression of the downstream transgene high levels of mRNA transcripts are attained. In this system, both monomers of the heterodimeric receptor are constitutively expressed from one vector, whereas the ecdysone-responsive promoter which drives expression of the gene of interest is on another plasmid. Engineering of this type of system into the gene transfer vector of interest would therefore be useful. Cotransfection of plasmids containing the gene of interest and the receptor monomers in the producer cell line would then allow for the production of the gene transfer vector without expression of a potentially toxic transgene. At the appropriate time, expression of the transgene could be activated with ecdysone or muristeron A.

Another inducible system that would be useful is the Tet-Off^{TM} or Tet-On^{TM} system (Clontech, Palo Alto, CA) originally developed by Gossen and Bujard (Gossen and Bujard, 1992; Gossen *et al.,* 1995). This system also allows high levels of gene expression to be regulated in response to tetracycline or tetracycline derivatives such as doxycycline. In the Tet-On^{TM} system, gene expression is turned on in the presence of doxycycline, whereas in the Tet-Off^{TM} system, gene expression is turned on in the absence of doxycycline. These systems are based on two regulatory elements derived from the tetracycline resistance operon of *E. coli.* The tetracycline operator sequence to which the tetracycline repressor binds, and the tetracycline repressor protein. The gene of interest is cloned into a plasmid behind a promoter that has tetracycline-responsive elements present in it. A second plasmid contains a regulatory element called the tetracycline-controlled transactivator, which is composed, in the Tet-Off^{TM} system, of the VP16 domain from the herpes simplex virus and the wild-type tertracycline repressor. Thus in the absence of doxycycline, transcription is constitutively on. In the Tet-On^{TM} system, the tetracycline repressor is not wild type and in the presence of doxycycline activates transcription. For gene therapy vector production, the Tet-Off^{TM} system would be preferable so that the producer cells could be grown in the presence of tetracycline or doxycycline and prevent expression of a potentially toxic transgene, but when the vector is introduced to the patient, the gene expression would be constituitively on.

In some circumstances, it may be desirable to regulate expression of a transgene in a gene therapy vector. For example, different viral promoters with varying strengths of activity may be utilized depending on the level of expression desired. In mammalian cells, the CMV immediate early promoter if often used to provide strong transcriptional activation. Modified versions of the CMV promoter that are less potent have also been used when reduced levels of expression of the transgene are desired. When expression of a transgene in hematopoetic cells is desired, retroviral promoters such as the LTRs from MLV or MMTV are often used. Other viral promoters that may be used depending on the desired effect include SV40, RSV LTR, HIV-1 and HIV-2 LTR, adenovirus promoters such as from the E1A, E2A, or MLP region, AAV LTR, cauliflower mosaic virus, HSV-TK, and avian sarcoma virus.

Similarly tissue specific promoters may be used to effect transcription in specific tissues or cells so as to reduce potential toxicity or undesirable effects to non-targeted tissues. For example, promoters such as the PSA, probasin, prostatic acid phosphatase or prostate-specific glandular kallikrein (hK2) may be used to target gene expression in the prostate.

In certain indications, it may be desirable to activate transcription at specific times after administration of the gene therapy vector. This may be done with such promoters as those that are hormone or cytokine regulatable. For example in gene therapy applications where the indication is a gonadal tissue where specific steroids are produced or routed to, use of androgen or estrogen regulated promoters may be advantageous. Such promoters that are hormone regulatable include MMTV, MT-1, ecdysone and RuBisco. Other hormone regulated promoters such as those responsive to thyroid, pituitary and adrenal hormones are expected to be useful in the present invention. Cytokine and inflammatory protein responsive promoters that could be used include K and T Kininogen (Kageyama *et al.,* 1987), c-fos, TNF-alpha, C-reactive protein (Arcone *et al.,* 1988), haptoglobin (Oliviero *et al.,* 1987), serum amyloid A2, C/EBP alpha, IL-1, IL-6 (Poli and Cortese, 1989), Complement C3 (Wilson *et al.,* 1990), IL-8, alpha-1 acid glycoprotein (Prowse and Baumann, 1988), alpha-1 antitypsin, lipoprotein lipase (Zechner *et al.,* 1988), angiotensinogen (Ron *et al.,* 1991), fibrinogen, c-jun (inducible by phorbol esters, TNF-alpha, UV radiation, retinoic acid, and hydrogen peroxide), collagenase (induced by phorbol esters and retinoic acid), metallothionein (heavy metal and glucocorticoid inducible), Stromelysin (inducible by phorbol ester, interleukin-1 and EGF), alpha-2 macroglobulin and alpha-1 antichymotrypsin.

Tumor specific promoters such as osteocalcin, hypoxia-responsive element (HRE), MAGE-4, CEA, alpha-fetoprotein, GRP78/BiP and tyrosinase may also be used to regulate gene expression in tumor cells. Other promoters that could be used according to the present invention include Lac-regulatable, chemotherapy inducible (*e.g*. MDR), and heat (hyperthermia) inducible promoters, radiation-inducible (*e.g.,* EGR (Joki *et al.,* 1995)), Alpha-inhibin, RNA pol III tRNA met and other amino acid promoters, U1 snRNA (Bartlett *et al.,* 1996), MC-1, PGK, β-actin and α-globin. Many other promoters that may be useful are listed in Walther and Stein (1996).

***Enhancers.*** Enhancers are genetic elements that increase transcription from a promoter located at a distant position on the same molecule of DNA. Enhancers are organized much like promoters. That is, they are composed of many individual elements, each of which binds to one or more transcriptional proteins. The basic distinction between enhancers and promoters is operational. An enhancer region as a whole must be able to stimulate transcription at a distance; this need not be true of a promoter region or its component elements. On the other hand, a promoter must have one or more elements that direct initiation of RNA synthesis at a particular site and in a particular orientation, whereas enhancers lack these specificities. Promoters and enhancers are often overlapping and contiguous, often seeming to have a very similar modular organization.

***Polyadenylation Signals.*** Where a cDNA insert is employed, one will typically desire to include a polyadenylation signal to effect proper polyadenylation of the gene transcript. The nature of the polyadenylation signal is not believed to be crucial to the successful practice of the invention, and any such sequence may be employed such as human or bovine growth hormone and SV40 polyadenylation signals. Also contemplated as an element of the expression cassette is a terminator. These elements can serve to enhance message levels and to minimize read through from the cassette into other sequences.

***IRES.*** In certain embodiments of the invention, the use of internal ribosome entry site (IRES) elements is contemplated to create multigene, or polycistronic, messages. IRES elements are able to bypass the ribosome scanning model of 5' methylated Cap dependent translation and begin translation at internal sites (Pelletier and Sonenberg, 1988). IRES elements from two members of the picornavirus family (poliovirus and encephalomyocarditis) have been described (Pelletier and Sonenberg, 1988), as well an IRES from a mammalian message (Macejak and Sarnow, 1991). IRES elements can be linked to heterologous open reading frames. Multiple open reading frames can be transcribed together, each separated by an IRES, creating polycistronic messages. By virtue of the IRES element, each open reading frame is accessible to ribosomes for efficient translation. Multiple genes can be efficiently expressed using a single promoter/enhancer to transcribe a single message.

Any heterologous open reading frame can be linked to IRES elements. This includes genes for secreted proteins, multi-subunit proteins, encoded by independent genes, intracellular or membrane-bound proteins and selectable markers. In this way, expression of several proteins can be simultaneously engineered into a cell with a single construct and a single selectable marker.

### (ii) Selectable Markers

In certain embodiments of the invention, the cells contain nucleic acid constructs of the present invention, a cell may be identified *in vitro* or *in vivo* by including a marker in the expression construct. Such markers would confer an identifiable change to the cell permitting easy identification of cells containing the expression construct. Usually the inclusion of a drug selection marker aids in cloning and in the selection of transformants, for example, genes that confer resistance to neomycin, puromycin, hygromycin, DHFR, GPT, zeocin and histidinol are useful selectable markers. Alternatively, enzymes such as herpes simplex virus thymidine kinase (*tk*) or chloramphenicol acetyltransferase (CAT) may be employed. Immunologic markers also can be employed. The selectable marker employed is not believed to be important, so long as it is capable of being expressed simultaneously with the nucleic acid encoding a gene product. Further examples of selectable markers are well known to one of skill in the art.

### (iii) Delivery of Expression Vectors

There are a number of ways in which expression vectors may introduced into cells. In certain embodiments of the invention, the expression construct comprises a virus or engineered construct derived from a viral genome. The ability of certain viruses to enter cells via receptor-mediated endocytosis, to integrate into host cell genome and express viral genes stably and efficiently have made them attractive candidates for the transfer of foreign genes into mammalian cells (Ridgeway, 1988; Nicolas and Rubenstein, 1988; Baichwal and Sugden, 1986; Temin, 1986). The first viruses used as gene vectors were DNA viruses including the papovaviruses (simian virus 40, bovine papilloma virus, and polyoma) (Ridgeway, 1988; Baichwal and Sugden, 1986) and adenoviruses (Ridgeway, 1988; Baichwal and Sugden, 1986). These have a relatively low capacity for foreign DNA sequences and have a restricted host spectrum. Furthermore, their oncogenic potential and cytopathic effects in permissive cells raise safety concerns. They can accommodate only up to 8 kb of foreign genetic material but can be readily introduced in a variety of cell lines and laboratory animals (Nicolas and Rubenstein, 1988; Temin, 1986).

***Adenoviruses.*** One of the preferred methods for *in vivo* delivery involves the use of an adenovirus expression vector. "Adenovirus expression vector" is meant to include those constructs containing adenovirus sequences sufficient to (a) support packaging of the construct and (b) to express an antisense polynucleotide that has been cloned therein. In this context, expression does not require that the gene product be synthesized.

The expression vector comprises a genetically engineered form of adenovirus. Knowledge of the genetic organization of adenovirus, a 36 kb, linear, double-stranded DNA virus, allows substitution of large pieces of adenoviral DNA with foreign sequences up to 7 kb (Grunhaus and Horwitz, 1992). In contrast to retrovirus, the adenoviral infection of host cells does not result in chromosomal integration because adenoviral DNA can replicate in an episomal manner without potential genotoxicity. Also, adenoviruses are structurally stable, and no genome rearrangement has been detected after extensive amplification. Adenovirus can infect virtually all epithelial cells regardless of their cell cycle stage. So far, adenoviral infection appears to be linked only to mild disease such as acute respiratory disease in humans.

Adenovirus is particularly suitable for use as a gene transfer vector because of its mid-sized genome, ease of manipulation, high titer, wide target cell range and high infectivity. Both ends of the viral genome contain 100-200 base pair inverted repeats (ITRs), which are *cis* elements necessary for viral DNA replication and packaging. The early (E) and late (L) regions of the genome contain different transcription units that are divided by the onset of viral DNA replication. The E1 region (E1A and E1B) encodes proteins responsible for the regulation of transcription of the viral genome and a few cellular genes. The expression of the E2 region (E2A and E2B) results in the synthesis of the proteins for viral DNA replication. These proteins are involved in DNA replication, late gene expression and host cell shut-off (Renan, 1990). The products of the late genes, including the majority of the viral capsid proteins, are expressed only after significant processing of a single primary transcript issued by the major late promoter (MLP). The MLP, (located at 16.8 m.u.) is particularly efficient during the late phase of infection, and all the mRNA's issued from this promoter possess a 5'-tripartite leader (TPL) sequence which makes them preferred mRNA's for translation.

In a current system, recombinant adenovirus is generated from homologous recombination between shuttle vector and provirus vector. Due to the possible recombination between two proviral vectors, wild-type adenovirus may be generated from this process. Therefore, it is critical to isolate a single clone of virus from an individual plaque and examine its genomic structure.

Generation and propagation of the current adenovirus vectors, which are replication deficient, depend on a unique helper cell line, designated 293, which was transformed from human embryonic kidney cells by Ad5 DNA fragments and constitutively expresses E1 proteins (Graham *et al.,* 1977). Since the E3 region is dispensable from the adenovirus genome (Jones and Shenk, 1978), the current adenovirus vectors, with the help of 293 cells, carry foreign DNA in either the E1, the D3 or both regions (Graham and Prevec, 1991). In nature, adenovirus can package approximately 105% of the wild-type genome (Ghosh-Choudhury *et al.,* 1987), providing capacity for about 2 extra kb of DNA. Combined with the approximately 5.5 kb of DNA that is replaceable in the E1 and E3 regions, the maximum capacity of the current adenovirus vector is under 7.5 kb, or about 15% of the total length of the vector. More than 80% of the adenovirus viral genome remains in the vector backbone and is the source of vector-borne cytotoxicity. Also, the replication deficiency of the E1-deleted virus is incomplete. For example, leakage of viral gene expression has been observed with the currently available vectors at high multiplicities of infection (MOI) (Mulligan, 1993).

Helper cell lines may be derived from human cells such as human embryonic kidney cells, muscle cells, hematopoietic cells or other human embryonic mesenchymal or epithelial cells. Alternatively, the helper cells may be derived from the cells of other mammalian species that are permissive for human adenovirus. Such cells include, *e.g*., Vero cells or other monkey embryonic mesenchymal or epithelial cells. As stated above, the preferred helper cell line is 293.

Recently, Racher *et al.,* (1995) disclosed improved methods for culturing 293 cells and propagating adenovirus. In one format, natural cell aggregates are grown by inoculating individual cells into 1 liter siliconized spinner flasks (Techne, Cambridge, UK) containing 100-200 ml of medium. Following stirring at 40 rpm, the cell viability is estimated with trypan blue. In another format, Fibra-Cel microcarriers (Bibby Sterlin, Stone, UK) (5 g/l) is employed as follows. A cell inoculum, resuspended in 5 ml of medium, is added to the carrier (50 ml) in a 250 ml Erlenmeyer flask and left stationary, with occasional agitation, for 1 to 4 h. The medium is then replaced with 50 ml of fresh medium and shaking initiated. For virus production, cells are allowed to grow to about 80% confluence, after which time the medium is replaced (to 25% of the final volume) and adenovirus added at an MOI of 0.05. Cultures are left stationary overnight, following which the volume is increased to 100% and shaking commenced for another 72 h.

Other than the requirement that the adenovirus vector be replication defective, or at least conditionally defective, the nature of the adenovirus vector is not believed to be crucial to the successful practice of the invention. The adenovirus may be of any of the 42 different known serotypes or subgroups A-F. Adenovirus type 5 of subgroup C is the preferred starting material in order to obtain the conditional replication-defective adenovirus vector for use in the present invention. This is because Adenovirus type 5 is a human adenovirus about which a great deal of biochemical and genetic information is known, and it has historically been used for most constructions employing adenovirus as a vector.

As stated above, the typical vector according to the present invention is replication defective and will not have an adenovirus E1 region. Thus, it will be most convenient to introduce the polynucleotide encoding the gene of interest at the position from which the E1-coding sequences have been removed. However, the position of insertion of the construct within the adenovirus sequences is not critical to the invention. The polynucleotide encoding the gene of interest may also be inserted in lieu of the deleted E3 region in E3 replacement vectors as described by Karlsson *et al.,* (1986) or in the E4 region where a helper cell line or helper virus complements the E4 defect.

Adenovirus is easy to grow and manipulate and exhibits broad host range *in vitro* and *in vivo.* This group of viruses can be obtained in high titers, *e.g.,* 10⁹-10¹¹ plaque-forming units per ml, and they are highly infective. The life cycle of adenovirus does not require integration into the host cell genome. The foreign genes delivered by adenovirus vectors are episomal and, therefore, have low genotoxicity to host cells. No side effects have been reported in studies of vaccination with wild-type adenovirus (Couch *et al.,* 1963; Top *et al.,* 1971), demonstrating their safety and therapeutic potential as *in vivo* gene transfer vectors.

Adenovirus vectors have been used in eukaryotic gene expression (Levrero *et al.,* 1991; Gomez-Foix *et al.,* 1992) and vaccine development (Grunhaus and Horwitz, 1992; Graham and Prevec, 1991 ). Recently, animal studies suggested that recombinant adenovirus could be used for gene therapy (Stratford-Perricaudet and Perricaudet, 1991; Stratford-Perricaudet *et al.,* 1990; Rich *et al.,* 1993). Studies in administering recombinant adenovirus to different tissues include trachea instillation (Rosenfeld *et al.,* 1991; Rosenfeld *et al.,* 1992), muscle injection (Ragot *et al.,* 1993), peripheral intravenous injections (Herz and Gerard, 1993) and stereotactic inoculation into the brain (Le Gal La Salle *et al.,* 1993).

***Retroviruses.*** The retroviruses are a group of single-stranded RNA viruses characterized by an ability to convert their RNA to double-stranded DNA in infected cells by a process of reverse-transcription (Coffin, 1990). The resulting DNA then stably integrates into cellular chromosomes as a provirus and directs synthesis of viral proteins. The integration results in the retention of the viral gene sequences in the recipient cell and its descendants. The retroviral genome contains three genes, gag, pol, and env that code for capsid proteins, polymerase enzyme, and envelope components, respectively. A sequence found upstream from the gag gene contains a signal for packaging of the genome into virions. Two long terminal repeat (LTR) sequences are present at the 5' and 3' ends of the viral genome. These contain strong promoter and enhancer sequences and are also required for integration in the host cell genome (Coffin, 1990).

In order to construct a retroviral vector, a nucleic acid encoding a gene of interest is inserted into the viral genome in the place of certain viral sequences to produce a virus that is replication-defective. In order to produce virions, a packaging cell line containing the gag, pol, and env genes but without the LTR and packaging components is constructed (Mann *et al.,* 1983). When a recombinant plasmid containing a cDNA, together with the retroviral LTR and packaging sequences is introduced into this cell line (by calcium phosphate precipitation for example), the packaging sequence allows the RNA transcript of the recombinant plasmid to be packaged into viral particles, which are then secreted into the culture media (Nicolas and Rubenstein, 1988; Temin, 1986; Mann *et al.,* 1983). The media containing the recombinant retroviruses is then collected, optionally concentrated, and used for gene transfer. Retroviral vectors are able to infect a broad variety of cell types. However, integration and stable expression require the division of host cells (Paskind *et al.,* 1975).

A novel approach designed to allow specific targeting of retrovirus vectors was recently developed based on the chemical modification of a retrovirus by the chemical addition of lactose residues to the viral envelope. This modification could permit the specific infection of hepatocytes via sialoglycoprotein receptors.

A different approach to targeting of recombinant retroviruses was designed in which biotinylated antibodies against a retroviral envelope protein and against a specific cell receptor were used. The antibodies were coupled via the biotin components by using streptavidin (Roux *et al.,* 1989). Using antibodies against major histocompatibility complex class I and class II antigens, they demonstrated the infection of a variety of human cells that bore those surface antigens with an ecotropic virus *in vitro* (Roux *et al.,* 1989).

There are certain limitations to the use of retrovirus vectors in all aspects of the present invention. For example, retrovirus vectors usually integrate into random sites in the cell genome. This can lead to insertional mutagenesis through the interruption of host genes or through the insertion of viral regulatory sequences that can interfere with the function of flanking genes (Varmus *et al.,* 1981). Another concern with the use of defective retrovirus vectors is the potential appearance of wild-type replication-competent virus in the packaging cells. This can result from recombination events in which the intact- sequence from the recombinant virus inserts upstream from the gag, pol, env sequence integrated in the host cell genome. However, new packaging cell lines are now available that should greatly decrease the likelihood of recombination (Markowitz *et al.,* 1988; Hersdorffer *et al.,* 1990).

***Herpesvirus.*** Because herpes simplex virus (HSV) is neurotropic, it has generated considerable interest in treating nervous system disorders. Moreover, the ability of HSV to establish latent infections in non-dividing neuronal cells without integrating in to the host cell chromosome or otherwise altering the host cell's metabolism, along with the existence of a promoter that is active during latency makes HSV an attractive vector. And though much attention has focused on the neurotropic applications of HSV, this vector also can be exploited for other tissues given its wide host range.

Another factor that makes HSV an attractive vector is the size and organization of the genome. Because HSV is large, incorporation of multiple genes or expression cassettes is less problematic than in other smaller viral systems. In addition, the availability of different viral control sequences with varying performance (temporal, strength, etc.) makes it possible to control expression to a greater extent than in other systems. It also is an advantage that the virus has relatively few spliced messages, further easing genetic manipulations.

HSV also is relatively easy to manipulate and can be grown to high titers. Thus, delivery is less of a problem, both in terms of volumes needed to attain sufficient MOI and in a lessened need for repeat dosings. For a review of HSV as a gene therapy vector, see Glorioso *et al.* (1995).

HSV, designated with subtypes 1 and 2, are enveloped viruses that are among the most common infectious agents encountered by humans, infecting millions of human subjects worldwide. The large, complex, double-stranded DNA genome encodes for dozens of different gene products, some of which derive from spliced transcripts. In addition to virion and envelope structural components, the virus encodes numerous other proteins including a protease, a ribonucleotides reductase, a DNA polymerase, a ssDNA binding protein, a helicase/primase, a DNA dependent ATPase, a dUTPase and others.

HSV genes form several groups whose expression is coordinately regulated and sequentially ordered in a cascade fashion (Honess and Roizman, 1974; Honess and Roizman 1975; Roizman and Sears, 1995). The expression of α genes, the first set of genes to be expressed after infection, is enhanced by the virion protein number 16, or α-transducing factor (Post *et al.,* 1981; Batterson and Roizman, 1983; Campbell *et al.,* 1983). The expression of β genes requires functional α gene products, most notably ICP4, which is encoded by the α4 gene (DeLuca *et al.,* 1985). γ genes, a heterogeneous group of genes encoding largely virion structural proteins, require the onset of viral DNA synthesis for optimal expression (Holland *et al.,* 1980).

In line with the complexity of the genome, the life cycle of HSV is quite involved. In addition to the lytic cycle, which results in synthesis of virus particles and, eventually, cell death, the virus has the capability to enter a latent state in which the genome is maintained in neural ganglia until some as of yet undefined signal triggers a recurrence of the lytic cycle. Avirulent variants of HSV have been developed and are readily available for use in gene therapy contexts (U.S. Patent 5,672,344).

***Adeno-Associated Virus.*** Recently, adeno-associated virus (AAV) has emerged as a potential alternative to the more commonly used retroviral and adenoviral vectors. While studies with retroviral and adenoviral mediated gene transfer raise concerns over potential oncogenic properties of the former, and immunogenic problems associated with the latter, AAV has not been associated with any such pathological indications.

In addition, AAV possesses several unique features that make it more desirable than the other vectors. Unlike retroviruses, AAV can infect non-dividing cells; wild-type AAV has been characterized by integration, in a site-specific manner, into chromosome 19 of human cells (Kotin and Berns, 1989; Kotin *et al.,* 1990; Kotin *et al.,* 1991; Samulski *et al.,* 1991); and AAV also possesses anti-oncogenic properties (Ostrove *et al.,* 1981; Berns and Giraud, 1996). Recombinant AAV genomes are constructed by molecularly cloning DNA sequences of interest between the AAV ITRs, eliminating the entire coding sequences of the wild-type AAV genome. The AAV vectors thus produced lack any of the coding sequences of wild-type AAV, yet retain the property of stable chromosomal integration and expression of the recombinant genes upon transduction both *in vitro* and *in vivo* (Berns, 1990; Berns and Bohensky, 1987; Bertran *et al.,* 1996; Kearns *et al.,* 1996; Ponnazhagan *et al.,* 1997a). Until recently, AAV was believed to infect almost all cell types, and even cross species barriers. However, it now has been determined that AAV infection is receptor-mediated (Ponnazhagan *et al.,* 1996; Mizukami *et al.,* 1996).

AAV utilizes a linear, single-stranded DNA of about 4700 base pairs. Inverted terminal repeats flank the genome. Two genes are present within the genome, giving rise to a number of distinct gene products. The first, the *cap* gene, produces three different virion proteins (VP), designated VP-1, VP-2 and VP-3. The second, the *rep* gene, encodes four non-structural proteins (NS). One or more of these *rep* gene products is responsible for transactivating AAV transcription. The sequence of AAV is provided by Srivastava *et al.* (1983), and in U.S. Patent 5,252,479.

The three promoters in AAV are designated by their location, in map units, in the genome. These are, from left to right, p5, p19 and p40. Transcription gives rise to six transcripts, two initiated at each of three promoters, with one of each pair being spliced. The splice site, derived from map units 42-46, is the same for each transcript. The four non-structural proteins apparently are derived from the longer of the transcripts, and three virion proteins all arise from the smallest transcript.

AAV is not associated with any pathologic state in humans. Interestingly, for efficient replication, AAV requires "helping" functions from viruses such as herpes simplex virus I and . II, cytomegalovirus, pseudorabies virus and, of course, adenovirus. The best characterized of the helpers is adenovirus, and many "early" functions for this virus have been shown to assist with AAV replication. Low level expression of AAV *rep* proteins is believed to hold AAV structural expression in check, and helper virus infection is thought to remove this block.

***Vaccinia Virus.*** Vaccinia virus vectors have been used extensively because of the ease of their construction, relatively high levels of expression obtained, wide host range and large capacity for carrying DNA. Vaccinia contains a linear, double-stranded DNA genome of about 186 kb that exhibits a marked "A-T" preference. Inverted terminal repeats of about 10.5 kb flank the genome. The majority of essential genes appear to map within the central region, which is most highly conserved among poxviruses. Estimated open reading frames in vaccinia virus number from 150 to 200. Although both strands are coding, extensive overlap of reading frames is not common.

At least 25 kb can be inserted into the vaccinia virus genome (Smith and Moss, 1983). Prototypical vaccinia vectors contain transgenes inserted into the viral thymidine kinase gene via homologous recombination. Vectors are selected on the basis of a tk-phenotype. Inclusion of the untranslated leader sequence of encephalomyocarditis virus, the level of expression is higher than that of conventional vectors, with the transgenes accumulating at 10% or more of the infected cell's protein in 24 h (Elroy-Stein *et al.,* 1989).

In a further embodiment of the invention, the expression construct may be entrapped in a liposome. Liposomes are vesicular structures characterized by a phospholipid bilayer membrane and an inner aqueous medium. Multilamellar liposomes have multiple lipid layers separated by aqueous medium. They form spontaneously when phospholipids are suspended in an excess of aqueous solution. The lipid components undergo self-rearrangement before the formation of closed structures and entrap water and dissolved solutes between the lipid bilayers (Ghosh and Bachhawat, 1991). Also contemplated are lipofectamine-DNA complexes.

Liposome-mediated nucleic acid delivery and expression of foreign DNA *in vitro* has been very successful. Wong *et al.,* (1980) demonstrated the feasibility of liposome-mediated delivery and expression of foreign DNA in cultured chick embryo, HeLa and hepatoma cells. Nicolau *et al.,* (1987) accomplished successful liposome-mediated gene transfer in rats after intravenous injection.

In certain embodiments of the invention, the liposome may be complexed with a hemagglutinating virus (HVJ). This has been shown to facilitate fusion with the cell membrane and promote cell entry of liposome-encapsulated DNA (Kaneda *et al.,* 1989). In other embodiments, the liposome may be complexed or employed in conjunction with nuclear non-histone chromosomal proteins (HMG-1) (Kato *et al.,* 1991). In yet further embodiments, the liposome may be complexed or employed in conjunction with both HVJ and HMG-1. In that such expression constructs have been successfully employed in transfer and expression of nucleic acid *in vitro* and *in vivo,* then they are applicable for the present invention. Where a bacterial promoter is employed in the DNA construct, it also will be desirable to include within the liposome an appropriate bacterial polymerase.

It is worth noting that, though suited well to delivery expression constructs, liposomes also are an excellent vehicle for the delivery of other agents such as oliogsaccharids, glycolipids, polysaccharides, proteins, glycoproteins, chemotherapeutics or radiotherpeutics.

### IX. Examples

The following examples are included to demonstrate preferred embodiments of the invention. It should be appreciated by those of skill in the art that the techniques disclosed in the examples that follow represent techniques discovered by the inventors to function well in the practice of the invention, and thus can be considered to constitute preferred modes for its practice. However, those of skill in the art should, in light of the present disclosure, appreciate that many changes can be made in the specific embodiments that are disclosed and still obtain a like or similar result without departing from the spirit and scope of the invention.

### EXAMPLE I

### E-selectin Induction in Endothelial Cells by Ionizing Radiation

This example shows that when the human endothelial cells HUVEC and HMEC are exposed to ionizing radiation, they exhibit dose and time-dependent increases in the endothelial leukocyte adhesion molecule E-selectin, in the absence of changes in VCAM-1, P-selectin and GMP 140 protein levels.

The acute and subacute clinical manifestations of ionizing radiation mimic the inflammatory response to a number of stimuli. For example, radiation-induced pneumonitis, cystitis, mucositis, esophagitis and dermatitis each demonstrate inflammation as a predominant component (Slauson *et al.,* 1976; Dunn *et al.,* 1986; Ward *et al.,* 1993). Furthermore, ionizing radiation is associated with neutrophilic vasculitis and interstitial inflammation (Narayan, 1982; Slauson *et al.,* 1976; Fajardo and Berthrong, 1988).

The pathophysiology of these sequelae is related to margination of neutrophils in the vasculature and infiltration of the perivascular region after irradiation (Reinhold *et al.,* 1990; Hopewell *et al.,* 1993; Dunn *et al.,* 1986; Matzner *et al.,* 1988). Increased adherence of neutrophils to endothelial cells occurs during acute pulmonary radiation injury (Slauson *et al.,* 1976). Similarly, oxygen radicals also induce human endothelial cells to bind neutrophils (Patel *et al.,* 1991).

Endothelial cells exposed to ionizing radiation respond in a manner analogous to that observed during acute inflammation. This response is associated with leukocyte margination and an increase in vascular permeability. These processes may account for the pathogenesis of radiation injury (Hopewell *et al.,* 1993). An understanding of the pathophysiology of the radiation-mediated inflammatory response will facilitate pharmacologic intervention for these sequelae of radiation therapy.

Endothelial cells rapidly and transiently produce a number of glycoproteins that influence neutrophil binding during the inflammatory reaction (Pober and Cotran, 1990). The potential pathology associated with expression of these proteins on the surface of the endothelium is avoided by their virtual absence prior to stimulation with cytokines or oxidants (Read *et al.,* 1994; Bevilacqua *et al.,* 1989).

Examples of inducible glycoproteins expressed rapidly and transiently within the lumina of blood vessels include E-selectin and P-selectin which have low constitutive expression and serve as receptors for neutrophils and lymphocytes (Bevilacqua, 1993; Pober and Cotran, 1990). This highly restrained transcriptional regulation is in contrast to that of other adhesion molecules such as ICAM-1 and VCAM-1, which have higher constitutive expression that increases further after exposure to cytokines.

The transcriptional regulation of E-selectin is judiciously controlled (Montgomery *et al.,* 1991; Ghersa *et al.,* 1992) because of its pivotal role in the endothelial cell response during inflammation and hypoxia, whereas ICAM induction is regulated less vigorously. Due to the association between oxidant injury and the expression of adhesion molecules on the surface of endothelial cells, the inventors chose to quantify the expression of E-selectin, VCAM, ICAM, and P-selectin in irradiated endothelial cells. The inventors found that the expression of E-selectin and ICAM is increased following irradiation, in a time course analogous to that observed after stimulation with cytokine.

### A. Materials and Methods

### 1. Cell Culture

Primary cultures of human umbilical vein endothelial cells (HUVEC) were prepared from fresh (<24 h old) human umbilical veins transported to the laboratory in sterile buffer at 4°C. The vein was cannulated, filled with 0.2% collagenase, and incubated at 37°C for 15 min. Cells were flushed and complete medium was added, followed by centrifugation at 2000 rpm for 5 min; the supernatant was discarded. The cell pellet was resuspended and maintained in M 199 with 10% fetal calf serum, 10% human serum, Pen/Strep/Amphotericin B solution (Sigma) on gelatin-coated (0.2%) tissue culture dishes at 37°C in 5% CO₂. Confluent cells were subcultured with 0.1 % collagenase 0.01 % EDTA. Cells (HUVEC) were used at third passage; this reduced the number of passenger cells and allow for uniform expression of cellular adhesion molecules.

Endothelial cells from human dermis immortalized with SV40 (HMEC) (Ades *et al.,* 1992) were maintained in endothelial basal medium MCDB 131 (GIBCO/BRL) supplemented with 15% FBS, 10 ng/ml epidermal growth factor (Collaborative Biomedical Products), 1 µg/ml hydrocortisone (Sigma), and Pen/Strep.

### 2. Quantification of adhesion molecules in irradiated endothelial cells

HUVEC and HMEC cells were grown to 90% confluence and irradiated with a GE Maxitron x-ray generator as previously described (Hallahan *et al.,* 1989). Cells were removed from flasks with 0.1% collagenase, 0.01% EDTA, 0.25% BSA and pelleted in 12 × 75 mm polystyrene tubes. The supernatant was discarded and the cells were incubated with primary IgG₁ antibody (mouse anti-human ICAM-1 and E-selectin-1; R&D Systems, Inc., Minneapolis, MN) for 20 min at 4°C.

The cells were then rinsed with isotonic phosphate buffered saline (PBS), pelleted and incubated with FITC-conjugated secondary antibody (goat anti-mouse IgG₁) for 20 min at 4°C. The fluorescently labeled cells were rinsed in PBS and fixed in PBS containing 0.01% paraformaldehyde. Nonspecific binding was evaluated with the use of FITC-conjugated secondary antibody alone and with a lymphocyte specific first-step antibody, anti-CD₁₀, which does not bind to endothelial cells.

Fluorescence activated cell sorting (FACS) analysis was utilized for quantitation of receptor expression of ICAM-1 and E-selectin on HUVEC. The Becton Dickinson FACScanner was used with Lysis II software. Forward and side scatter fluorescence data identified 10,000 viable endothelial cells in each experimental group for unlabeled cells, nonspecific-antibody-labeled cells, ICAM-1-labeled cells and E-selectin-labeled cells. Fluorescence data were then accumulated on each group of 10,000 cells at 530 nm, the wavelength emitted by FITC. The fluorescence data were expressed as histograms of events versus log fluorescence and analyzed in comparison to the autofluorescence of unlabeled cells as well as the fluorescence of baseline ICAM-1 labeled or E-selectin-1 labeled cells as appropriate. During inhibition studies, PKC inhibitors H7 100 nM and staurosporin 10 nM or Phospholipase A2 inhibitors BPB 10 µM or mepacrine 20 µM were added to cell cultures 30 min prior to irradiation.

### 3. RNA analysis

HUVEC cells were grown to 90% confluence and exposed to x-rays (10 Gy, GE Maxitron x-ray generator) as previously described (Hallahan, 1989). RNA was extracted with the single-step guanidinium thiocyanate-phenol/chloroform method (Chomczynski and Sacchi, 1987) at 1 h after irradiation. Control RNA was obtained from nonirradiated cells treated under otherwise identical conditions. RNA was size-fractionated by 1% agarose formaldehyde electrophoresis. Ethidium bromide staining of the RNA demonstrated equal loading of each lane. RNA gels were then transferred to a nylon membrane (Genescreen Plus, New England Nuclear). Northern blots were hybridized to ³²P labeled E-selectin cDNA (Collins *et al.,* 1991) probe followed by autoradiography for 3 days at -85°C with intensifying screens.

### B. Results - Quantitation of adhesion molecules in irradiated HUVEC

To determine the effects of cell adhesion molecules, the inventors expanded primary culture HUVEC from single human umbilical veins that were irradiated, followed by fixation and incubation with antibodies to E-selectin, P-selectin, ICAM-1, and VCAM. The log fluorescence of cells incubated with the antibody to E-selectin shifted by 15 to 32% at 4 h after irradiation. In comparison, the log fluorescence of cells incubated with the antibody to ICAM shifted by 30 to 35% at 20 h after irradiation. However, there was no significant increase in P-selectin or VCAM protein expression following irradiation. Interleukin-1 (IL-1 ) as used as a positive control and shifted the log fluorescence for E-selectin by 43%, ICAM-1 (31%), VCAM-1 (25%), and P-selectin (30%). These data indicate that x-ray induction of CAM's is specific for E-selectin and ICAM whereas IL-1 induces all CAM's quantified.

To examine the time-dependent increase in the radiation-mediated expression of cell adhesion molecules, the inventors irradiated HUVEC with 10 Gy and incubated them with antibody at 2 h intervals after irradiation. E-selectin expression began to increase at 2 h, peaked at 4 to 6 h, and gradually returned to baseline at 20 h. In contrast, ICAM expression remained at baseline levels until 16 h after irradiation, and peak expression occurred at 24 to 36 h following irradiation. HUVEC were then irradiated with doses ranging from 0.5 to 50 Gy and assayed at 4 or 24 h for study of the dose dependence of the x-ray-mediated expression of cell adhesion molecules. E-selectin expression increased at 4 h after exposure to 0.5 Gy and increased in a dose dependent manner up to 20 Gy, where a plateau was reached. When cells were assayed at 24 h following irradiation, only cells treated with 20 Gy or higher doses had a persistent increase in E-selectin expression, whereas those treated with lower doses approximated baseline expression. In contrast, ICAM expression was not increased at x-ray doses below 5 Gy, but demonstratable increases occurred at 24 h when treated with higher doses. These data indicate that E-selectin is induced transiently after low doses of irradiation, whereas ICAM induction requires high radiation doses and is sustained.

### EXAMPLE II

### Ionizing Radiation Mediates Expression of Cell Adhesion Molecules in Distinct Histologic Patterns Within the Lung

Inflammatory cell infiltration of the lung is observed early during radiation-mediated lung injury (Hopwell *et al.,* 1993). Most authors describe the presence of inflammatory cells within the alveolar space, alveolar septum, and perivascular space following irradiation (Ward *et al.,* 1993; Fuks *et al.,* 1995; Reinhold *et al.,* 1990; Steinberg *et al.,* 1993). The histopathologic findings observed during radiation pneumonitis consist of interstitial edema and profuse inflammatory cell infiltration associated with thickening of the alveolar septa (Fuks *et* *al.,* 1995; Travis, 1980). A significant increase in the number of macrophages, granulocytes, and lymphocytes is found in bronchoalveolar lavage fluid from patients as well as mice receiving total-body irradiation (Ward *et al.,* 1993; Steinberg *et al.,* 1993). It has been proposed that the inflammatory mediators released from leukocytes are involved in the pathogenesis of radiation pneumonitis (Graham *et al.,* 1990). The present study addresses the mechanisms by which ionizing radiation mediates the inflammatory response in the lung.

The inventors have previously shown that increased expression of E-selectin and ICAM occurs after x-irradiation of endothelial cells in culture (Hallahan *et al.,* 1995, 1996). The objective of the present study was to determine whether thoracic irradiation alters the histologic pattern of expression of P-selectin, E-selectin and ICAM-1 within the irradiated lung. The inventors treated mice with thoracic irradiation and stained lung sections with antibodies to P-selectin, E-selectin and ICAM-1. X-irradiation induced the expression of E-selectin on the endothelium of larger vessels, whereas there was little expression in the pulmonary microvascular endothelium. ICAM-1 was expressed in the pulmonary capillary endothelium, but minimal expression was observed in the endothelium of larger vessels following thoracic irradiation. P-selectin was present in Weibel-Palade bodies in the endothelium prior to irradiation, and migrated to the vascular lumen within 30 minutes after irradiation. These findings indicate that ionizing radiation alters the histologic pattern of expression of the principle cell adhesion molecules that regulate leukocyte emigration from the circulation.

### A. Methods

### 1. Thoracic irradiation and immunohistochemical staining

Twelve week old C3H mice (Jackson Labs, Bar Harbor, MA) were treated with thoracic irradiation in the dose range and at the time intervals described in the Results section. Lead shields protected the head and abdomen. Mice were euthanized by intraperitoneal injection of barbiturate at 30 minutes, 6 hours, and 1, 2 and 7 days after irradiation. Lungs were fixed in formalin and embedded in paraffin. Paraffin blocks were then sectioned (5 µm thick) and placed on slides. Five micrometer sections of each lung were mounted onto Superfrost Plus slides (Fisher Scientific, Pittsburgh, PA).

### 2. Immunohistochemistry

Lung sections were baked at 60°C for 1 hour, cleared in xylene, and hydrated through a descending alcohol series to distilled water. For E-selectin and CD45 immunostaining, the hydrated sections were incubated with Protease I (Ventana Biotech, Tucson, AZ) for 8 minutes at 42°C. For ICAM immunostaining, the hydrated sections were incubated with Protease II (Ventana Biotech) for 8 minutes at 42°C. After washing briefly in ddH₂O, endogenous activity was blocked by treatment of the sections with 3% hydrogen peroxide in methanol for 20 min. Two tissue sections from each case were then incubated overnight at 4°C at a titer of 2.5 µg/ml for P-selectin and ICAM, and at 7.5 µg/ml for E-selectin (E-selectin [09521D], ICAM [01542D], Pharmingen, San Diego, CA). One slide from each sample was treated in a similar fashion and incubated overnight in normal serum immunoglobulin (Ventana Medical Systems, Tucson, AZ). The immunohistochemical staining was performed on a Ventana Gen¹¹ system (Ventana Medical Systems). The Ventana Gen¹¹ uses an indirect strepavidin-biotin system conjugated with horseradish peroxidase for detecting the immunocomplex and diaminobenzidine as a substrate for localization. The Ventana Gen¹¹ uses a cartridge delivered avidin/biotin blocking kit to block endogenous biotin. The immunostained sections were counterstained with hematoxylin, dehydrated through an ascending alcohol series, cleared, and coverslipped.

### 3. Quantitation of E-selectin expression by use of immunofluoresence.

Lung sections of mice treated with thoracic irradiation were incubated with anti-E-selectin antibody as described above. Following incubation with biotinylated secondary antibody, blocking solution was added for 30 min in a humid chamber at 37°C. Avidin-CY3 (5 µg/mL) was added to 200 µL of blocking buffer and filtered through a 0.2-µm millipore filter. Avidin-fluorochrome solution was added to tissue sections, coverslipped and incubated for 30 min in a humid chamber at 37°C. Coverslips were removed and sections were washed using 4X SSC/0.1% Triton X at 39°C. Slides were counterstained in DAPI and rinsed with 2X SSC for 10 seconds. Slides were then coverslipped with antifade and blotted. Fluorescence was then visualized using UV microscopy and NU200 software as described (Hallahan *et al.,* 1997). Fluorescence intensity of pulmonary vessels was measured by use of NIH Image 1.58 software. DAPI staining of nuclei was used as a control to verify that fluorescence was measured in the same number of cells in each lung section. Fifty nuclei were framed and anti-E-selectin immunofluorescence was determined by use of NIH Image software. Fluorescence intensity was determined for each pixel within the framed cells and the number of fluorescent pixels were counted by use of NIH Image. The increase in the number of pixels showing fluorescence was determined. The mean and SEM of anti-E-selectin immunofluorescence of three lungs was determined for each dose of thoracic irradiation.

### B. Results

### 1. E-selectin expression in pulmonary vascular endothelium of mice treated with thoracic irradiation

To study radiation-induced expression of cell adhesion molecules (CAMs) in the pulmonary vessels of mice, the inventors irradiated the thorax of C3H mice. Six, 24, 48 and 72 hours after irradiation, lungs were fixed and embedded in paraffin. Slides containing lung sections were then incubated with rat anti-mouse E-selectin IgG2a (Pharmingen). Lungs from irradiated mice showed E-selectin expression primarily in the endothelium of large vessels, and minimally in the microvascular endothelium after x-irradiation. E-selectin expression increased at 6 hours and returned to baseline at 72 hours after irradiation.

### 2. ICAM-1 expression in the irradiated lung

To determine the pattern of x-ray-induced ICAM-1 expression, the inventors stained lung sections with rat anti-mouse ICAM-1. Low levels of ICAM-1 expression were found in the pulmonary vascular endothelium prior to irradiation. ICAM-1 expression was increased at 24 hours after irradiation and persisted for 7 days after irradiation. Radiation-induced ICAM-1 expression was increased in the pulmonary capillary endothelium, whereas there was little increase in ICAM-1 staining in the endothelium of larger vessels. Thus, the histologic staining pattern for E-selectin (larger vessels) and ICAM-1 (microvascular endothelium) varied in the irradiated lung.

### 3. Histologic pattern of P-selectin expression in pulmonary vascular endothelium following thoracic irradiation

Prior to thoracic irradiation, P-selectin was constitutively expressed within Weibel-Palade bodies in the endothelium. P-selectin migrates to the vascular lumen within 30 minutes after irradiation. P-selectin then entered the vascular lumen and was undetectable in the pulmonary endothelium at 6 hours after irradiation. At 24 hours after thoracic irradiation, P-selectin accumulated in the pulmonary vascular endothelium and returned to basal levels.

### 4. Dose-dependent increase and E-selectin expression in the irradiated lung

The inventors have determined that a dose dependent increase in ICAM-1 expression in the irradiated lung. To determine whether E-selectin expression is induced following exposure to therapeutic doses of radiation, C3H mice were treated with increasing doses of thoracic irradiation and lungs were sectioned and stained using the anti-E-selectin antibody. CY3-avidin was used for immunofluoresence after biotinylated secondary antibody, because elastin in the pulmonary airways fluoresces at the same wavelength as fluorescein. Anti-E-selectin immunofluoresence in pulmonary blood vessels at 6 hours after x-irradiation was calculated by use of NIH Image software. Mice were treated with 0, 2, and 10 Gy thoracic irradiation. Fluorescence intensity of pulmonary vessels was measured in three experiments. E-selectin staining increased to 5-fold greater than control after 2 Gy, 12-fold in response to 5 Gy and plateaued at 18-fold after 10 Gy or more.

### 5. Radiation Induction of Intercellular Signal Transduction

The inventors have identified several proteins and glycoproteins that contribute to the response of tissues to ionizing radiation. These include ICAM, E-selectin, P-selectin and von Willebrand factor (vWF). These molecules may be regulated at the level of transcription (ICAM and E-selectin) or posttranscription (P-selectin and vWF). P-selectin and VWF are constitutively expressed in the vascular endothelium and are stored in reservoirs or granules within the cytoplasm. These granules rapidly undergo exocytosis to initiate intercellular signal transduction. This rapid process is the initial step in radiation-mediated leukocyte and platelet activation and is the focus of this invention.

### 6. Intercellular Signaling Through Cell Adhesion Molecule Expression

One aspect of the invention is to determine whether intercellular signaling participates in the radiation response. The inventors have developed a model to study this paradigm in which signals are presented at the tissue-blood interface following irradiation to activate nonirradiated circulating cells. The circulation brings untreated cells into the irradiated tissue where they are exposed to intercellular signals. The resting vascular endothelium does not activate circulating leukocytes and platelets, but has the capacity to initiate intercellular signal transduction at the blood interface. The first mechanism of intercellular signaling is a rapid exocytosis of preformed proteins and proteoglycans. In the second mechanism, intercellular signaling is controlled at the level of transcription and is delayed but more prolonged than the rapid form of signaling. This delayed, inducible response in the vascular endothelium occurs within 4 to 24 hours and involves induction of the cell adhesion molecule genes, E-selectin and ICAM-1.

The inventors have found that the E-selectin gene is inducible by ionizing radiation. E-selectin is not constitutively expressed in the endothelium but is induced rapidly after x-radiation of the vascular endothelium. E-selectin induction is controlled at the level of transcription and requires activation of NFkB. Following the transcriptional activation of the E-selectin gene, this cell adhesion molecular is expressed on the luminal surface of the irradiated vascular endothelium. Most recently data indicates that leukocytes are activated within the irradiated blood vessels during the time of E-selectin expression. Moreover, the inventors have found that transcription regulates intercellular signaling between the irradiated endothelium and untreated leukocytes. This invention is not directed toward molecules that are regulated at the level of transcriptions, but will focus on the novel paradigm of rapid exocytosis as a mechanism of intercellular signaling in irradiated tissues.

ICAM-1 is a second radiation-inducible intercellular signaling molecule that is regulated at the level of transcription. The inventors found that ICAM-1 is constitutively expressed at low levels, and expression is markedly induced following transcriptional activation of the ICAM-1 gene. To determine whether ICAM-1 participates in intercellular signaling, the inventors utilize the ICAM-1 blocking antibody which attenuated leukocyte adhesion to the irradiated endothelium. To further examine the role of ICAM-1 expression and radiation-mediated intercellular signaling, the inventor utilize the ICAM-1 knockout mouse. This animal model demonstrated no ICAM-1 induction by ionizing radiation. Leukocyte sequestration in irradiated blood vessels was markedly attenuated. These findings support the view that intercellular signaling plays an important role in the radiation response.

### 7. Translocation of P-Selectin on the Irradiated Endothelium

During the study of CAM expression *in vivo,* the inventors selected proteins that are constitutively expressed in the vascular endothelium to serve as controls for immunohistochemical analysis. Two such proteins are P-selecting and PECAM-1, which are expressed in the endothelium. Immunohistochemical analysis of P-selectin revealed the interesting finding that P-selectin expression in the vascular endothelium was absent at six hours after irradiation. To determine the mechanism of P-selectin depletion in irradiated vascular endothelium, the inventors studies P-selectin expression at earlier time points. This showed that P-selectin was translocated from the cystoplasmic granules to the luminal surface of the vascular endothelium at 30 mins. following irradiation. The inventors utilized an *in vitro* model to further study the mechanism of P-selectin translocation in irradiated endothelial cell cultures. P-selectin immunofluorescence was utilized to localize P-selectin within the cytoplasm and cell membrane. In nonirradiated control cells, P-selectin was localized to Weibel-Palade (WP) bodies throughout the cytoplasm. At 10 mins. following irradiation, Weibel-Palade bodies began translocating by exocytosis to the cell membrane at 30 mins. P-selectin remained tethered to the cell membrane following irradiation, *in vitro.* P-selectin immunofluorescence later diminished in a dose dependent manner. To determine whether P-selectin was released into the medium. P-selectin was not released into the medium. Exocytosis and depletion of P-selectin staining within the endothelial cells was used as a marker for dose-dependent increase in Weibel-Palade body exocytosis. It was found that P-selectin translocation was dose-dependent with a threshold dose of 2 Gy. A plateau-effect was reached after 10 Gy or more. When doses of 5 to 10 Gy were used, depletion of P-selectin began at 60 mins. *in vitro.* The inventors contemplate that higher doses activate apoptosis and proteolytic degradation.

Leukocyte activation within irradiated tissue may not require that direct effect of ionizing radiation on leukocytes. Intercellular signaling through the translocation of P-selectin or IL-8 may be sufficient. The inventors added the monocytoid cells ML-1 to irradiated (10 Gy) HUVEC. TNF production by ML-1 cells was measured by ELISA. The inventors found a two-fold increase in TNF production in ML-1 cells added to x-irradiated endothelial cells as compared to ML-1 cells added to untreated endothelial cell cultures. This model will allow the inventors to add blocking agents such as P-selectin blocking antibody into the cell culture prior to the addition of monocytes and neotrophils.

To determine whether leukocyte adherence to irradiated pulmonary blood vessels occurs after P-selectin translocation, the inventors fixed and sectioned lungs from C56BL6 mice at 4 hrs. after irradiation. Sections were then immunostained with the leukocyte common antigen (CD45) which detects all inflammatory cells within the lung. The inventors found a marked increase in leukocytes within the vessels as compared to animals treated with sham irradiation. Translocated P-selectin may bind to its counterreceptor PGSL on leukocytes to activate expression of cytokines and chemokines and initiate diapedesis and extravasation of leukocytes from the circulation. The inventors will stain sections with antibodies to each of the cytokines and chemokines known to be induced by P-selectin adhesion to monocytes and neutrophils.

To establish an *in vivo* model for the study of intercellular signaling within irradiated tissues, the inventors' have characterized the P-selectin knockout mouse. Immunohistochemical analysis of P-selectin was used to verify that there is no P-selectin staining within the endothelium of these P-selectin deficient mice, and there is no translocation of P-selectin to the luminal surface following irradiation.

### 8. Von Willebrand Factor Release During WPB Exocytosis in Irradiated Endothelial Cells

In addition to P-selectin, von Willebrand factor is a component of Weibel-Palade bodies (WPB) in endothelial cells. This protein binds to the GP-1B and GP-II glycoproteins on platelets to activate platelet aggregation. The inventors' found that platelet aggregation occurs within irradiated blood vessels at 5 h following irradiation. Because of this association between ionizing radiation-induced exocytosis and radiation-induced platelet aggregation, the inventors' quantified release of von Willebrand factor from endothelial cells following irradiation. The inventors found that the quantity of von Willebrand factor released into the medium increases 5-fold following x-irradiation (2 Gy) of endothelial cells within 30 min. Like P-selectin translocation, vWF release from irradiated endothelial cells was diminished when doses of 5 to 10 Gy were used.

### 9. Mechanisms of P-selectin Translocation

The initial step in discerning the potential role of various signal transduction pathways is to add enzyme inhibitors prior to x-radiation. Exocytosis of Weibel-Palade bodies is an active process requiring the translocation of cytoplasmic granules. Previous studies have shown that intracellular signal transduction required for Weibel Palade body translocation involves influx of calcium and protein kinase C activation (calcium, PKC). To determine whether these signaling pathways participate in radiation-mediated Weibel-Palade body exocytosis, the inventors added the intracellular calcium chelator BAPTA and the protein kinase C inhibitor, calphostin, to HUVEC cells prior to irradiation. Cells were then treated with 10 Gy and immunofluorescence was used to localize P-selectin. Irradiated HUVEC cells demonstrate translocation of P-selectin to the cell membrane. HUVEC cells pretreated with BAPTA or calphostin showed attenuation of P-selectin translocation. Conversely, the tyrosin kinase inhibitor, herbimycin A, had no effect on Weibel-Palade exocytosis. To determine whether calcium chelation and protein kinase inhibition prevent all molecular responses to ionizing radiation, the inventors' added BAPTA and calphostin prior to radiation-induction of ICAM-1 expression. BAPTA and calphostin had no effect on x-ray induction of ICAM-1. However, herbimycin A and the NFkB inhibitor ALLN attenuated x-ray induction of ICAM-1. These data demonstrate that intracellular calcium and PKC signal transduction are specific for Weibel-Palade body exocytosis and have no effect on ICAM gene induction following irradiation.

### 10. Protein Kinase C (PKC) Activation By Ionizing Radiation

Protein kinase C (PKC) is a family of related phosphotransferase enzymes that participate in signal transduction. The inventors have found that ionizing radiation rapidly activates the phosphotransferase activity of PKC. These data demonstrate that PKC is activated within 30 seconds of irradiation. This family of enzymes participates in x-ray induction of genes that participate in intracellular signaling. Examples of radiation-inducible genes that require PKC activation and activate nonirradiated cells include E-selectin and TNF. The mechanisms by which ionizing radiation activates PKC include the production of DAG, arachanonic acid, and calcium.

### C. Discussion

The purpose of this study was to characterize the radiation-mediated induction of cell adhesion molecules in vivo. E-selectin and ICAM-1 are radiation-inducible genes that are expressed in vascular endothelial cells (Hallahan *et al.,* 1995, 1996), and are associated with recruitment of inflammatory cells into sites of tissue injury (Collins, 1995; Springer, 1994). The time course and dose-dependent increase in E-selectin expression on irradiated endothelial cells in culture is similar to that observed in the pulmonary vascular endothelial cells of mice treated with thoracic irradiation (Hallahan *et al.,* 1995). The histologic pattern of expression of E-selectin in the irradiated lung differed from that observed for ICAM-1 and P-selectin. ICAM-1 was primarily expressed in the endothelium of the pulmonary microvasculature, in contrast to E-selectin which was expressed primarily in the endothelium of larger vessels. P-selectin was expressed from the endothelium of larger vessels, and was never expressed in the microvasculature. This contrasting pattern of expression may be associated with the function of each of these CAMs. The selectins slows the velocity of leukocytes (Springer, 1994), whereas ICAM-1 contributes to leukocyte extravasation from the microvasculature (Collins, 1995; Luscinskas *et al.,* 1991; Smith *et al.,* 1988). This pattern is associated with the histologic pattern of inflammatory cell infiltration into alveolar septa (Fuks *et al.,* 1995; Franko *et al.,* 1997).

The inventors also have determined that a dose dependent increase in ICAM-1 expression in x-irradiated pulmonary vascular endothelium exists. The present study demonstrates that E-selectin expression in the pulmonary vascular endothelium increases in dose and time dependent manners following thoracic irradiation. E-selectin expression increases after a dose as low as 2 Gy and plateaus when doses above 10 Gy are used. This threshold dose was similar to that for ICAM-1, but the fold-increase in immuno-fluorescence was greater for E-selectin than ICAM. This may be due to the higher basal expression of ICAM in the lung as compared to E-selectin.

The radiation-mediated increase in E-selectin and ICAM-1 mRNA expression requires no de novo protein synthesis and is blocked by the transcription inhibitor actinomycin D (Hallahan *et al.,* 1995, 1996). The 587-base-pair segment of the 5' regulatory region of the E-selectin gene and the 1.2 kb segment of the 5' regulatory region of the ICAM-1 gene are both sufficient to regulate activation of radiation-induced transcription (Hallahan *et al.,* 1995, 1996). The inventors also have shown that deletion of the NFκB binding region within the E-selectin promoter eliminates radiation induction of this CAM gene (Hallahan *et al.,* 1995). It is noted that radiation induction of cell adhesion molecules is distinct from that observed after cytokine stimulation in that radiation induction was limited to E-selectin and ICAM, but did not occur with VCAM-1 (Hallahan *et al.,* 1995). Furthermore, production of TNF and IL-1 following irradiation of macrophages occurs 12 to 18 hours after irradiation (Hallahan *et al.,* 1989), whereas E-selectin gene expression occurs 2 hours after irradiation (Hallahan *et al.,* 1995). Moreover, NFκB binding to the E-selectin promoter occurs within 10 minutes after irradiation, indicating that this molecular response is rapid (Hallahan *et al.,* 1995). Taken together, these data suggest that TNF and IL-1 are not necessary for radiation-mediated E-selectin induction in the vascular endothelium.

### EXAMPLE III

### P-selectin Translocation to Endothelial Cell Lumen In Vivo

Many methods are available to induce P-selectin translocation to tumor vasculature endothelial cell lumen *in vivo,* as exemplified by the following.

To induce P-selectin translocation *in vivo* one would, generally, expose the cells to ionizing radiation at a dose sufficient to cause the P-selectin to translocate. When using the invention to treat cancer, the tumor site itself will generally be subjected to x-ray irradiation, allowing for spatially controlled P-selectin translocation, which is a particular advantage of this invention.

Effective doses are found to be between about 0.5 Gy to about 50 Gy. The inventors contemplate that the tumors of cancer patients will be irradiated with between about 2 Gy up to a total dose of about 20 Gy. Daily irradiation can also be used to persistently translocate P-selectin. Examples of ionizing radiation include not only x-rays, but also γ-irradiation. Clincially appropriate doses are 1-20 Gy.

### EXAMPLE IV

### Antibodies to P-selectin

Where antibodies to P-selectin are used, it is preferred to use monoclonal antibodies (MAbs), as may be obtained from a variety of commercial sources. Should one wish to prepare a novel anti-P-selectin MAb, techniques are readily available. Means for preparing and characterizing antibodies are well known in the art (*see, e.g.,* Antibodies: A Laboratory Manual, Cold Spring Harbor Laboratory, 1988; incorporated herein by reference). The methodological references in this area are supplemented by the specific teachings of, *e.g*., Mulligan *et al.* (1991) and Norton *et al.* (1993).

The methods for generating MAbs generally begin along the same lines as those for preparing polyclonal antibodies. Briefly, a polyclonal antibody is prepared by immunizing an animal with an P-selectin immunogenic composition and collecting antisera from that immunized animal. A wide range of animal species can be used for the production of antisera. Typically the animal used for production of anti-antisera is a rabbit, a mouse, a rat, a hamster, a guinea pig or a goat. Because of the relatively large blood volume of rabbits, a rabbit is a preferred choice for production of polyclonal antibodies.

As is well known in the art, a given composition may vary in its immunogenicity. It is often necessary therefore to boost the host immune system, as may be achieved by coupling a peptide or polypeptide immunogen to a carrier. Exemplary and preferred carriers are keyhole limpet hemocyanin (KLH) and bovine serum albumin (BSA). Other albumins such as ovalbumin, mouse serum albumin or rabbit serum albumin can also be used as carriers. Means for conjugating a polypeptide to a carrier protein are well known in the art and include glutaraldehyde, m-maleimidobencoyl-N-hydroxysuccinimide ester, carbodiimyde and bis-biazotized benzidine.

As is also well known in the art, the immunogenicity of a particular immunogen composition can be enhanced by the use of non-specific stimulators of the immune response, known as adjuvants. Exemplary and preferred adjuvants include complete Freund's adjuvant (a non-specific stimulator of the immune response containing killed *Mycobacterium tuberculosis),* incomplete Freund's adjuvants and aluminum hydroxide adjuvant.

The amount of immunogen composition used in the production of polyclonal antibodies varies upon the nature of the immunogen as well as the animal used for immunization. A variety of routes can be used to administer the immunogen (subcutaneous, intramuscular, intradermal, intravenous and intraperitoneal). The production of polyclonal antibodies may be monitored by sampling blood of the immunized animal at various points following immunization. A second, booster injection, may also be given. The process of boosting and titering is repeated until a suitable titer is achieved. When a desired level of immunogenicity is obtained, the immunized animal can be bled and the serum isolated and stored, and/or the animal can be used to generate MAbs.

MAbs may be readily prepared through use of well-known techniques, such as those exemplified in U.S. Patent 4,196,265. Typically, this technique involves immunizing a suitable animal with a selected immunogen composition. In this case, the immunogen will generally be a purified or partially purified P-selectin protein, polypeptide or peptide, or even a population of cells known to express P-selectin.

The immunizing composition, whether purified protein- or cell-based, is administered in a manner effective to stimulate antibody producing cells. Rodents such as mice and rats are preferred animals, however, the use of rabbit, sheep frog cells is also possible. The use of rats may provide certain advantages (Goding, 1986, pp. 60-61), but mice are preferred, with the BALB/c mouse being most preferred as this is most routinely used and generally gives a higher percentage of stable fusions.

Following immunization, somatic cells with the potential for producing antibodies, specifically B lymphocytes (B cells), are selected for use in the MAb generating protocol. These cells may be obtained from biopsied spleens, tonsils or lymph nodes, or from a peripheral blood sample. Spleen cells and peripheral blood cells are preferred, the former because they are a rich source of antibody-producing cells that are in the dividing plasmablast stage, and the latter because peripheral blood is easily accessible. Often, a panel of animals will have been immunized and the spleen of animal with the highest antibody titer will be removed and the spleen lymphocytes obtained by homogenizing the spleen with a syringe. Typically, a spleen from an immunized mouse contains approximately 5 × 10⁷ to 2 × 10⁸ lymphocytes.

The antibody-producing B lymphocytes from the immunized animal are then fused with cells of an immortal myeloma cell, generally one of the same species as the animal that was immunized. Myeloma cell lines suited for use in hybridoma-producing fusion procedures preferably are non-antibody-producing, have high fusion efficiency, and enzyme deficiencies that render then incapable of growing in certain selective media which support the growth of only the desired fused cells (hybridomas).

Any one of a number of myeloma cells may be used, as are known to those of skill in the art (Goding, pp. 65-66, 1986; Campbell, pp. 75-83, 1984. cites). For example, where the immunized animal is a mouse, one may use P3-X63/Ag8, X63-Ag8.653, NS1/1.Ag 4 1, Sp210-Ag14, FO, NSO/U, MPC-11, MPC11-X45-GTG 1.7 and S194/5XX0 Bul; for rats, one may use R210.RCY3, Y3-Ag 1.2.3, IR983F and 4B210; and U-266, GM1500-GRG2, LICR-LON-HMy2 and UC729-6 are all useful in connection with human cell fusions.

One preferred murine myeloma cell is the NS-1 myeloma cell line (also termed P3-NS-1-Ag4-1), which is readily available from the NIGMS Human Genetic Mutant Cell Repository by requesting cell line repository number GM3573. Another mouse myeloma cell line that may be used is the 8-azaguanine-resistant mouse murine myeloma SP2/0 non-producer cell line.

Methods for generating hybrids of antibody-producing spleen or lymph node cells and myeloma cells usually comprise mixing somatic cells with myeloma cells in a 2:1 proportion, though the proportion may vary from about 20:1 to about 1:1, respectively, in the presence of an agent or agents (chemical or electrical) that promote the fusion of cell membranes. Fusion methods using Sendai virus have been described by Kohler and Milstein (1975; 1976), and those using polyethylene glycol (PEG), such as 37% (v/v) PEG, by Gefter *et al.* (1977). The use of electrically induced fusion methods is also appropriate (Goding pp. 71-74, 1986).

Fusion procedures usually produce viable hybrids at low frequencies, about 1 × 10⁻⁶ to 1 × 10⁻⁸. However, this does not pose a problem, as the viable, fused hybrids are differentiated from the parental, unfused cells (particularly the unfused myeloma cells that would normally continue to divide indefinitely) by culturing in a selective medium. The selective medium is generally one that contains an agent that blocks the *de novo* synthesis of nucleotides in the tissue culture media. Exemplary and preferred agents are aminopterin, methotrexate, and azaserine. Aminopterin and methotrexate block *de novo* synthesis of both purines and pyrimidines, whereas azaserine blocks only purine synthesis. Where aminopterin or methotrexate is used, the media is supplemented with hypoxanthine and thymidine as a source of nucleotides (HAT medium). Where azaserine is used, the media is supplemented with hypoxanthine.

The preferred selection medium is HAT. Only cells capable of operating nucleotide salvage pathways are able to survive in HAT medium. The myeloma cells are defective in key enzymes of the salvage pathway, *e.g*., hypoxanthine phosphoribosyl transferase (HPRT), and they cannot survive. The B cells can operate this pathway, but they have a limited life span in culture and generally die within about two weeks. Therefore, the only cells that can survive in the selective media are those hybrids formed from myeloma and B cells.

This culturing provides a population of hybridomas from which specific hybridomas are selected. Typically, selection of hybridomas is performed by culturing the cells by single-clone dilution in microtiter plates, followed by testing the individual clonal supernatants (after about two to three weeks) for the desired reactivity. The assay should be sensitive, simple and rapid, such as radioimmunoassays, enzyme immunoassays, cytotoxicity assays, plaque assays, dot immunobinding assays, and the like. Screening hybridomas for those that react exclusively with P-selectin is also described by Mulligan *et al.* (1991).

The selected hybridomas would then be serially diluted and cloned into individual antibody-producing cell lines, which clones can then be propagated indefinitely to provide MAbs. The cell lines may be exploited for MAb production in two basic ways. A sample of the hybridoma can be injected (often into the peritoneal cavity) into a histocompatible animal of the type that was used to provide the somatic and myeloma cells for the original fusion. The injected animal develops tumors secreting the specific monoclonal antibody produced by the fused cell hybrid. The body fluids of the animal, such as serum or ascites fluid, can then be tapped to provide MAbs in high concentration. The individual cell lines could also be cultured *in vitro,* where the MAbs are naturally secreted into the culture medium from which they can be readily obtained in high concentrations. MAbs produced by either means may be further purified, if desired, using filtration, centrifugation and various chromatographic methods such as HPLC or affinity chromatography.

A molecular cloning approach could also be used to generate monoclonals. For this, combinatorial immunoglobulin phagemid libraries are prepared from RNA isolated from the spleen of the immunized animal, and phagemids expressing appropriate antibodies are selected by panning, *e.g.,* using cells expressing P-selectin and control cells. The advantages of this approach over conventional hybridoma techniques are that approximately 10⁴ times as many antibodies can be produced and screened in a single round, and that new specificities are generated by H and L chain combination which further increases the chance of finding appropriate antibodies.

Functional antibody fragments, *e.g.,* Fab', Fab, F(ab')₂, Fv and scFv fragments, can also be employed, so long as the antibody or fragment exhibits the desired binding specificity for E-selectin. Methods for generating active antibody fragments are well known in the art. Means for making radiolabeled Fab'-Fabs are also known (Le Duossal *et al.,* 1992). Methods for employing anti-P-selectin fragments, in contexts other than with radiation-induction, have also been described, which can now be utilized in the present invention.

Monoclonal antibodies to human P-selectin can be labeled, *e.g*., using ¹²⁵I, ¹³¹I, or any other desired agent, using previously described techniques. For example, using the oxidative reagent from Iodogen (Fraker and Speck, 1978) or the lactose periodase from LKB (Klein, 1989). Harrison also described astatine-211-1 abeling of MAbs. Mehta *et al.* (1990) describe methods for coupling Yttrium-90 (90Y) to diethylene triaminepenta acetic acid (DTPA) and then covalently linking this to a monoclonal antibody.

Deshpande *et al.* (1988) also describe an effective technique for use in labeling antibodies with copper-67. 67Cu is one of the most promising radiometals for radioimmunotherapy because of its 61.5 hr physical half-life, abundant beta particles, and gamma emissions suitable for imaging. However, 67Cu is readily transferred from the usual chelates of EDTA or DTPA to albumen. Deshpande *et al.* (1988) developed a new macrocycle (6-p-nitrobenzyl-TETA) to chelate copper. The bifunctional chelating agent p-bromoacetamidobenzyl-TETA was conjugated to a monoclonal antibody without significantly altering its immunoreactivity.

Many methods are also available for use in linking MAbs to anti-tumor proteins, such as, *e.g.*, neocarzinostatin (NCS). For example, Luders *et al.* (1985) used the heterobifunctional reagent N-succinimidyl 3-(2-pyridyldithio)-propionate (SPDP). The conjugate retained both the reactivity of the antibody and the toxicity of the drug. Scott *et al.* (1987) described a method for preparing an immunotoxin-like compound composed of a monoclonal antibody linked by a disulfide bond to the ribosome-inactivating protein gelonin.

Braslawsky *et al.* (1990) also describe methods for chemically coupling adriamycin to monoclonal antibodies. In these studies, immunoconjugates were prepared by linking to the MAb and ADM derivative, Adriamycin 13-3-(2-pyridyldithio) propionyl]hydrazone (ADM-HZN), which releases ADM under mild acidic conditions.

Many methods are also available for use in linking MAbs to gene segments, such as gene segments encoding anti-tumor proteins, thrombolytic agents and anticellular agents that kill or suppress the growth or cell division of disease-associated endothelial cells. Examples of effective thrombolytic agents are streptokinase and urokinase. Bode *et al.* (1985) described an antibody-directed urokinase that functioned as a specific fibrinolytic agent. The urokinase-MAb conjugate retained the original binding specificity of the antibody and showed 100-fold increased fibrinolysis *in vitro* when compared to unmodified urokinase. This technology could also be used in connection with the present invention following covalently coupling urokinase to a monoclonal antibody against E-selectin.

Collen *et al.* (1990) also reported on the thrombolytic and pharmacokinetic properties of a conjugate of recombinant single-chain urokinase-type plasminogen activator with a monoclonal antibody. This chemical conjugate between recombinant single-chain urokinase-type plasminogen activator (rscu-PA) and a murine monoclonal antibody was produced by cross-linking with SPDP.

In pre-clinical animal studies designed for optimization, antibodies to mouse PE-selectin are contemplated to be useful. Animal models designed to allow optimization of anti-cancer strategies are routinely employed prior to translating the results to a clinical environment. Such animal models are generally held to be predictive of results in humans.

The inventors will show that ionizing radiation increases binding of P-selectin ligand-conjugated-cytotoxins in cancer xenografts in mice. Antibodies to mouse P-selectin are used here as the endothelial cells within experimental tumors will be of mouse origin. Monoclonal antibodies to murine P-selectin and glycyrrhizin-albumin conjugates are bound to ¹³¹I and injected into the tail veins of mice 4 h after ionizing radiation exposure. ¹³¹I localization to irradiated tumors and tissues will be characterized using total body scanning as previously described (DeSombre *et al.,* 1990; Lessem *et al.,* 1980) and scintillation counting.

### EXAMPLE V

### Targeting P-selectin with Antibody Conjugates

Pre-clinically, the feasibility of using ionizing radiation to increase *in vivo* transfection of tumors will be proven using a predictive animal model. The inventors have already optimized the use of liposomes to transfect human tumor xenografts in nude mice. Human breast cancer xenografts in nude mice will be used to determine which model is most or best suited to study efficacy. Xenografts and analytical techniques have been described (Brunner *et al.,* 1993; Miller *et al.,* 1994; Weichselbaum *et al.,* 1994; Pai *et al.,* 1992).

Binding of P-selectin-targeted conjugates to the cells in the irradiated tumor vasculature will be compared to control unirradiated tumors in the opposite hind limb. Other controls will include tumors that are irradiated and contacted with, *e.g*., Lipofectin liposomes without P-selectin ligands. Furthermore, mice without tumors will receive local irradiation to lung, liver and hind limb to determine whether radiation-induction of P-selectin occurs in various tissues.

The Mulligan *et al.* (1991) methodology may be used in the methods of this invention, following radiation-induction of P-selectin translocation. These authors showed that certain MAbs *blocked in vitro* adherence of neutrophils to TNF alpha-treated endothelial cells and the killing of TNF alpha-treated rat endothelial cells by phorbol ester activated neutrophils.

The methodology and studies reported by Williams *et al.* (1990) may also be used in conjunction with the present invention. These authors used radiolabeled antibodies to target tumor antigens. However, the strategy will be essentially the same, once P-selectin has been translocated. The regimens described by Williams *et al.* (1990) are considered to be a suitable guideline for glioma treatment.

In a clinical setting, anti-human P-selectin is conjugated to a cytotoxic agent, such as pseudomonas exotoxin, staph endotoxin, ricin, a conventional chemotherapeutic agent, or a radioligand, such as ¹³¹I, and then given to a patient. For example, ¹³¹I bound murine monoclonal anti-human P-selectin is administered at a dose of 1 mCi 4 h after irradiation. Patients then undergo gamma camera scanning to quantify the localization of ¹³¹I. This schedule of delivery is to be repeated daily to a total dose of 20 Gy from external irradiation, and the dose will be escalated by 4 Gy in cohorts of 3 patients unless a major toxicity is observed when 3 more patients will be added at that dose level. Alternatively, bispecific antibody may be employed to target a virus, such as herpesvirus, retrovirus, adenovirus, adeno-associated virus, vaccinia virus or a polyoma virus to the vasculature.

### EXAMPLE VI

### Oligosaccharides that Bind to P-selectin

Many oligosaccharides are known that bind to selectin. Most agents that bind E-selectin also will bind P-selectin, and *vice versa.* A preferred oligosugar is PGSL, given its specific affinity for P-selectin. Still further ones can now be developed following the teaching of the present disclosure in combination with the knowledge in the art. All such oligosaccharides are contemplated for use in the present invention.

Some of the best studied P-selectin-binding oligosaccharides are the sialyl Lewis X/A compounds (Walz *et al.,* 1990; Phillips *et al.,* 1990; Berg *et al.,* 1991). Indeed, there is general agreement that the ligands for P-selectin include sialylated, polylactosamine, oligosaccharides, with a fucose on the first (sialyl-Lewis^{x} (sLe^{x}) epitope,
Sia(α2-3)Gal((β1-4)[Fuc(α1-3)]GlcNAc-R) or second [VIM-2 epitope,
Sia(α2-3)Gal(β1-4)GlcNAc(β1-3)Gal(β1-4)[Fuc(α1-3)]GlcNAc-R] *N*-acetyl-glucosamine from the nonreducing end.

Sialyl Lewis X/A compounds may either be synthesized or purchased. For example, 2-3 sLe^{x} hexa glycolipids and 2-3 sLe^{x} pentaglycolipids may be synthesized as described by Kameyama *et al.* (1991), Hasegawa *et al.* (1994) and Yoshida *et al.* (1993). 2-3 sLe^{x} tetra compounds can be obtained from Glycomed (Alameda, CA). 2-3 sLe^{x} hexa glycolipid is sia(α2-3)Gal(β1-4) [Fuc (α1-3) ] GlcNAc (β1-3) Gal (β1-4) Glc-ceramide; 2-3 sLe^{x} penta glycolipid is Sia (α2-3)Gal((β1-4) [Fuc(α1-3)] GlcNAc (β1-3)Gal-ceramide; 2-3 sLe^{a} hexa is Sia (α2-3) Gal (β1-3) [Fuc (α1-4) ]GlcNAc(βI-3)Gal(βi-4)Glc; and 2-3 sLe^{x} tetra is Sia(α2-3)Gal (β1-4) [Fuc (α1-3) ]GlcNAc.

Tyrrell *et al.* (1991) analyzed the structural requirements for carbohydrates ligand of selectins and reported a detailed investigation into the minimum structural requirements for E-selectin carbohydrate recognition. Using both direct binding and inhibition studies, Tyrrell *et al.* (1991) demonstrated that the sialyl Lewis^{x} tetrasaccharides
Sia(α2-3)(Gal(β1-4)[Fuc(α1-3)]GlcNAc, and Sia(α2-3)Gal(β1-4)[Fuc(α1-3)]Glc are the smallest oligosaccharides recognized by the lectin. In addition, an oligosaccharide containing the sialyl Lewis^{a} epitope was also recognized, but less avidly

The Tyrrell *et al.* (1991) model, together with NMR data on the conformation of oligosaccharides in solution, lead to a hypothesis for the binding face of oligosaccharides interacting with selectins. The Tyrrell model incorporates the following conclusions: (i) fucose and sialic acid are required for selectin binding; (ii) 2-6-linked sialic acid is not recognized; (iii) removal of carbons 8 and 9 on the sialic acid or substitution of an *N-*glycolyl for the *N*-acetyl of the sialic acid residue do not effect binding; (iv) removal of the N-acetyl group from the N-acetylglucosamine does not reduce binding; (v) reduction of 2-3 sLe^{x}(Glc) does not substantially reduce its inhibitory activity. The 2-3 sLe^{x} and 2-3 sLe^{a} oligosaccharides, in their minimum-energy conformation, present the same orientation of sialic acid, galactose, and fucose.

When linked 2-6, the sialic acid residue assumes a much different conformation in space. In the Tyrrell *et al.* (1991) model the glycerol side chain (carbons 7, 8, and 9) and the *N*-acetyl group of the sialic acid are oriented away from the recognized face of the oligosaccharide. In addition, opening the reducing terminal sugar results in three minimum-energy configurations, one of which closely matches the original configuration. The carboxyl group of the sialic acid, the 4- and 6-hydroxyls of galactose, and the 2-, 3-, and 4-hydroxyls of fucose are involved in recognition.

Selectin-binding oligosaccharides that match the Tyrrell *et al.* (1991) criteria are contemplated to be particularly useful as targeting agents in the invention. The oligosaccharides described in the papers by Yuen *et al.* (1992; 1994), Nelson *et al.* (1993), Green *et al.* (1992), Kojima *et al.* (1992), Munro *et al.* (1992); Mulligan *et al.* (1993) and Narasinga Rao *et al.* (1994) may also be employed as E-selectin-binding oligosaccharides, according to the following reasoning.

Yuen *et al.* (1992) identified another class of oligosaccharides that bind to the human selectins using oligosaccharides on an ovarian cystadenoma glycoprotein. This was achieved by application of neoglycolipid technology to oligosaccharides released from the glycoprotein by mild alkaline β-elimination. Oligosaccharides were conjugated to lipid, resolved by thin-layer chromatography, and tested for binding by Chinese hamster ovary cells which had been transfected to express the full-length E-selectin molecule. Several components with strong selectin binding activity were revealed among acidic oligosaccharides. The smallest among these was identified as an equimolar mixture of the Le^{a-} and Le^{x}/SSEA-1-type fucotetrasaccharides sulfated at position 3 of outer galactose (Yuen *et al.,* 1992).

The binding activity of this is substantially greater than those of lipid-linked Le^{a} and Le^{x}/SSEA-1 sequences and is at least equal, if not superior, to that of the 3'-sialyl-Le^{x}/SSEA-1 glycolipid analogue. Therefore, this compound is also particularly contemplated for use in the present invention.

The 3'-sulphated Lea/Le^{x} type tetrasaccharides have also been shown to be more strongly bound to selectins than 3'-sialyl analogues (Green *et al.,* 1992). A considerable-binding was observed to the 3'-sulphated oligosaccharide backbone in the absence of fucose but not to a 3'-sialyl analogue or fuco-oligosaccharide analogues lacking sulphate or sialic acid. These studies highlight the relative importance of sulphate in the adhesive specificity of this protein and establish that 3'-sulphated Le^{a}/Le^{x} tetrasaccharides are effective ligands for selectin binding. The inventors also envision using the tetrasaccharides described by Green *et al.* (1992) in the targeting embodiments described herein.

A further series of synthetic oligosaccharides based on sialyl Lewis^{x} (sLe^{x}) and sialyl Lewis a (sLe^{a}) were used to study the binding interactions of selectins by Nelson *et al.* (1993). These authors found that solution-phase sLe^{x} is a more potent blocker of E-selectin than is sLe^{x}. Furthermore, addition of an aliphatic aglycone in combination with an amino substitution on the GlcNAc of sLe^{a} resulted in a compound with 36-fold higher activity than sLe^{x}, as measured in a competitive binding assay (Nelson *et al.,* 1993).

More specifically, Nelson *et al.* (1993) showed that the attachment of an 8-methoxycarbonyloctyl aglycone in a β linkage to the anomeric carbon of the GlcNAc of sLe^{x} or sLe^{a} increased their blocking (*i.e.,* binding) activity nearly twofold. Replacement of the 2-*N*-acetyl substituent of the GlcNAc by an azido or amino group resulted in substantial increases in activity, with the most potent inhibitor being amino substituted sLe^{a}, which was 36-fold more active (IC₅₀ = 21±3 µM) than the reducing tetrasaccharide sLe^{x}. Aglycone and amino substituted sLe^{a} compounds are thus also contemplated for use in selectin binding and targeting following site specific induction.

Yuen *et al.* (1994) also reported that the sulfated Le^{a} tetra- and pentasaccharides are particularly potent selectin ligands. The inhibitory activity of the sulfated Le^{a} pentasaccharide was reportedly substantially greater than that of the sialyl-Le^{x} trisaccharide, which is currently the most widely used inhibitor of E-selectin binding: 45-, 35-, or 15-fold greater depending on whether adhesion is to sialyl-Le^{a}, sulfated Le^{a}, or sialyl-Le^{x} pentasaccharides, respectively. These findings can be utilized in designing second generation selectin binding agents, as may be used in the present invention.

Mulligan *et al.* (1993) have described the protective effects of sialylated oligosaccharides in immune complex-induced acute lung injury. These authors showed that tetra- and pentasaccharide derivatives of sialyl Lewis^{x} oligosaccharides derived from fucosyl transferase-expressing cells, or generated synthetically, protected against acute lung damage after deposition of immunoglobulin (Ig)G or IgA immune complexes.

In the IgG immune complex model of lung injury, which is E-selectin dependent, sialyl Lewis^{x} oligosaccharide preparations provided dose-dependent protective effects, as assessed by changes in lung vascular permeability and hemorrhage. Protective effects were associated with diminished tissue accumulation of neutrophils in lungs. Morphological assessment revealed reduced physical contact of neutrophils with the pulmonary vascular endothelium and reduced tissue accumulation of neutrophils (Mulligan *et al.,* 1993). These studies show that sialyl Lewis^{x} oligosaccharides are safe for use in therapeutic embodiments.

Narasinga Rao *et al.* (1994) used conformational energy computations, high field NMR, and structure-function studies to define distance parameters of critical functional groups of sLe^{x}. This sLe^{x} pharmacophore was used to search a three-dimensional data base of chemical structures. Compounds that had a similar spatial relationship of functional groups were tested as inhibitors of selectin binding. Glycyrrhizin, a triterpene glycoside, was identified and found to block selectin binding to sLe^{x} *in vitro.* Using technology such as that described by Narasinga Rao *et al.* (1994), the inventors contemplate that other E-selectin binding agents may be identified, expanding the group of compounds available for use in this invention.

Narasinga Rao *et al.* (1994) also substituted different sugars for the glucuronic acids of glycyrrhizin and found the L-fucose derivative to be the most active *in vitro* and *in vivo.* A C-fucoside derivative, synthesized on a linker designed for stability and to more closely approximate the original sle^{x} pharmacophore, resulted in an easily synthesized, effective selectin blocker with anti-inflammatory activity.

The Narasinga Rao approach is based generally on the finding that only the charged group of sialic acid is essential to allow binding of carbohydrate epitopes to selectins (Tyrrell *et al.,* 1991). In searching the Fine Chemicals Directory data base using MACCS three-dimensional software against a pharmacophore derived from solution conformations of sle^{x}, compounds that matched the pharmacophore definition were identified. Such an approach may allow less expensive selectin-binding components to be identified.

In a first approximation, Narasinga-Rao *et al.* constructed a pharmacophore using the spatial dispositions of the carboxylate group of the sialic acid and the vicinal hydroxyls of fucose, which were found to be separated by 10-12 Å. In the initial two-dimensional search of ~75,000 compounds in the data base, nearly 400 compounds were identified. However, by modifying the query to more closely match the pharmacophore, the number of compounds dropped to 23 (Narasinga-Rao *et al.,* 1994).

An examination of this list revealed that there were (i) multiple redundancy (*i.e.* different salt forms), (ii) organic salts, (iii) sugar-uronic acids, and (iv) modified forms of certain core structures within the list. Therefore, the list was reduced to 9 potential compounds for screening as inhibitors of selectin binding to immobilized sLe^{x} (Narasinga-Rao *et al.,* 1994).

In addition to glycyrrhizin and glycyrrhizic acid (NH⁺4), natural products from licorice, other compounds blocked selectin binding to sLe^{x} (Narasinga-Rao *et al.,* 1994). Of the other compounds identified in this search, the effective ones include: carminic acid, which also blocked selectin binding at concentrations comparable to those of glycyrrhizin; α-Hederin, which showed a weaker activity, inhibiting at 2-3 mM concentrations; carmine; picrocarmine; carmine ammonia; rhein-8-glucoside; kasugamycin hydrochloride; kasugamycin; meglumine diatrizoate; [*ring*-¹⁴C]Chlorhexidine; trigalacturonic acid; escin; metrizoic acid (meglumine salt); and *N*-(α-Rhamnopyranosyloxy hydroxyphosphonyl)- Leu-Trp (sodium salt). All of these compounds have potential for use in the invention, following proper safety and efficacy testing, and are commercially available from one or more of the following vendors: Aldrich, Apin, BDH, Calbio, Fluka, ICN K&K Laboratories, Inc., Sigma, Schweitze, and TCI America.

The Narasinga-Rao *et al.* (1994) results indicate that 18-β-glycyrrhetinic acid, the aglycon of clycyrrhin, is relatively ineffective at blocking selectin activity (IC₅₀ > 2 mM), suggesting that the sugar residues are important for *in vitro* function. To further simplify the structure and to increase efficacy, glucose (Glu-*O*-GA), galactose (Gal-*O*-GAj), fucose (Fuc-*O*-GA), maltose (Malt-*O*-GA) were linked to glycyrrhetinic acid in place of the two glucuronic acid residues found on glycyrrhizin. The natural *O*-glycosidic linkage to the triterpene core was retained. Most of these substitutions resulted in a loss of selectin inhibition activity *in vitro.*

Based on these data, as well as the fact that fucose is an essential component of the native sLe^{x} ligand for the selectins (Brandley *et al.,* 1993), a C-fucoside of glycyrrhetinic acid (Fuc-*C*-GA) was synthesized (Narasinga-Rao *et al.,* 1994). This compound was designed to incorporate a more chemically and metabolically stable carbon linkage in place of the natural *O*-glycosidic linkage and to more closely mimic the 10-12-Å distance between the carboxylate group and fucose in sLe^{x}. In the ELISA for selectin inhibition, Fuc-*C*-GA was more potent than glycyrrhizin against E-selectin and L-selectin and of equal efficacy against P-selectin. *In vivo,* Fuc-*C*-GA demonstrated greatly increased efficacy over glycyrrhizin and was equal to Fuc-*O*-GA.

Glycyrrhizin is used in Chinese herbal medicines as an anti-inflammatory agent (Davis and Morris, 1991) and is therefore safe for human administration. The synthetic derivatives, especially the *C*-fucoside of glycyrrhetinic acid, are also contemplated for use *in vivo.* Further compounds identified by pharmacophore searches are expected to have utility in P-selectin binding and targeting.

To label an oligosaccharide, a "bridging" molecule may be employed, *e.g*., albumin. For example, albumin is conjugated to glycyrrhizin and the albumin-glycoconjugate is then iodinated with ¹³¹I using previously described methods (Fraker and Speck, 1978) and lactose peroxidase from LKB (Klein, 1989). This technology can be employed with any of the E-selectin-binding oligosaccharides. Glycoconjugates of Lewis X gangliosides have also been described (Terada *et al.,* 1994), and these synthetic methods may be employed in connection with the invention.

### EXAMPLE VII

### Targeting Translocated P-selectin with Oligosaccharide Conjugates

Following labeling of, *e.g.,* glycyrrhizin, with abridging molecule and ion, *e.g.,* albumin and ¹²⁵I, the binding properties of the resultant conjugate can then be analyzed. For example, ¹²⁵I-glycyrrhizin- albumin conjugates are added to endothelial cells before and after ionizing radiation exposure, in an analogous manner to that described in the earlier examples for the targeting of P-selectin with antibody conjugates.

Clinically, ¹³¹I-albumin-glycoconjugate is added to cells within about 30 min after irradiation as described above in an analogous manner to that in the earlier examples. The ¹³¹I-albumin-glycoconjugate binding to x-ray-induced P-selectin is then quantified by scintillation counting. Efficacy is determined by colony formation assay as described in Hallahan *et al.* (1989). Tumor regression is analyzed as described in Weichselbaum *et al.* (1994).

The doses of radiolabeled glycyrrhizin or glycyrrhizin-selected agent conjugates for use in human diagnosis or treatment protocols are contemplated to be within the range of the doses previously employed with glycyrrhizin in non-radiation methods. For example, Soma *et al.* (1994) described the effect of glycyrrhizin on cortisol metabolism, in which 225 mg/day of glycyrrhizin was given for seven consecutive days.

Okunu *et al.* (1994) gave large doses of glycyrrhizin to patients with hepatitis C. The doses described by Okunu *et al.* (1994) could also be employed in the invention. Numazaki *et al.* (1994) gave glycyrrhizin to children with liver dysfunction associated with CMV infection. Specifically, 0.2% glycyrrhizin was dissolved in saline to 2 mg/ml, supplemented with glycine and cystein and administered intravenously at 50 ml/day for more than one week. This is also contemplated effective dosage range for use in this invention.

Akao *et al.* (1994) gave 100 mk/kg glycyrrhizin orally to rats, when it was found that glycyrrhizin itself is poorly absorbed from the gut. This should be taken into account in optimizing the appropriate doses of glycyrrhizin and glycyrrhizin-containing formulations. Tsai *et al.* (1992) also gave glycyrrhizin at doses of 100 mk/kg. However, it is important to note that Krahenbuhl *et al.* (1994) gave doses of 18β-glycyrrhetic acid, a major metabolite of glycyrrhizin, to healthy human volunteers in up to doses or 1500 mg orally.

### EXAMPLE VIII

### P-selectin-Binding Polysaccharides and Glycoproteins

Human protein C also inhibits selectin-mediated cell adhesion. The human anticoagulant factor, Protein C, is a unique fucosylated plasma glycoprotein that has reported anti-ischemic and anti-inflammatory properties. It has been reported that both human plasma-derived and human cell-produced recombinant Protein C inhibit selectin-mediated cell adhesion (Grinnell *et al.,* 1994). This effect was reportedly not mediated through the serine protease activity of Protein C, but through its carbohydrates. Using oligosaccharides isolated from human cell-produced Protein C, Grinnell *et al.* (1994) defined a polylactosamine structural determinant that inhibits adhesion. This uncharged determinant appears to be a more potent ligand for selectins than the sialylated Lewis X antigen (Grinnell *et al.,* 1994), and can therefore also be used in the present invention to bind to P-selectin.

A 150 kd glycoprotein ligand for E-selectin has also been found on mouse myeloid cells (Lenter *et al.,* 1994). In addition, glycoproteins of 230 kd and 130 were identified on mature mouse neutrophils that also bound both to E-selectin in a Ca(2+) dependent fashion (Lenter *et al.,* 1994), although the signals detected for these ligands were 15-20-fold weaker than those for the monospecific ligands. All of these glycoproteins are contemplated for use as P-selectin binding ligands, as are polysaccharides, such as polylactosamine.

### EXAMPLE IX

### Liposomes as Agents for P-selectin Delivery

Oligosaccharides, polysaccharides or glycolipids may be used alone as the targeting component of a drug delivery agent, or may be formulated into liposomes, or attached to a liposome, resulting in a liposomal targeting and delivery agent.

Cells are irradiated with 50, 100, or 200 cGy. Thirty minutes following irradiation, cells are transfected with *LacZ* expression vectors using, *e.g*., a sialyl Lewis X-DOTMA conjugate or a anti-P-selectin-DOTMA conjugate. The optimal time for exposure of cells to Lipofectin is about 6 h. Therefore, following irradiation, cells are exposed to sialyl Lewis X-DOTMA conjugate for 2, 4, 6, or 8 h to determine which time period is optimal for reducing the transfection time. Controls include irradiated cells transfected with Lipofectin and unirradiated cells transfected with sialyl Lewis X-DOTMA conjugate. *LacZ* encodes β-galactosidase and this enzyme is quantified as previously described by Hallahan *et al.* (1992).

Naturally, in addition to oligosaccharides, polysaccharides and glycolipids, any other P-selectin targeting agent could be formulated into a liposome preparation and used for delivery and targeting. The liposomes may contain any selected agent, whether in the form of a gene therapy agent, a protein, toxin, radionuclide and the like.

Liposome formulations particularly contemplated for use include DOTMA, DOTMA/DOPE and DORIE. However, virtually any liposome that binds to P-selectin may be employed. Liposomes and nanoparticles are known to be safe for human administration. For example, they have been used in the targeted antibiotic therapy of intracellular bacterial infections and diseases as they can generally entrap, and then liberate antibiotics such as ampicillin in a stable and reproducible way (Henry-Michelland *et al.,* 1987).

Further liposome formulations contemplated for use in the present invention include cationic liposomes. Trubetskoy *et al.* (1992) showed that cationic liposomes enhanced targeted delivery and expression of exogenous DNA mediated by N-terminal modified poly-L-lysine-antibody conjugate in mouse lung endothelial cells. DNA:antibody conjugates and cationic liposomes form a ternary electrostatic complex which preserves the ability to bind specifically to the target cells. The addition of liposomes has been reported to enhance the specific transfection efficiency of antibody-polylysine/DNA binary complex by 10 to 20-fold in mouse lung endothelial cells in culture (Trubetskoy *et al.,* 1992). The utilization of these types of methods, following P-selectin radioinduction as provided by this invention, is contemplated to be particularly useful.

Liposomes may also be used in conjunction with anti-P-selectin antibodies, as described by Klibanov *et al.* (1991). Such liposomes coupled to monoclonal antibodies, termed "immunoliposomes", have been reported to show specific localization *in vivo* Klibanov *et al.* (1991). This will provide an added advantage when used with the present invention, in which specific P-selectin would have previously been induced. Liposomes made by conjugating a targeting antibody directly to the liposome surface were also reported to be efficiently internalized and retained in studies by Matthay *et al.* (1989).

To avoid side effects due to intracellular polymeric overloading, ultrafine particles (sized around 0.1 µm) may be designed using polymers able to be *degraded in vivo.* Biodegradable polyalkyl-cyanoacrylate nanoparticles that meet these requirements may be used. They are easily made, as described by Couvreur *et al.* (1984, U.S. Patent 4,489,555; 1988). More recently, liposomes were developed with improved serum stability and circulation half-times (Gabizon and Papahadjopoulos, 1988; Allen and Choun, 1987).

Liposomes are formed from phospholipids that are dispersed in an aqueous medium and spontaneously form multilamellar concentric bilayer vesicles (also termed multilamellar vesicles (MLVs). MLVs generally have diameters of from 25 nm to 4 µm. Sonication of MLVs results in the formation of small unilamellar vesicles (SUVs) with diameters in the range of 200 to 500 Å, containing an aqueous solution in the core.

Liposomes bear many resemblances to cellular membranes and are contemplated for use in connection with the present invention as carriers for the second agents and/or genes. They are widely suitable as both water- and lipid-soluble substances can be entrapped, *i.e.,* in the aqueous spaces and within the bilayer itself, respectively. It is possible that drug-bearing and gene-bearing liposomes may even be employed together for site-specific delivery of active agents to P-selectin expressing cells.

Liposomes have been used to transfect syngeneic tumors cells *in vivo* following irradiation of human tumor xenografts and syngeneic tumor models, so their safety has been established. Pro4L murine fibrosarcoma is a syngeneic tumor model that has been used. Tumor cells are injected subcutaneously into the hind limb of mice. Tumors are grown to 160 mm³. Liposomes containing pE-sel-TNF are injected directly into tumors or administered systemically. Tumors are irradiated (10 Gy) 24 h after liposome injection. TNF is quantified from excised tumors as described by Weichselbaum *et al.* (1994), and tumor control is quantified as described by Weichselbaum *et al.* (1994).

The above model can be easily adapted for use in the present invention. Liposomes are used to transfect tumor vasculature endothelial cells *in vivo*, following initial irradiation of the tumor to translocate P-selectin. After irradiation and P-selectin translocation, liposomes are injected directly into tumors, directed to the tumor site using a catheter, or administered to the animal systemically. The use of iridium wire in blood vessels to control vascular gene therapy in a site directed manner is also contemplated. Tumors are then irradiated again (10 Gy) 24 h after liposome injection and TNF and tumor regression is quantified.

The use of liposomes in gene transfer has been established to be safe. For example, Caplen *et al.* (1995) used liposomes to transfer the CFTR gene to the nasal epithelium to patients with cystic fibrosis. If delivery to the nasal epithelium was specifically desired, the techniques of Caplen *et al.* (1995) could be followed, after local irradiation to translocate P-selectin.

The use of antitumor drugs housed within liposomes conjugated with anti-tumor antibodies has also been described (Konno *et al.,* 1987; Betageri *et al.,* 1993). These methods for the immunospecific delivery of drugs and other materials to antigenic target cells can be adapted for use in this invention.

### EXAMPLE X

### Cells that Bind to P-selectin

Targeting leukocytes containing therapeutic genes, or other selected agents, to tumor vasculature is another element of this invention. P-selectin and E-selectin bind essentially the same cellular ligand, and hence, the same cells.

Both neutrophils and eosinophils have been shown to bind to the inducible endothelial cell adhesion molecule E-selectin. Counter ligands on eosinophils for E-selectin. have been identified as sialyl dimeric Le(x) compounds (Bochner *et al.,* 1994). It has also been demonstrated that human CD56+CD16+/CD3-NK cells adhere to the E-selectin expressed by stimulated HUVEC in a sialidase-and Ca(2+)-dependent manner (Pinola *et al.,* 1994).

Norton *et al.* (1993) and Lo *et al.* (1994) also showed that human neutrophils bind to E-selectin. Wakita *et al.* (1994) demonstrated that E-selectin is the critical adhesion molecule for trafficking of memory T cells into certain skin lesions. Olofsson *et al.* (1994) also showed that E-selectin mediates leukocyte rolling in interleukin-1-treated rabbit mesentery venules.

A subset of human helper memory T cells is known to adhere to E-selectin expressed on cytokine-activated endothelial cells. A distinct type of sialyl Lewis X antigen, defined by a novel monoclonal antibody, has been shown to be selectively expressed on helper memory T cells (Ohmori *et al.,* 1993). Of the various molecular species of sialyl Lex antigens present on carbohydrate side chains of cellular glycoproteins, it has been reported that helper memory T cells express a distinct type of sialyl Lex antigen. Cultured ATL cells expressing the 2F3-defined antigen showed a clear E-selectin-dependent adhesion to cytokine-activated endothelial cells (Ohmori *et al.,* 1993).

These studies of Weichselbaum *et al.* (1994) can be adapted for use in the present invention by irradiating the tumor site first to induce P-selectin translocation. This new P-selectin-mediated therapy is thus contemplated to enhance tumor targeting in vasculature without increasing normal tissue toxicity, particularly when used with gene therapy.

Transfected TIL cells have been used to treat cancer (Hwu *et al.,* 1993). These authors showed that TNF is effective in causing the regression of selected murine tumors when administered at high concentrations. Therapeutic levels in humans cannot be obtained systemically, however, because of dose-limiting toxicity. The development of immunotherapy with IL-2 and tumor-infiltrating lymphocytes (TIL), which can accumulate at tumor sites in some patients, and of efficient retroviral techniques for gene transfer into eukaryotic cells has allowed new therapeutic approaches using TNF.

Hwu *et al.* (1993) retrovirally transduced human TIL with the gene for TNF in an attempt to deliver high concentrations of TNF to the tumor site without dose-limiting systemic toxicity. Successful gene insertion was confirmed and transduced selected TIL cultures produced greater amounts of TNF, compared with nontransduced controls. In an attempt to increase TNF production, TIL were transduced with a mutated form of TNF containing the IFN-_{γ} signal peptide in place of the transmembranous region, to enhance secretion into the endoplasmic reticulum.

More specifically, Hwu *et al.* (1993) used the following methods. TIL were derived from enzymatically digested tumor biopsies as well known in the art. Briefly, melanoma tumor biopsies were digested overnight with collagenase type IV (1 µg/ml), hyaluronidase (0.1 µg/ml), and DNase {30 U/ml) (Sigma Chemical Co., St. Louis, MO). After digestion, the single-cell suspensions were passed through a sterile wire screen grid and subjected to Ficoll-Hypaque separation to remove dead cells and RBC.

TIL cell cultures were established at 5.0 × 10⁵ cells/ml in 24-well sterile tissue culture plates, in medium consisting of RPMI 1640 supplemented with 10% human A serum (Bio-Whitaker, Walkersville, MD). This medium was mixed 1:1 (v/v) with AIM V serum-free medium (GIBCO, Grand Island, NY) and was further supplemented with gentamicin sulfate (10 µg/ml), penicillin G sodium (10,000 U/ml), glutamine (200 mM) (all from GIBCO), 7200 IU/ml IL-2 (Cetus, Emeryville, CA), and 10% (v/v) lymphokine-activated killer cell-conditioned supernatant.

Because TIL can double every 2 to 4 days, TIL densities were maintained at 5.0 × 10⁵ by splitting cultures every 3 to 5 days with fresh medium containing IL-2 and passaging cells to larger cell culture plates (six-well) when required. When TIL reached greater than 2 × 10⁸ in number, they were cultured i AIM V serum-free medium alone containing 6000 IU/ml IL-2. The culture vessels used for large-scale expansion were gas-permeable PL732 3-liter plastic bags (Fenwal, Deerfield, IL).

The methods described by Hwu *et al.* (1993) can be adapted for use in the present invention with the added advantage that by irradiating the tumor site first, P-selectin translocation will be induced, allowing for enhanced targeting. Transfected TIL cells are injected as described by Hwu *et al.* (1993)-4 h after P-selectin translocation by irradiation of tumors. Lymphocytes will bind to P-selectin.

### EXAMPLE XI

### Cellular Ligands that Bind to P-selectin

Distinct cell surface ligands mediate T lymphocyte attachment and rolling on selectins under physiological flow (Alon *et al.,* 1994). Such antigens can be used in conjunction with the present invention.

The HECA-452 antigen is a homing receptor for lymphocyte migration into skin. HECA-452 was later identified as a group of related sugar moieties that bind to E-selectin. Direct evidence has been reported to show that the antigen recognized by HECA-452 is involved in the adhesion of leukocytes to endothelial cells, and that this antigenic epitope is different from that reactive to the sialylated Lewis X antigen (De Boer *et al.,* 1994). The HECA-452 antigen can also be used in conjunction with the present invention.

The ligand L-CanAg (light cancer antigen), secreted by a colon carcinoma cell line COLO 205; may also be used in certain selected binding embodiments (Zhang *et al*.; 1994), particularly those *in vitro* assays, such as conducted on biopsy material.

### EXAMPLE XII

### Vectors and Gene Therapy Following P-selectin Translocation

The field of gene transfer into vascular cells is emerging as a new approach for studying the pathophysiology of vascular disease and for developing potential new genetic treatments for these disorders (Nabel *et al.,* 1994). P-selectin-mediated control of vascular gene therapy forms another aspect of the present invention. The text and figures of Nabel *et al.* (1994) and Gutierrez *et al.* (1992) are referred to for the purposes of even further supplementing the present disclosure in terms of describing gene transfer strategies and vectors, genes and antisense oligonucleotides, drugs, viral vectors, liposomes, animal models of vascular gene transfer, normal tissue protection, and the like.

Recombinant vectors capable of expressing a protein in vascular endothelial cells will be targeted to such cells using an P-selectin binding moiety. The vectors may have an ionizing radiation-inducible promoter that directs the expression of the protein, in which case, a subsequent round of irradiation will induce expression of the encoded protein in the cells. Effective ionizing radiation-inducible promoters include the CArG domain of the *Egr-1* promoter, the *fos* promoter, the c-jun promoter and the TNF-α promoter.

The vectors will generally direct the expression of an anticellular agent capable of killing or suppressing the growth or cell division of tumor-associated endothelial cells. One group of such anticellular agents are the tumor suppressor proteins, such as p53, p16 (Kamb *et al.,* 1994), proteins produced in response to p53, the retinoblastoma gene product (Rb), the Wilms' tumor gene product (WT1), and certain cyclins (Marx, 1994). In addition, further tumor suppressors include APC, DCC, NF-1, NF-2, MEN-I, MEN-II, BRCA1, VHL, FCC and MCC.

The protein p53, a 53 kd nuclear phosphoprotein that controls cell proliferation, is one example of a tumor suppressor. The p53 protein is highly conserved through evolution and is expressed in most normal tissues. Point mutations in the p53 gene and allele loss on chromosome 17p, where the p53 gene is located, are among the most frequent alterations identified in human malignancies. Even single base substitution in p53 can result in p53 proteins with altered growth regulatory properties, which leads to cancer.

The p53 gene has been found to be the most frequently mutated gene in common human cancers (Hollstein *et al.,* 1991; Weinberg, 1991), and is particularly associated with those cancers linked to cigarette smoke (Hollstein *et al.,* 1991). The over-expression of mutated p53 in breast tumors has also been documented (Casey *et al.,* 1991). Transfection of wild-type p53 into certain types of breast and lung cancer cells can restore growth suppression control in cell lines.

Although the present invention concerns targeting to tumor vasculature endothelial cells, the delivery of a tumor suppressor gene, such as p53, to the cancer environment using this invention is contemplated to be useful. Supplying the normal tumor suppressor proteins may serve to suppress the growth of the endothelial cells, and may even act on certain tumor cells themselves, following uptake into tumor cells in the vicinity of the blood vessels. In a similar manner, supplying antisense constructs of certain oncogenes is also contemplated. Exemplary oncogenes that are appropriate targets for antisense constructs include *ras, myc, neu, raf, erb, src, fms, jun, trk, ret, gsp, hst, bcl* and *abl.*

Vectors expressing chemotherapeutic agents and toxins are further examples of anticellular agents capable of killing or suppressing the cell growth or division of tumor-associated endothelial cells. Exemplary chemotherapeutic agents include IFNs, TNF-α and IL-12. Exemplary cytotoxic agents include mammalian cell-, plant-, fungus- and bacterial-derived toxins, such as diphtheria toxin, *Pseudomonas* exotoxin, ricin A chain and deglycosylated ricin A chain. All such chemotherapeutic agents and toxic agents may be used in connection with the present invention.

The vectors may also be used to express an enzyme capable of converting a non-toxic pro-drug into a cytotoxic drug. Examples of this are the herpes simplex virus (HSV) thymidine kinase (tk) enzyme and the cytosine deaminase enzyme.

The vectors may be plasmids, retroviral vectors in recombinant retroviruses, adeno-associated virus (AAV) vectors in AAV virions, adenoviral vectors in replication-deficient adenovirus, and the like. Recombinant vectors may also be included within cells or liposomes. Targeting may be facilitated using a bispecific antibody directed to the virus, on one had, and P-selectin on the other.

### EXAMPLE XIII

### Pharmaceutical Compositions and Delivery

Aqueous compositions of the present invention comprise an effective amount of the P-selectin-targeting agent:selected agent conjugate dissolved or dispersed in a pharmaceutically acceptable carrier or aqueous medium. The phrases "pharmaceutically or pharmacologically acceptable" refer to molecular entities and compositions that do not produce an adverse, allergic or other untoward reaction when administered to an animal, or a human, as appropriate.

As used herein, "pharmaceutically acceptable carrier" includes any and all solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions.

In addition to the compounds formulated for parenteral administration, such as intravenous or intramuscular injection, other pharmaceutically acceptable forms include, e.g., tablets or other solids for oral administration; time release capsules; and any other form currently used, including cremes and lotions, and even mouthwashes, inhalants and the like.

To formulate the compounds or cells for parenteral administration, e.g., for injection via the intravenous, intramuscular or sub-cutaneous routes, typically, such compositions will be prepared as injectables, either as liquid solutions or suspensions; solid forms suitable for using to prepare solutions or suspensions upon the addition of a liquid prior to injection; or as emulsified preparations.

Solutions of the P-selectin-based active compounds as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating action of microorganisms, such as bacteria and fungi.

Anti-P-selectin antibodies and saccharides and conjugates thereof can be formulated into a composition in a neutral or salt form. Pharmaceutically acceptable salts, include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

The carrier can also be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 mL of isotonic NaCl solution and either added to 1000 mL of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject.

The preparation of more, or highly, concentrated solutions for intramuscular injection is also contemplated where necessary. In this regard, the use of DMSO as solvent is contemplated as this will result in extremely rapid penetration, delivering high concentrations of the active agents to a small area. Therapeutic formulations in accordance with the present invention may also be reconstituted in the form of mouthwashes, in conjunction with antifungal reagents. Inhalant forms are also envisioned, which again, may contain the active agents alone, or in conjunction with other agents, such as, *e.g*., pentamidine.

The therapeutic formulations of the invention may also be prepared in forms suitable for topical administration, such as in cremes and lotions. These forms are envisioned to be particularly suitable for additionally treating radiation induced dermatitis. The preparation of oleaginous or water-soluble ointment bases is also well known to those in the art. For example, these compositions may include vegetable oils, animal fats, and more preferably, semisolid hydrocarbons obtained from petroleum. Particular components used may include white ointment, yellow ointment, cetyl esters wax, oleic acid, olive oil, paraffin, petrolatum, white petrolatum, spermaceti, starch glycerite, white wax, yellow wax, lanolin, anhydrous lanolin and glyceryl monostearate. Various water-soluble ointment bases may also be used, including glycol ethers and derivatives, polyethylene glycols, polyoxyl 40 stearate and polysorbates. Even delivery through the skin may be employed if desired, *e.g.*, by using transdermal patches, iontophoresis or electrotransport.

The P-selectin-directed agents of the invention may also be advantageously employed for the preparation of ophthalmic solutions. Thus, for these embodiments, a conjugate or active composition of the invention would be administered to the eye of the subject in need of treatment in the form of an ophthalmic preparation prepared in accordance with conventional pharmaceutical practice, see for example "Remington's Pharmaceutical Sciences" 15th Edition, pages 1488 to 1501 (Mack Publishing Co., Easton, PA).

The ophthalmic preparation will generally contain active agents in a concentration from about 0.01 to about 1% by weight, preferably from about 0.05 to about 0.5% in a pharmaceutically acceptable solution, suspension or ointment. Some variation in concentration will necessarily occur, depending on the particular compound employed, the condition of the subject to be treated and the like, and the person responsible for treatment will determine the most suitable concentration for the individual subject. The ophthalmic preparation will preferably be in the form of a sterile aqueous solution containing, if desired, additional ingredients, for example preservatives, buffers, tonicity agents, antioxidants and stabilizers, nonionic wetting or clarifying agents, viscosity-increasing agents and the like. Suitable preservatives for use in such a solution include benzalkonium chloride, benzethonium chloride, chlorobutanol, thimerosal and the like. Suitable buffers include boric acid, sodium and potassium bicarbonate, sodium and potassium borates, sodium and potassium carbonate, sodium acetate, sodium biphosphate and the like, in amounts sufficient to maintain the pH at between about pH 6 and pH 8, and preferably, between about pH 7 and pH 7.5. Suitable tonicity agents are dextran 40, dextran 70, dextrose, glycerin, potassium chloride, propyleneglycol, sodium chloride, and the like, such that the sodium chloride equivalent of the ophthalmic solution is in the range 0.9 plus or minus 0.2%. Suitable antioxidants and stabilizers include sodium bisulfite, sodium metabisulfite, sodium thiosulfite, thiourea and the like. Suitable wetting and clarifying agents include polysorbate 80, polysorbate 20, poloxamer 282 and tyloxapol. Suitable viscosity-increasing agents include dextran 40, dextran 70, gelatin, glycerin, hydroxyethylcellulose, hydroxmethylpropylcellulose, lanolin, methylcellulose, petrolatum, polyethylene glycol, polyvinyl alcohol, polyvinylpyrrolidone, carboxymethylcellulose and the like.

The ophthalmic preparation will be administered topically to the eye of the subject in need of treatment by conventional methods, for example in the form of drops or by bathing the eye in the ophthalmic solution.

In certain embodiments, active compounds may be administered orally. This is contemplated for agents that are generally resistant, or have been rendered resistant, to proteolysis by digestive enzymes. Such compounds are contemplated to include chemically designed or modified agents; dextrorotatory peptidyl agents, *e.g*., as may used in combination with other E-selectin binding agents; and peptide and liposomal formulations in time release capsules to avoid peptidase and lipase degradation.

For oral administration, the active compounds may be administered, for example, with an inert diluent or with an assimilable edible carrier, or they may be enclosed in hard or soft shell gelatin capsule, or compressed into tablets, or incorporated directly with the food of the diet. For oral therapeutic administration, the active compounds may be incorporated with excipients and used in the form of ingestible tablets, buccal tables, troches, capsules, elixirs, suspensions, syrups, wafers, and the like. Such compositions and preparations should contain at least 0.1% of active compound. The percentage of the compositions and preparations may, of course, be varied and may conveniently be between about 2 to about 60% of the weight of the unit. The amount of active compounds in such therapeutically useful compositions is such that a suitable dosage will be obtained.

The tablets, troches, pills, capsules and the like may also contain the following: a binder, as gum tragacanth, acacia, cornstarch, or gelatin; excipients, such as dicalcium phosphate; a disintegrating agent, such as corn starch, potato starch, alginic acid and the like; a lubricant, such as magnesium stearate; and a sweetening agent, such as sucrose, lactose or saccharin may be added or a flavoring agent, such as peppermint, oil of wintergreen, or cherry flavoring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar or both. A syrup of elixir may contain the active compounds sucrose as a sweetening agent methyl and propylparabens as preservatives, a dye and flavoring, such as cherry or orange flavor. Of course, any material used in preparing any dosage unit form should be pharmaceutically pure and substantially non-toxic in the amounts employed. In addition, the active compounds may be incorporated into sustained-release preparation and formulations.

### EXAMPLE XIV

### Determining the Level of Radiation Exposure

Anti-P-selectin can be used to visualize any vasculature following irradiation. This will be used for diagnostic radiology such as SPEC scanning.

Again, although the present invention provides a surprising use of P-selectin, in that it is to be specifically induced using radiation, other methods are available that can now be used in conjunction with the invention. In that the present methods for determining the level of radiation exposure are generally based upon determining the levels of P-selectin in exposed animals or patients, previously described methods for quantifying P-selectin are envisioned to be particularly useful.

This approach will follow that of Keelan *et al.* (1994b) who assessed the imaging potential of an anti-E-selectin Mab 1.2B6 in a model of arthritis in the pig. Injection of phytohaemagglutinin (PHA) into a knee led to E-selectin expression on vessels in the synovium and draining deep inguinal lymph nodes, as demonstrated by immunohistology. No E-selectin expression was seen in the control knee injected with buffer alone. Animals were given 111In-Mab 1.2B6 or 111In-control antibody intravenously 3 hr after the intra-articular injection of PHA. Scintigraphy performed 24 hr after 111In-Mab 1.2B6 injection showed obvious localization of activity in the inflamed knee in each of three animals. Radiolabeled anti-E-selectin Mab was thus successfully used to image localized inflammatory tissues (Keelan *et al.,* 1994b).

This approaches of Keelan *et al.* (1994a;b) to quantify changes in vascular luminal expression of E-selectin in models of inflammation and arthritis is considered as a suitable model for adaptation for analyzing P-selectin changes in relation to radiation treatment or damage.

Chapman *et al.* (1994) described the non-invasive imaging of E-selectin expression by activated endothelium in urate crystal-induced arthritis. In this study, they assessed the expression of E-selectin during the evolution of urate crystal-induced arthritis, using a radiolabeled MAb imaging technique. Monosodium urate (MSU) crystals and saline alone were injected respectively into the right (inflamed) and left. This format also could be employed here.

The Gosset *et al.* (1995) method is another method that could be employed using the invention. Biopsies would be obtained from patients after radiation and the selectin level determined to give an indication of radiation exposure.

### EXAMPLE XV

### Treating and Preventing Radiation Damage

This invention also provides compositions and methods for use in preventing or treating radiation-induced inflammation using P-selectin-based therapeutics, in the absence or presence of a second selected agent. The principle behind this method is to administer an agent to the site of irradiation that will bind to P-selectin and interfere with P-selectin's interaction with inflammatory cells such as leukocytes. Secondary agents may be included with the P-selectin binding agent. Such agents are those known to the skilled artisan to reduce inflammation, including any class of anti-inflammatory compound.

Human leukemia cell line HL60 has been used to quantify leukocyte adhesion to endothelial cells (Jones *et al.,* 1995). HL60 cells were added to human umbilical vein in endothelial cells (HUVECs) after exposure to 10 Gy or cells treated with otherwise identical conditions without irradiation. Cultures were washed and cells were counted using a hemocytometer. Consecutive cell washes of culture revealed increased binding of HL60 cells at 4 h after irradiation as compared to untreated controls. Glycyrrhizin, carminic acid or sialyl Lewis X (1 mM each, SIGMA) were added to irradiated cells at 4 h after irradiation and 30 min prior to the addition of HL60 cells. Each agent blocked the adherence of HL60 cells to irradiated HUVECs.

Radiation is known to induce pneumonitis, cystitis, mucositis, esophagitis, dermatitis, neutrophilic vasculitis, acute pulmonary radiation injury and interstitial inflammation (Slauson *et al.,* 1976; Dunn *et al.,* 1986; Ward *et al.,* 1993; Narayan, 1982; Fajardo and Berthrong, 1988; Hopewell *et al.,* 1993), each of which can be treated using this invention. Animal models are also available for studying radiation-induced diseases, *e.g.,* as described by Ward et al. (1993) for the study of radiation-induced pulmonary edema, alveolitis and fibrosis. Such models are intended for use in optimizing appropriate doses.

Following approval by the FDA, glycyrrhizin, carminic acid, sialyl Lewis X, a sialyl Lewis X/A mimic, or other signaling inhibitors, are to be used in phase I trials in patients undergoing radiotherapy. Glycyrrhizin is used as an antiinflammatory agent in Asia (Kanoka *et al.,* 1990; Narasinga Rao *et al.,* 1994), and so is safe for clinical use. These P-selectin-binding agents will be administered to patients receiving radiation therapy as topical pharmaceuticals in water based creams as treatment for radiation dermatitis. The advantage of these agents over glucocorticoid creams is that glucocorticoids slow would healing and re-epithelialization (Fajardo and Berthrong, 1988). After efficacy is demonstrated, these agents will be used as intravenous injections and oral preparations in phase I dose escalation trials to treat severe radiation inflammation, such as in the lung and pericardium.

It is interesting to note that Silber *et al.* (1994) showed that intravenous infusions of neutralizing doses of F(ab')2 fragments of murine antibodies to E-selectin during the early inductive phases of delayed hypersensitivity (DHR) resulted in IgG localization to dermal endothelium. The relative numbers of lymphocytes localized to the inflammatory site were significantly reduced in DHR modified with infusions of antibodies to E-selectin, while the numbers of lymphocytes recruited to skin in the animal given F(ab')2 fragments of an irrelevant murine monoclonal antibody of the same isotype and at the same dose were not changed (Silber *et al.* (1994). A similar approach is to be employed with P-selectin.

### EXAMPLE XVI

### P-selectin translocation to the lumen of irradiated blood vessels is microtubule-dependent

In the present study, the inventors investigated the role of a cell adhesion molecule that is preformed and is activated rapidly without the need for transcription. P-selectin is such a cell adhesion molecule that is preformed in membranous organelles within the vascular endothelium and is rapidly translocated to the vascular lumen. The inventors utilized ionizing radiation to localize the external stimulus to specific organs or blood vessels. X-rays are tissue penetrating and allow precise localization of oxidative injury to a small segment of an organ.

To determine whether radiation-induced P-selectin translocation was specific for vascular endothelium *in vivo*, the inventors irradiated primary-culture HUVECs. Human umbilical vein endothelial cell (HUVEC) cultures were prepared from fresh (24-hr-old) human umbilical veins transported to the laboratory in sterile buffer at 4°C as described (Hallahan *et al.,* 1995; Hallahan *et al.,* 1996). The vein was cannulated, filled with 0.2% collagenase, and incubated at 37°C for 15 min. Cells were flushed and complete medium was added, followed by centrifugation at 2000 rpm for 5 min. The cell pellet was resuspended and maintained in M199 with 10% fetal calf serum, 10% human serum, and pen/strep/amphotericin B solution (Sigma) on gelatin-coated (0.2%) tissue culture dishes at 37°C in 5% CO₂. The purity of endothelial cell cultures was verified by staining for factor VIII. Confluent cells were harvested with 0.1% collagenase 0.01% EDTA and subcultured at a ratio of 1:3. HUVECs were used at third passage; this reduced the number of passenger cells and allowed for uniform expression of cellular adhesion molecules. Thrombin was purchased from SIGMA.

To determine whether microtubules or actin are required for radiation-induced exocytosis of P-selectin, the inventors utilized the microtubule depolymerizing agents, colcemid and nocodozol, or the actin antagonist cytochalasin-B. Primary-culture vascular endothelial cells were grown to 80% confluence on glass slides. HUVEC were pretreated with these agents for 45 min followed by irradiation.

Cells were treated with either thrombin or gamma irradiation from a ⁶⁰CO source (Gammacell 220) as the inventors have previously described (Hallahan *et al.,* 1995; Hallahan *et al.,* 1996). After treatment, HUVECs were fixed with 4% paraformaldehyde for 10 min at room temperature, washed 3 times with antibody buffer (4 gm bovine serum albumin, 0.1 gm Nₐ azide, 0.75 gm glycine, and 100 µl PBS) and 2 times in Hanks' salt solution. Non-specific binding of antibody was blocked with 50% goat serum for 30 min at 37°C in a humid chamber. Cells were then washed with antibody buffer and Hanks' salt solution and incubated with 100 µl of 10 µg/ml anti-P-selectin primary antibody (Pharmingen, cat. #09361A) for 2 to 3 h at 37°C in a humid chamber. Cells were then washed with antibody buffers and Hanks' solution and incubated with 10 µl of a 1:300 dilution of FITC conjugated goatanti-rabbit IgG (cat. #L42001) for 30 min. Cells were washed, counterstained with DAPI, and mounted with antifade mounting medium. After washings, cells were visualized with a Zeiss Photomicroscope III fluorescence microscope for incident-light excitation. Slides were mounted and examined for fluorescence and by phase microscopy.

Using images of cells from a 100X objective using confocal microscopy, the inventors measured fluorescence intensity, in pixels, on the cell membrane. Fluorescence intensity was measured by NIH Image software as described (Hallahan and Virudachalam, 1997). Experiments were performed 3 to 4 times. All data were analyzed by use of Statistica for Windows software (StatSoft, Inc., Tulsa, OK).

Immunofluorescent confocal microscopy showed P-selectin translocation to the cell membrane of irradiated HUVEC. At 60 min following irradiation, HUVEC treated with radiation alone showed the "starry sky" pattern of immunofluorescence of P-selectin on the cell membrane. The microtubule depolymerizing agents colcemid and nocodozol inhibited x-ray induced translocation of P-selectin and showed P-selectin localized to cytoplasmic storage reservoirs in a pattern similar to untreated control. HUVEC were pretreated with buffer alone, colcemid or nocodozol. Immunofluorescence of anti-P-selectin antibody staining was performed 60 minutes after x-irradiation of HUVEC. Conversely, cells pretreated with cytochalasin-B showed no inhibition of P-selectin translocation (FIG. 2). Localization of P-selectin on the cell membrane was quantified by use of immunofluorescence confocal microscopy, which was quantified by NIH Image software. This showed an 8-fold increase in P-selectin immunofluorescence on the cell surface, which was abrogated by Colcemid and nocodozol, but not the actin antagonist cytochalasin-B (FIG. 2).

Immunofluorescence microscopy allowed the visualization of P-selectin in endothelial cells. P-selectin was compartmentalized in Weibel-Palade bodies, which underwent membrane transport to the cell membrane after exposure to ionizing radiation. Prior to irradiation, P-selectin was localized to storage reservoirs within the cytoplasm of endothelial cells. At 15 min after irradiation (2 Gy), WPB began translocation to the cell membrane. Translocation of P-selectin to the cell membrane was complete at 30 min after irradiation. P-selectin remained tethered to the cell membranes at 60 min after exposure to 2 Gy and P-selectin immunofluorescence stained in a starry sky pattern. The increased intensity of immunofluorescence after exocytosis may be due to increased accessibility of epitopes once P-selectin is translocated to the cell membrane. HUVECs were treated with 2 Gy and fixed at 0 min, 30 min and 60 min.

To determine whether radiation-induced P-selectin translocation is dose-dependent, the inventors treated HUVEC with 1, 2, and 5 Gy. Confocal microscopy was used to measure immunofluorescence on the cell surface, which was quantified by NIH Image software (FIG. 3). There was minimal WPB exocytosis in response to 1 Gy, but the inventors found that 2 Gy was sufficient to induce P-selectin translocation to the cell membrane of endothelial cells. Higher doses induced no more rapid or efficient translocation than 2 Gy, indicating that there is no dose dependence in x-ray-induced P-selectin translocation.

To determine whether radiation induces P-selectin secretion, the inventors measured P-selectin in the medium of endothelial cell cultures by use of ELISA. HUVEC were grown in 60 mm plates and x-irradiated with 2, 5 and 10 Gy using the maxitron x-ray generator. At 1, 2, 4, 6, and 24 h after irradiation, medium from irradiated cells was sampled and placed into ELISA wells (R&D Systems). Assays were performed 3 to 4 times and mean and standard error of the mean were measured. Medium from untreated HUVECs had no measurable P-selectin levels. After irradiation, there was no increase in P-selectin within the medium.

To determine whether P-selectin is released from irradiated tissues, the inventors measured P-selectin in the serum of mice treated with total body irradiation. There was no increase in serum P-selectin after exposure to 2, 5 or 10 Gy.

The mechanisms of WPB translocation have not been well characterized. Disruption of microtubules by colchicine attenuates vWF secretion from thrombin-treated endothelial cells. This finding suggests that WPB translocation is microtubule-dependent. Several mechanisms of transport of membranous organelles have been described. Mechanochemical ATPases function as motors to move intracellular cargo to the cell membrane (reviewed in Bloom and Endow, 1995). These motors include kinesin, dynein, and actin (Goodson *et al.,* 1997). Dynein and kinesin are the primary protein motors for transported membranes following packaging in the Golgi apparatus (Lafont and Simons, 1996). These protein motors are not mutually exclusive, because protein complexes have been shown to be sites of coordination between the actin- and microtubule-dependent motility systems.

Membranous organelle motility has been studied by both light and electron microscopy and has been shown to be associated with microtubules (Bloom and Endow, 1995). Movement along microtubules requires ATP. and is inhibited by the ATP analogue AMP-PNP (Lasek and Brady, 1985). The mechanochemical cycle for force during organelle mobility is dependent primarily upon kinesin, which is an ATPase. Kinesin is localized to the membranous portion of intracellular organelles within human neutrophils (Rothwell *et al.,* 1993). The carboxy-terminus of kinesin interacts with cytoplasmic microtubules in mammalian cells (Navone *et al.,* 1992). Microinjection of anti-kinesin antibodies prevents the movement of lysosomal tubules (Hollenbeck and Swanson, 1990).

The biological significance of WPB fusion with the cell membrane is that the contents include P-selectin, nitric oxide synthase (Fukuda *et al.,* 1995), vWF (Pinsky *et al.,* 1996), CD63 (Vischer and Wagner, 1993), and interleukin-8 (IL-8) (Rot, 1992). Each of these components may contribute to intercellular signaling from the vascular endothelium to circulating blood components, or to adjacent endothelial cells. WPB are translocated to the luminal surface of the vascular endothelium following cytokine stimulation. The inventors' data indicate that WPB translocate to the vascular lumen within 30 minutes of irradiation. The contents of WPB are released into the vascular lumen or remain tethered to the cell membrane. The importance of tethered intercellular signal transduction is that it does not disperse in the circulation, but remains localized. Tethered protein and proteoglycan components of WPB, CD63, IL-8 and P-selectin, participate in leukocyte recruitment, activation, and extravasation. The inventors observed that P-selectin was translocated to the blood-tissue interface of the irradiated vascular endothelium, where leukocytes were found to adhere to stained P-selectin. P-selectin binds to its counterreceptors, carbohydrate ligands and PSGL-1 on leukocytes to activate signal transduction within the inflammatory cells (Haller *et al.,* 1997). Monocyte tethering by P-selectin activates secretion of monocyte chemotactic protein-1 (MCP-1) and TNF secretion (Weyrich *et al.,* 1995). P-selectin specifically enhanced nuclear translocation of NFkB in monocytes, which is required for MCP-1 and TNF induction (Weyrich *et al.,* 1995; Lorant *et al.,* 1991). Tissue factor is also induced within monocytes following this binding to P-selectin (Celi *et al.,* 1994). P-selectin activates neutrophils to produce oxygen radicals (Tsuji *et al.,* 1994), and it releases proteolytic enzymes and arachidonic acid, resulting in tissue injury (Lorant *et al.,* 1991). Neutrophils respond with polarization (diapedesis) and increased affinity of the integrin CD11/CD18 on the leukocyte for its counterreceptor, ICAM-1, on the endothelium (Femadez-Segura *et al.,* 1996; Evangelista *et al.,* 1996; Lo *et al.,* 1991). These integrins, in turn, create the firm adhesion required for transendothelial migration. The significance of P-selectin activation by ionizing radiation is that this activation is required for neutrophil-dependent acute lung injury (Mulligan *et al.,* 1992; Mulligan *et al.,* 1993). P-selectin knockout mice have marked attenuation of inflammatory emigration from the circulation (Mayadas *et al.,* 1993).

To determine the radiation-induced P-selectin translocation in animals, C57BL6 Mice (Jackson Laboratories,) were irradiated as we have described (Hallahan and Virudachalam, 1997a; Hallahan and Virudachalam, 1997b). The C57BL6 mice were treated with thoracic irradiation (10 Gy). Ten, 30, 60, or 120 min after irradiation, mice were euthanized by intraperitoneal injection of xylazine and ketamine. Tissues were fixed in formalin and embedded in paraffin. Tissue blocks were then sectioned in 5 µm thick sections.

Tissue sections were baked at 60°C for 1 h, cleared in xylene, and hydrated through a descending alcohol series to distilled water. For E-selectin and CD45 immunostaining, the hydrated sections were incubated with Protease I (Ventana Biotech, Tucson, AZ) for 8 min at 42°C. For ICAM immunostaining, the hydrated sections were incubated with Protease II (Ventana Biotech) for 8 min at 42°C. After brief washing in ddH₂O, endogenous activity was blocked by treatment of the sections with 3% hydrogen peroxide in methanol for 20 min. Two tissue sections from each case were then incubated overnight at 4°C at a titer of 2.5 µg/ml for anti-P-selectin antibody (Pharmingen, San Diego, CA). One slide from each sample was treated in a similar fashion and incubated overnight in normal serum immunoglobulin (Ventana Medical Systems, Tucson, AZ). The immunohistochemical staining was performed on a Ventana Gen¹¹ system (Ventana Medical Systems, Tucson, AZ). The Ventana Gen¹¹ uses an indirect strepavidin-biotin system conjugated with horseradish peroxidase for detection of the immunocomplex and diaminobenzidine as a substrate for localization. The Ventana Gen¹¹ uses a cartridge delivered avidin/biotin blocking kit to block endogenous biotin. The immunostained sections were counterstained with hematoxylin, dehydrated through an ascending alcohol series, cleared, and coverslipped.

During immunohistochemical analysis of the irradiated tissues, the inventors observed that P-selectin protein in the vascular endothelium was translocated to the tissue-blood interface after irradiation. Prior to irradiation, P-selectin staining was localized to the endothelial cells, but it was translocated to the tissue-blood interface within 1 h of irradiation. Leukocytes colocalize with translocated P-selectin at the blood-tissue interface in irradiated pulmonary vessels.

To determine whether P-selectin translocation to the vascular lumen after irradiation is tissue-specific, the inventors studied tissue sections from irradiated murine small intestine, colon, kidney, and brain. The inventors observed that P-selectin translocation occurred following doses of radiation that are used in fractionated radiation therapy. The irradiated small intestine showed P-selectin translocation to the blood-tissue interface at one hour after irradiation. Likewise, the vasculare endothelium within the irradiated large intestine showed P-selectin translocation. In contrast to the lung and intestine, the brain, kidney and liver showed no P-selectin staining and no translocation to the vascular lumen either before or after irradiation.

To study the duration of P-selectin translocation to the luminal surface of irradiated blood vessels, the inventors studied later time points. P-selectin localization to the blood-tissue interface persisted at 6 hours after irradiation. P-selectin is also present in the granules of platelets and was therefore observed within platelet aggregates that were first observed within irradiated blood vessels at 6 hours after irradiation. At 24 h, P-selectin immunohistochemistry revealed staining of platelet aggregates within irradiated blood vessels of the lung and intestine. Platelet aggregation was transient, and resolution began by 48 h after irradiation. At 24 h after irradiation, leukocytes adhered to P-selectin within platelet aggregates.

### EXAMPLE XVII

### X-ray-induced P-selectin expression in Tumor Blood Vessels

To determine whether the endothelium within neoplasms responds to x-rays in a manner that is distinct from the response of normal tissues, the inventors studied P-selectin translocation after x-irradiation of tumors. To determine whether radiation-induced exocytosis of endothelial P-selectin was tumor type-specific or species-specific, the inventors studied tumors in rats, C3H mice, C57BL6 mice, and nude mice.

Rat C6 glioma cells were maintained in Ham's F10 medium with 15% horse serum, 2.5% fetal bovine serum, and 10 mM HEPES. Murine GL261 gliomas were maintained in an F-12/DME 50% mixture and 7% fetal calf serum, and pen/strep. The human colon carcinoma cell line WIDR was maintained in MEMa, 1% NEAA and 10% fetal calf serum, and P/S.

Tumors were induced by injection of tumor cells either subcutaneously or stereotactically into the rat brain. Subconfluent tumor cells were trypsinized, washed, and injected subcutaneously into the hindlimbs of mice. MCA4 tumors were excised, minced, and implanted by use of an 18-gauge needle subcutaneously into the hindlimbs of C3H mice (Jackson Labs). Lewis lung carcinoma cells (10⁶) were injected into the hindlimbs of C57BL6 mice (Jackson Labs). Rats C6 cells were injected into Wistar rats (250-300 g) (Charles River, Wilmington, MA). Human colon carcinoma WIDR cells (10⁶) were injected into nude mice (Jackson Labs). Tumors were grown to a volume of 300 to 500 mm³ prior to treatment with x-rays or cytokines.

To determine whether P-selectin in tumor blood vessels is translocated to the vascular lumen, the inventors treated tumors with x-rays. Tumors were treated with 250 kV x-rays as the inventors have previously described (Hallahan *et al.,* 1995), with 2, 4, or 10 Gy at a dose rate of 1 Gy per minute. At 1, 6, and 24 h after irradiation, mice were sacrificed by intraperitoneal injection of xylazine and ketamine.

To determine whether P-selectin is present in the vascular endothelium of tumors, the inventors utilized immunohistochemistry for P-selectin. Formalin fixed tumors were embedded in paraffin blocks and sectioned (5 µm thick). Sections were placed unto Superfrost Plus glass slides (Fisher Scientific). Tissue sections were baked at 60°C for 1 h, cleared in xylene, and hydrated through a descending alcohol series to distilled water. After brief washing in ddH₂O, endogenous activity was blocked by treatment of the sections with 3% hydrogen peroxide in methanol for 20 min. Two tissue sections from each case were then incubated overnight at 4°C at a titer of 2.5 µg/ml for anti-P-selectin and anti-GP-IIIa antibodies. One slide from each sample was treated in a similar fashion and incubated overnight in normal serum immunoglobulin (Ventana Medical Systems, Tucson, AZ). The immunohistochemical staining was performed on a Ventana Gen¹¹ system (Ventana Medical Systems). The Ventana Gen¹¹ uses an indirect strepavidin-biotin system conjugated with horseradish peroxidase for detecting the immuno-complex and diaminobenzidine as a substrate for localization. The Ventana Gen¹¹ uses a cartridge delivered avidin/biotin blocking kit to block endogenous biotin. The immunostained sections were counterstained with hematoxylin, dehydrated through an ascending alcohol series, cleared, and coverslipped. Stained sections were imaged under a 40x objective. All blood vessels throughout the entire section were observed, and 3 to 5 sections were analyzed for each tumor.

P-selectin translocation was observed in all tumors including MCA4 in C3H mice, Lewis lung carcinoma in C57BL6 mice, and WIDR tumor xenografts in nude mice. P-selectin translocation to the vascular lumen occurred in all tumor types, independent of the implantation site (brain, flank, or hind limb), strain of mouse (C3H, C57BL6, or nude), and species (mouse versus rat). Untreated control and x-irradiated tumors were sectioned and stained with anti-P-selectin antibody.

To determine the threshold and plateau doses for induction of P-selectin translocation, the inventors utilize 1, 2, 4, 10, and 20 Gy. The inventors observed no translocation of P-selectin in tumors treated with 1 Gy. Efficient translocation of P-selectin occurred following irradiation with 2 Gy. There was no increase in P-selectin translocation or degree of staining at 1 h, when higher doses of x-rays were used. This finding suggested that P-selectin translocation to the vascular lumen is an all or none type of response at 1 h with a threshold dose of 2 Gy. MCA4 tumors in C3H mice were excised and fixed at 1 h following irradiation and sections were stained with anti-P-selectin antibody.

The inventors studied later time points in irradiated tumors to determine whether P-selectin expression increased over 24 h. The inventors compared tumors treated with 2 and 10 Gy and found that there was no increase in expression over 24 h in tumors treated with 2 Gy. On the other hand, 10 Gy did produce an increase expression that accumulated over 24 h. P-selectin staining was present at a low baseline level at 1 h, and increased at 6 and 24 h.

The vascular endothelium in the brain is distinct from the endothelium in the periphery (Barkalow *et al.,* 1996). Moreover, Weibel-Palade bodies have been identified in blood vessels within gliomas (Miyagami and Nakamura, 1996). To determine whether malignant gliomas induced in the brain have a distinct P-selectin expression as compared to that in peripheral tumors, the inventors induced C6 gliomas in the brains of Wistar rats.

Intracranial gliomas were induced by stereotactic injection of rat C6 glioma cells into rat brains. C6 cells (ATCC) were received at passage 35 and maintained in Ham's F10 medium with 15% horse serum, 2.5% fetal bovine serum, and 10 mM HEPES. Growing cells were trypsinized and resuspended in PBS at 10⁸ cells/mL. Male Wistar rats (250-300 g) (Charles River, Wilmington, MA) were anesthesized with a mixture of ketamine (90 mg/kg) and xylazine (10 mg/kg) and placed in a stereotactic frame (David Kopf Instruments, Tujunga, CA). The head was shaved and the skin incised, and a hole was drilled in the skull with a 1.8 mm trephine (Fine Science Tools Inc., Foster City, CA). Ten microliters of cell suspension were injected 4 mm beneath the surface of the skull with a 50 ml Hamilton syringe 2 mm from the midline and 2 mm anterior to the coronal suture. The skull was sealed with dental cement, the wound was stitched, and the animals were kept in separate cages for 2-3 days to prevent mutilation. Thirteen days after implantation, the animals were irradiated. They were lightly anesthesized (0.75 of the regular dose of drugs on the first session, 0.5 in all further sessions) and placed in a cylinder custom made from 2 mm lead. The head protruded from a hole on the top for exposure. Irradiation was performed in a ¹³⁷Cs irradiator at 395 cGy /min. The entire brain was irradiated with 4 Gy. At 1, 6, 24, and 48 h after irradiation, the animals were anesthesized again, and the brains were perfused with 10 mM sodium cacodylate, pH 7.0, 1.5% formaldehyde, 0.1 % glutaraldehyde. The brains were further fixed in formaline, embedded in paraffin, and sectioned. Five micron sections were processed and stained with hematoxylin-eosin.

The normal brain blood vessels showed no P-selectin in untreated controls or following irradiation. On the other hand, gliomas showed P-selectin staining in the endothelium of untreated tumors. At 1 h after irradiation, P-selectin staining at the blood-tissue interface increased. At 6 h after irradiation, P-selectin staining in the lumen of blood vessels increased intensely. The entire brains of the rats and C6 gliomas induced in Wistar rats were treated with 4 Gy, and the brains were fixed, sectioned, and stained with anti-P-selectin antibody immunohistochemistry.

P-selectin in platelets may account for the time dependent increase in staining within the vascular lumen after irradiation. The inventors therefore utilized immunohistochemistry for platelet antigen GP-IIIa to differentiate between endothelial and platelet localization of P-selectin. GP-IIIa is a platelet antigen that is not found in the vascular endothelium. The inventors utilized anti-GP-IIIa antibodies to determine whether the time-dependent increase in P-selectin staining is due to platelet aggregation. Lewis lung carcinoma tumors in C57BL6 mice were irradiated and stained with anti-GP-IIIa antibody. The inventors found little GP-IIIa staining in blood vessels at 1 h following irradiation. However, GP-IIIa staining increased at 6 and 24 h following irradiation. These findings indicate that the increased P-selectin staining within the vascular lumen of irradiated tumors was, in part, due to platelet aggregation. Lewis lung carcinoma tumors were induced in C57BL6 mice and treated with 10 Gy. Tumors were excised and fixed at 1 h, 6 h and 24 h after irradiation.

These findings indicate that ionizing radiation induces P-selectin exocytosis from the vascular endothelium of neoplasms, which is associated with aggregation of platelets and P-selectin staining on platelets. The inventors observed that P-selectin was present in the vascular endothelium of all tumor types, regardless of species or implantation site. Moreover, the inventors observed that radiation-induced P-selectin translocation was associated with platelet aggregation within the irradiated tumor blood vessels. P-selectin translocation is specific to the microvasculature of malignant gliomas and is not present in the blood vessels of the irradiated normal brain. Moreover, radiation-induced platelet aggregation in tumor vessels was abrogated in P-selectin knockout mice. These data indicate that the vascular endothelium within neoplasms responds to ionizing radiation by translocating P-selectin to the vascular lumen, with subsequent platelet aggregation.

The mechanism by which ionizing radiation induces the translocation of P-selectin to the vascular lumen is presently unknown, but may be related to findings which have shown that WPB exocytosis is inhibited by microtubular depolymerizing agents (Sinha and Wagner, 1987). These findings implicate motor proteins in the movement of WPB to the cell membrane. This rapid response within the irradiated vascular endothelium implicates the function of these contents in initiating the biological response to radiation. The irradiated endothelium activates circulating blood components rapidly after x-irradiation.

Rapid exocytosis of WPB contents plays a critical role in hemostasis of injured blood vessels (reviewed in Caen and Rosa, 1995). In the present study, the inventors found that P-selectin translocation to the vascular lumen is followed by platelet aggregation and subsequent P-selectin expression on the surface of platelets. These data indicate that P-selectin translocation may contribute to the response of tumor blood vessels to ionizing radiation.

P-selectin has been shown to contribute to thrombotic vasculitis following stimulation with inflammatory mediators (Subramaniam *et al.,* 1996). The Schwarzman reaction consists of thrombosis and inflammation in tissues treated with local TNF injection followed by systemic administration of TNF (Shwartzman, 1928). The inventors have demonstrated that local administration of TNF combined with local irradiation results in thrombotic vasculitis within tumor vessels (Hallahan *et al.,* 1995; Mauceri *et al.,* 1996). This results in necrosis and inflammatory cell infiltration analogous to the Schwarzman reaction. The present study suggests a paradigm for mechanisms of interaction between TNF and radiation. TNF primes tumor blood vessels, with increased expression of cell adhesion molecules. Ionizing radiation, in turn, activates the endothelium. This interaction results in thrombosis of tumor blood vessels and subsequent necrosis of tumors. The therapeutic implication of radiation activation of the vascular endothelium is that the precise localization of ionizing radiation by modem day radiation therapy may allow for localized thrombotic vasculitis in neoplasms. Additionally, the inventors have injected ¹²³I conjugated anti-P-selectin antibodies into the aorta of mice with hindlimb tumors or the carotid of rats with brain tumors, and these radioimmunoconjugates localized to irradiated tumors. A four fold enhancement in the localization of radioconjugated antibody in irradiated tumors was detected by autoradiography and scintigraphy when compared to unirradiated controls, demonstrating that binding or targeting components to P-selectin can be specifically targeted to tissues after inducing P-selectin translocation.

### EXAMPLE XVIII

### P-selectin-dependent platelet aggregation in blood vessels of x-irradiated tissues

To determine whether platelet aggregation in irradiated blood vessels is associated with WPB exocytosis, the inventors utilized immunohistochemical analysis of the WPB components P-selectin and vWF.

C57BL6 mice were irradiated as the inventors have described (Hallahan and Virudachalam, 1997a; Hallahan and Virudachalam, 1997b). Ten, 30, 60, or 120 min after irradiation, mice were euthanized by intraperitoneal injection of xylazine and ketamine. Tissues were fixed in formalin and embedded in paraffin.

Tissue sections were baked at 60°C for 1 h, cleared in xylene, and hydrated through a descending alcohol series to distilled water. After brief washing in ddH₂O, endogenous activity was blocked by treatment of the sections with 3% hydrogen peroxide in methanol for 20 min. Two tissue sections from each mouse were then incubated overnight at 4°C at a titer of 2.5 µg/ml for anti-P-selectin antibody (Pharmingen, San Diego, CA) or platelet antibody anti-GP-IIIa. One slide from each sample was treated in a similar fashion and incubated overnight in normal serum immunoglobulin (Ventana Medical Systems, Tucson, AZ). The immunohistochemical staining was performed on a Ventana Gen (Hallahan and Virudachalam, 1997) system (Ventana Medical Systems) which uses an indirect strepavidin-biotin system conjugated with horseradish peroxidase for detecting the immunocomplex and diaminobenzidine as a substrate for localization, as well as a cartridge delivered avidin/biotin blocking kit to block endogenous biotin. The immunostained sections were counterstained with hematoxylin, dehydrated through an ascending alcohol series, cleared, and coverslipped.

Immunofluorescence staining was performed as the inventors have described (Hallahan and Virudachalam, 1997; Hallahan *et al.,* 1997). Tissue sections (5 µm) were mounted on slides and labeled with anti-GP-IIIa antibodies as described above. After incubation with biotinylated secondary antibody, sections were incubated with 200 µl of Avidin-Cy3 (10 µg/ml) for 30 min in a humid chamber at room temperature. Avidin-Cy3 (Amersham Corp., Arlington Heights, IL), 5 µg/mL was added to 200 µL of blocking buffer and filtered through a 0.2-µm Millipore filter, before addition of the fluorochrome to slides. Coverslips were removed, and sections were washed with 4X SSC/0.1% Triton X at room temperature. Slides were counterstained in DAPI and rinsed with 2X SSC for 10 sec. The slides were then coverslipped with antifade and blotted. Immunofluorescent images were visualized with a Zeiss Photomicroscope III fluorescence microscope.

Prior to irradiation, P-selectin was localized to the vascular endothelium. Within 60 min of irradiation, P-selectin was translocated to the blood-tissue interface. P-selectin extended from the irradiated endothelium into the vascular lumen. The inventors observed that, at four hours after irradiation, platelet aggregates were stained within these blood vessels. Platelet aggregations continued to accumulate in irradiated blood vessels for 24 h. The inventors irradiated lungs of mice and stained for platelet aggregation using anti-P-selectin and anti-GP IIIa antibody. At one h following irradiation, there was no increase in platelet aggregation, whereas P-selectin in the vascular endothelium was translocated to the blood tissue interface.

Because P-selectin contributes to the slowing and activation of platelets, the inventors studied radiation-induced platelet aggregation in irradiated tissues. To quantify platelet aggregation, the inventors stained tissue sections from irradiated lungs with anti-P-selectin antibody. Blood vessels in ten 40x objective fields were scored for the presence or absence of platelet aggregates.

Blood vessels were identified by autofluorescence of red blood cells using the FITC filter. The inventors measured fluorescence intensity, in pixels, within blood vessels. Ten blood vessel in each tissue section were photographed using CCD camera. Fluorescence intensity was measured by NIH Image software as the inventors have described (Hallahan and Virudachalam, 1997). Experiments were performed 3 to 4 times. All data were analyzed by use of Statistica for Windows software (StatSoft, Inc., Tulsa, OK).

The percentage of irradiated blood vessels with platelet aggregates increased over time. Four h after irradiation, 40% of blood vessels showed platelet aggregation. The percentage of pulmonary vessels with aggregates increased to 75% at 24 h (p = 0.01). Thereafter, platelet aggregation decreases. C57BL6 mice were treated with thoracic irradiation, and tissues were fixed at the indicated times.

To determine whether radiation-induced platelet aggregation was associated with leukocyte adhesion, the inventors studied leukocytes staining within platelet aggregates. At one h after irradiation, the inventors found leukocyte adhesion to P-selectin at the blood tissue interface of the irradiated vascular endothelium. Six h after irradiation, leukocyte adhesion to platelet aggregates was observed. At 24 h following irradiation, the inventors observed leukocyte staining in 20 percent of platelet aggregates. The inventors quantified leukocyte localization to platelet aggregates over time. The time course of leukocyte adhesion correlated with the number of blood vessels showing platelet aggregation. P-selectin immunohistochemistry of irradiated tissues was conducted in lung, intestine, brain and kidney.

To determine whether radiation-induced P-selectin translocation to the vascular lumen is associated with platelet aggregation, the inventors studied the number of platelet aggregates in murine blood vessels that stained positively for P-selectin, as compared to tissues without P-selectin. The inventors found that P-selectin translocation occurred in pulmonary venules, but not in the pulmonary microvascular endothelium. P-selectin translocation also occurred in the irradiated small intestine and colonic endothelium. P-selectin staining was not observed in the irradiated brain, or kidney. Radiation-induced platelet aggregation is associated with P-selectin translocation, anti-platelet antibodies were incubated with tissue sections from irradiated lung, intestine, brain and kidney.

The inventors quantified platelet aggregation in each of these tissues by use of immunofluorescence of platelet antigen. A significant increase in the number of blood vessels staining positively for platelet aggregation in pulmonary vessels, and in vessels of the small and large intestine was seen. In contrast, blood vessels that did not stain positively for P-selectin, such as the brain and kidney showed no increase in platelet staining. Moreover, the pulmonary microvascular endothelium did not contain P-selectin and did not show platelet aggregation. C57BL6 mice were treated with total body irradiation, and tissues were fixed at 24 h after irradiation. Platelet aggregates were counted in 40 blood vessels of lung, small intestine, colon, brain, kidney. Platelet aggregation occurred in irradiated blood vessels in which P-selectin translocation was observed (*eg*. lung, tumors and intestine), but not in blood vessels lacking radiation-induced P-selectin translocation (kidney or brain).

The inventors hypothesize that radiation-induced P-selectin translocation to the lumen contributes to platelet activation by slowing circulating platelets to allow aggregation in the irradiated vessel and not downstream from it. To determine the role of P-selectin in radiation-induced platelet activation, the inventors studied platelet aggregation in P-selectin-deficient mice treated with thoracic irradiation.

The P-selectin knockout mouse has prolonged breeding times (Subramaniam *et al.,* 1996) and therefore serves as a model. To determine whether P-selectin is required for radiation-induced platelet aggregation, the inventors irradiated P-selectin knockout mice and stained for platelets utilizing the platelet antibody anti-GP IIIa.

The inventors found marked attenuation of platelet aggregation in tissues of irradiated P-selectin knockout mice. The inventors utilized immunofluorescence staining for platelet antigens to measure of the difference in platelet aggregation in the P-selectin knockout mouse as compared to the P-selectin +/+ mouse. The inventors observed increased platelet aggregation in blood vessels at 6 h following irradiation of wild-type mice. Immunoflurescence intensity of platelet aggregation was abrogated in the P-selectin knockout mouse.

To determine whether x-ray-induced platelet aggregation is also attenuated in angiogenic blood vessels in P-selectin -/- mice, the inventors induced syngeneic tumors in the hind limbs of P-selectin +/+ mice and knockouts. GL261 gliomas were induced in P-selectin -/and P-selectin +/+ C57BL6 mice.

Lewis lung carcinoma cells were maintained in F-12/DME 50% mixture and 7% fetal calf serum, and P/S. Mice were bred in our transgenic mouse core laboratory. Subconfluent tumor cells were trypsinized, washed, and injected subcutaneously into the hindlimb.

Lewis lung carcinoma (10⁶ cells) were injected into hindlimbs of C57BL6 mice (Jackson Labs, Bar Harbor, ME) and were grown to 400 mm³. Tumors were treated with x-irradiation using 250 kV x-rays as the inventors have previously described (Hallahan *et al.,* 1995). Tumors were treated with 10 Gy at a dose rate of 1Gy per min.

P-selectin -/- mice were obtained from Jackson Laboratories, Bar Harbor, ME (Mayadas *et al.,* 1993). Lewis lung carcinoma (10⁶) were injected subcutaneously into the hindlimb of P-selectin -/- mice. Tumors were growth to a volume of 400 mm³ and irradiated with 10 Gy. At 6 and 24 h following irradiation, mice were euthanized by intraperitoneal injection of xylazine and ketamine. Tumors were fixed in formalin and embedded in paraffin. Tumors were sectioned and stained with the anti-GPIIIa antibody as described above.

Irradiated tumors in P-selectin +/+ mice showed platelet aggregation at 6 and 24 h. Tumors in P-selectin -/- mice, however, showed attenuation of GP-IIIa staining in blood vessels at 6 and 24 h after irradiation. This model supports the original findings that radiation-induced platelet aggregation is attenuated in tissues that lack P-selectin translocation to the blood-tissue interface. Radiation-induced platelet aggregation in GL261 gliomas in C57BL6 mice was detected using anti-GP-IIIa antibody in stain tumor sections from P-selectin +/+ and P-selectin -/- mice.

To study the physiologic significance of P-selectin-dependent platelet aggregation, P-selectin -/- and P-selectin +/+ mice were treated with 10 Gy. At day 5, the knockout mice showed tachypnea and hypomotility. Wild type mice demonstrated no reduction in movement and no tachypnia. To determine the cause of respiratory distress in P-selectin -/- mice treated with radiation, the lungs were dissected, fixed, and sectioned at 48 h and 7 days following irradiation. Lungs from P-selectin -/- mice showed red blood cells within the alveoli within 48 h of irradiation. The lungs of mice with respiratory distress (day 7) showed pulmonary hemorrhage with no evidence of bacterial infection either by gram staining or bacterial culture. The mice were treated with 10 Gy and lungs were fixed and sectioned for H & E staining.

The inventors found that platelet aggregation occurred in the lungs of P-selectin +/+ mice following thoracic irradiation. In contrast, P-selectin-deficient mice showed no platelet aggregation within the irradiated lungs. P-selectin is absent from both platelets and endothelium in P-selectin deficient mice (Subramaniam *et al.,* 1996; Mayadas *et al.,* 1993). These data indicate that the rapid translocation of P-selectin to the blood-tissue interface plays an important role in slowing the flow of platelets within injured blood vessels.

These studies demonstrate that P-selectin participates in the pathogenesis of platelet aggregation in blood vessels of irradiated tissues. The P-selectin knockout mouse represents a model for the study of the physiologic significance of these components in the pathogenesis of radiation-induced tissue injury. Taken together with the other studies described above in the non-knockout mice, these data indicate that P-selectin translocation to the vascular lumen plays an important role in the pathogenesis of radiation-induced platelet aggregation.

The clinical significance of this finding is the high incidence of radiation pneumonitis in bone marrow transplant patients treated with single dose total body irradiation. The present study shows that platelets aggregate in the lungs following thoracic irradiation. The inventors observed leukocyte adhesion to platelet aggregates. Leukocyte recruitment by platelet P-selectin into a thrombus has been demonstrated in primates (Palabrica *et al.,* 1992). The contribution of platelets to radiation-induced tissue injury may include inflammatory cell activation (Furie and Furie, 1995). Inflammatory cells adhere to the irradiated endothelium within 4 h of irradiation (Panes *et al.,* 1995; Fliss and Menard, 1994). Platelet aggregation in irradiated vessels was associated with leukocyte adhesion in the present study. Activated platelets express P-selectin on the membranous surface and thereby activate inflammatory cells (Furie and Furie, 1995; Tsuji *et al.,* 1994). P-selectin adhesion to circulating leukocytes induces a conformational change of integrin B2 (Evangelista *et al.,* 1996). Such a conformational change is required for adhesion to ICAM. Taken together with the findings that P-selectin activates inflammatory cells, the inhibition of platelet adhesion to the vascular endothelium may attenuate radiation-induced tissue injury. Moreover, enhancement of platelet aggregation within tumor blood vessels can achieve obliteration of tumor vasculature (Hallahan *et al.,* 1995; Mauceri *et al.,* 1996). The inventors contemplate that procoagulants, including tumor necrosis factor can enhance the effects of tissue injury or tumor control by working in conjunction with agents that promote P-selectin activation. The inventors also contemplate that agents that attenuate the action of P-selectin, including anti-P-selectin antibodies, can also attenuate tissue injury.

### EXAMPLE XIX

### Additional Examples of the Methods of the Invention

To determine whether intercellular signal transduction participates in the radiation response, the inventors will study the molecular response to ionizing radiation in which intercellular signal transduction occurs independently of gene induction.

Radiation-mediated intercellular signaling involves the release of preformed molecules that in turn activate circulating leukocytes and platelets. These molecules include those contained within endothelial cell Weibel-Palade bodies such as P-selectin IL-8 and von Willebrand factor. In addition to Weibel-Palade bodies, other endothelial cell granules serve as reservoirs for constitutively expressed proteins. These reservoirs include vesiculovacuolar organelles which are present in tumor microvasculature (Qu *et al.,* 1995), multivesicular bodies, and tissue factor pathway inhibitor (TFPI)-specific granules (Lupu *et al.,* 1995). There are two general mechanisms by which these stored proteins participate in intercellular signaling: secretion and translocation to the membrane. The inventors will analyze both the medium and the cell membrane to determine whether stored proteins are translocated in response to ionizing radiation. LDH is a marker of cell lysis and will serve as a control for protein secretion.

Endothelial cells from veins, arteries, pulmonary microvasculature, dermal microvasculature, and tumors will be irradiated as the inventors have described (Hallahan *et al.,* 1996a, Hallahan *et al.,* 1995a, Hallahan and Virudachalam, 1997a, Hallahan *et al.,* 1996b). Endothelium will be stained for the following proteins: antibodies to P-selectin will determine Weibel Palade body translocation, antibody to tissue factor pathway inhibitor will be used for the TFPI-specific granules, antibodies to endothelin will be used for the multivesicular bodies (Doi *et al.,* 1996, Turner and Murphy, 1996), antibodies to VEGF will be used to study translocation of vesiculovacuolar organelles. Control antibody staining for PECAM-1 will be used during irradiation because the inventors have found that this protein is not altered by x-irradiation of endothelial cells. Untreated endothelial cells will serve as a negative control and thrombin-stimulated endothelial cells will serve as a positive control.

In separate studies, mice will be treated with total body irradiation (TBI) using 2, 5, 10, and 15 Gy. The brain, lung, intestine, liver, kidneys, and tumors will be dissected, fixed in paraffin and sectioned by microtome, as the inventors have previously described (Hallahan and Virudachalam, 1997a, Hallahan and Virudachalam, 1997a, Hallahan *et al.,* 1995b). Tissues will be dissected and fixed at 10 min, 30 min, 60 min, and 120 min after irradiation. These studies will allow the inventors to determine whether there is a time- or dose-dependent translocation of cytoplasmic granules following irradiation.

Mice will be treated with total body irradiation. Ten, 30, 60, 120 min after irradiation, mice will be euthanized by intraperitoneal injection of barbiturate. Tissues will be fixed in formalin and embedded in paraffin as the inventors have described (Hallahan *et al.,* 1995b, Hallahan *et al.,* 1996b). Five µm sections of each tissue will be mounted onto Superfrost Plus slides (Fisher Scientific, Pittsburgh, PA) baked at 60°C for 1 h, cleared in xylene, and hydrated through a descending alcohol series to distilled water. The hydrated sections will be incubated with Protease II (Ventana Biotech) for 8 min at 42°C. Endogenous activity will be blocked by treatment of the sections with 3% hydrogen peroxide in methanol for 20 min. Two tissue sections from each case will be then incubated overnight at 4°C at a liter of 2.5 µg/ml rat anti-mouse P-selectin (Pharmingen). One slide from each sample will be treated in a similar fashion and incubated overnight in normal serum immunoglobulin (Ventana Medical Systems, Tucson, AZ). The immunohistochemical staining will be performed on a Ventana Gen¹¹ system (Ventana Medical Systems). The Ventana Gen¹¹ utilizes an indirect strepavidin biotin system conjugated with horseradish peroxidase for detecting the immunocomplex and diaminobenzidine as substrate for localization. The Ventana Gen¹¹ uses a cartridge delivered avidin/biotin blocking kit to block endogenous biotin. The immunostained sections will be counterstained with hematoxylin, dehydrated through an ascending alcohol series, cleared, and coverslipped.

Primary culture vascular endothelial cells will be grown to 80% confluence on glass slides and irradiated with a GE Maxitron x-ray generator as previously described (Hallahan *et al.,* 1996a, Hallahan *et al.,* 1995a). Cells will be labeled with anti-P-selectin antibodies as a positive control. HUVECs will be grown on glass slides and treated with interleukin-1 (IL-1) or x-rays or under identical conditions without x-rays or cytokines. Thirty and 60 min after treatment, cells will be fixed in 4% paraformaldehyde for 1 h. Nonspecific binding of antibody will be blocked by incubation with 5% goat serum for 30 min at 37°C. Cells will be washed with buffer and incubated with mouse anti-E-selectin antibody, followed by incubation with FITC-conjugated goat anti-mouse IgG. Following washings, cells will be visualized with a Zeiss Photomicroscope III equipped with an epifluorescence condenser, which converts a photomicroscope to a fluorescence microscope for incident-light excitation. Slides will be mounted and examined for fluorescence and by phase microscopy.

Immunofluorescence will replace HRP immunohistochemistry when quantitation will be needed, because fluorescence intensity can be measured. Lung sections (5 µm) will be mounted on slides and labeled with antibodies as described above. Following incubation with biotinylated secondary antibody, blocking solution will be added for 30 min in a humid chamber at 37°C. Avidin-Cy3 (Amersham, Arlington Heights, IL) 5 µg/mL will be added to 200 µL of blocking buffer and filtered through a 0.2 µm millipore filter. Avidin-Cy3 solution will be added to tissue sections, coverslipped and incubated for 30 min in a humid chamber at 37°C. Coverslips will be removed and sections will be washed using 4X SSC/0.1% Triton X at 39°C. Slides will be counterstained in DAPI and rinsed with 2X SSC for 10 sec. Slides will then be coverslipped with antifade and blotted. Immunofluorescence will be visualized using UV microscopy and NIH Image software as described (Hallahan and Virudachalam, 1997a).

During quantitation of ICAM-1 *in vivo,* immunofluorescence will be visualized using UV microscopy, NU200 and NIH Image software. DAPI staining of nuclei will be used as a control to verify that fluorescence will be measured in the same number of cells in each lung section. Fifty nuclei will be framed and anti-P-selectin immunofluorescence will be determined by use of NIH Image software as the inventors have described (Hallahan and Virudachalam, 1997a). Fluorescence intensity will be determined for each pixel within the flamed cells and the number of fluorescent pixels will be counted by use of NIH Image. The increase in the number of pixels showing fluorescence will be determined as the inventors have described (Hallahan and Virudachalam, 1997a). The mean and SEM of anti-ICAM-1 immunofluorescence of three lungs will be determined for each dose of thoracic irradiation.

The inventors expect that ionizing radiation will activate a signal transduction pathway in endothelial cells that consists of calcium mobilization and activation of PKC. This will, in turn, activate translocation of cytoplasmic granules to the cell surface. The inventors also expect that cytoplasmic granule translocation will vary among the endothelium of each of the tissue types studied. Once translocation of cytoplasmic granules has been characterized, the inventors will study the mechanisms of intercellular signaling in the irradiated vascular endothelium as described below.

The following method may be used to determine whether there is a dose-dependent reservoir translocation in the irradiated vascular endothelium, and the optimum dose for reservoir translocation.

The inventors will determine whether the signaling pathways occur after low dose irradiation or whether high dose irradiation is required. The inventors' data suggest that a threshold dose and plateau dose are related to the percentage of cells undergoing apoptosis. The inventors contemplate that high doses will induce a more intense and rapid onset of apoptosis which will obscure the exocytosis that is observable at lower doses. The inventors will test this by studying the response to a range of doses.

Vascular endothelial cells will be grown, irradiated with doses of 1, 2, 5, 10, and 20 Gy, and will then be fixed and stained at various time points ranging from 10 min to 6 h using the methods described previously described herein.

The inventors expect that a dose dependent translocation of cytoplasmic granules will occur in irradiated vascular endothelium. The inventors' data indicate that a threshold dose will in the range of 1-2 Gy, and a plateau is reached at approximately 10 Gy. The significance of these studies is that intercellular signaling occurs in clinically relevant radiation doses. The data obtained from this study will be applied to the methods described below. The inventors will determine whether apoptosis diminishes translocation.

The inventors will determine the mechanisms of Weibel-Palade body translocation in the irradiated vascular lumen. The inventors' data indicate that P-selectin within Weibel-Palade bodies is translocated to the cell membrane within minutes of irradiation (Hallahan and Virudachalam, 1997a). Immunofluorescence was used to visualize the translocation of P-selectin to the cell surface. This showed a rapid migration of P-selectin protein that occurred within 30 min of irradiation. Within 60 min, P-selectin was no longer detected within irradiated endothelial cells. Hypoxia-induced Weibel-Palade body exocytosis is dependent upon the influx of extracellular calcium (Pinsky *et al.,* 1996). Moreover, superoxide induced von Willebrand Factor exocytosis is inhibited by the intracellular calcium chelator BAPTA and is due to release of calcium from intracellular stores (Vischer *et al.,* 1995). Reactive oxygen species increase intracellular calcium in endothelial cells (Dreher and Junod, 1995). The inventors have previously shown that BAPTA inhibits x-ray-mediated signal transduction (Hallahan *et al.,* 1994c). Increased intracellular calcium is also a mechanism of radiation-mediated intracellular signal transduction within tumor cells [calcium]. The inventors' results indicate that the calcium chelator attenuates x-ray-mediated P-selectin translocation. The inventors contemplate that intracellular calcium flux contributes to Weibel-Palade body exocytosis. The inventors will utilize BAPTA, EDTA, and inhibitors of calcium channels. The inventors will also determine the mechanism of radiation-mediated increased intracellular calcium.

To quantify radiation-mediated increases in intracellular calcium, endothelial cells will be grown as the inventors have described (Hallahan *et al.,* 1996a, Hallahan *et al.,* 1995a) and labeled with Fura-2 as the inventors have described (Hallahan *et al.,* 1994c). Intracellular calcium will then be quantified by use of UV microscopy as the inventors have described (Hallahan *et al.,* 1994c). Endothelial cells will be irradiated as the inventors have previously described (Datta *et al.,* 1992). The inventors will use a dose range of 1-10 Gy to determine whether there is a dose-dependent increase in intracellular calcium.

In separate studies, endothelial cells will be grown in the absence in extracellular calcium using the extracellular calcium chelator EDTA as the inventors have described (Hallahan *et al.,* 1994c). Calcium channel blockers will also be added to endothelial cell cultures prior to irradiation. Cells treated with EDTA or calcium channel blockers will then be stained with the anti-P-selectin antibody for immunofluorescence to visualize translocation of Weibel Palade bodies as described above. The inventors will also quantify von Willebrand factor released into the medium by use of ELISA assay. These studies will allow the inventors to determine whether increased intracellular calcium results from extracellular or intracellular stores. The inventors will also determine whether increased intracellular calcium is required for Weibel Palade body exocytosis.

Intracellular calcium concentrations ([Ca2⁺]*i*) will be measured using the Ca²⁺ sensitive fluorophore fura-2 (Molecular Probes) in standard buffer solution (138mM NaCl, 2mM CaC 12, 1 mM MgC12, 5 mM KC1. HEPES and 10 mM glucose adjusted to pH 7.4 with NaOH) at 22-24°C. Glass cover slips of cells will be loaded with fura-2 by incubation with fura-2 acetoxymethy ester (5 µM in standard buffer for 30 min at 30°C. Glass cover slips of cells will be loaded with fura-2 by incubation with fura-2 acetoxymethy ester (5 µM in standard buffer for 30 min at 30°C) and then will behed for 40 min at the same temperature. The cover slips will be mounted in the perfusion chamber, which will be positioned on the opening of the microscope stage. A suitable cell will be identified and aligned within the collimated viewfinder of the photomultiplier tube, as described previously (Hallahan *et al.,* 1994c). The cells will be perfused continuously with a solution containing Na- and Ca²⁺ as detailed below. Drugs will be added to the external solutions. Emission will be measured alternately at excitation wavelengths of 340 and 380 nm, and the ratio of the emission intensities at 510 nm will be translated in [Ca²⁺]*i*, using a predetermined calibration curve. Values for [Ca²⁺]*i* are displayed each second and represent the mean calculated [Ca²⁺]*i* for ratios measured every 16 ms. Rapid solution change in the cell superfusion system will be obtained by maintaining a low chamber fluid volume of 200-400 µl and laminar flow perfusion of 2 ml min. The tubing between the medium reservoir and the inlet to the chamber delayed the onset of the solution change by approximately 15 s. Results will be corrected for this perfusion delay. Cells will be exposed to ⁹⁰Sr using an applicator (Amersham, Arlington Heights, IL) at a dose rate of 4 Gy min for 5 to 15 min, while [Ca²⁺]*i* will be quantified. The radiation dose will be quantified using thermoluminescent dosimeters (TLDs). The TLDs will be placed on cover slips and irradiated for 1 min during [Ca²⁺]*i* quantification and the radiation dose will be determined as described. After irradiation, [Ca²⁺]*i* will be quantified for an additional 30 min. Ca²⁺ free solutions will be prepared by using standard buffer solution with Ca²⁺ omitted and 20 µM AM-BAPTA added.

The inventors contemplate that intracellular calcium levels will increase following x-irradiation. The inventors contemplate that this may result from influx of calcium from the extracellular solution. The inventors also contemplate that calcium chelation will attenuate radiation-mediated Weibel Palade body exocytosis. Once these studies determine a mechanism of calcium flux, the inventors will determine the role of [Ca²⁺]*i* in PKC activation and intercellular signal transduction.

The inventors have previously shown that protein kinase C is rapidly activated following irradiation of a number of cell types (Hallahan *et al.,* 1991). The inventors' data indicate that the PKC inhibitor calphostin attenuates radiation-mediated P-selectin translocation to the cell membrane. The inventors contemplate that PKC is activated within irradiated endothelial cells and participates in signal transduction resulting in Weibel Palade body exocytosis. The inventors will quantify PKC activation within irradiated endothelial cells and the inventors will study phosphorylation of PKC substrates and determine whether PKC participates in Weibel Palade body translocation.

Vascular endothelial cells from veins, arteries, microvascular pulmonary endothelium, and dermal microvascular endothelium will be grown as the inventors have previously described (Hallahan *et al.,* 1996a, Hallahan *et al.,* 1995a, Hallahan and Virudachalam, 1997a, Hallahan *et al.,* 1996b). Cells will be irradiated with the high dose rate ⁶⁰Co irradiator because PKC is rapidly activated (Hallahan *et al.,* 1994a, Hallahan *et al.,* 1994c, Hallahan *et al.,* 1991a). Total cellular protein will be extracted and the phosphotransoferase activity of PKC will be determined as the inventors have previously described (Hallahan *et al.,* 1994c, Hallahan *et al.,* 1991 b).

In a separate study the inventors will study Weibel Palade body exocytosis in the presence of PKC inhibitors. Various PKC inhibitors including H7, calphostin and staurosporin will be used to determine whether these isoform specific inhibitors all attenuate Weibel Palade body exocytosis. In addition, the inventors will determine whether calcium dependent PKC isoform is required for signaling by use of the calcium chelator BAPTA.

The inventors expect that ionizing radiation will activate PKC and this in turn will phosphorylate cytoplasmic proteins required for Weibel Palade body exocytosis.

P-selectin gene induction is a mechanism of intercellular signaling within the brain vasculature. In addition to P-selectin exocytosis, the inventors contemplate that P-selectin gene expression is also increased within irradiated endothelial cells. The inventors will perform Northern blot analysis for the P-selectin gene following x-irradiation of various endothelial cell types.

Endothelial cells from vein, artery, lung, and dermis will be grown and irradiated as the inventors have previously described (Hallahan *et al.,* 1996a, Hallahan *et al.,* 1995a, Hallahan and Virudachalam, 1997a, Hallahan *et al.,* 1996b). RNA will be extracted at times ranging from 1 to 8 h. Northern blot analysis and densitometry will be used to quantify gene expression. Hybridization to GAPDH will be used to demonstrate equal loading of lanes.

Primary culture vascular endothelial cells will be exposed to 2 to 10 Gy (GE Maxitron X-ray generator) as previously described (Hallahan *et al.,* 1996a, Hallahan *et al.,* 1995a, Hallahan and Virudachalam, 1997a, Hallahan *et al.,* 1996b). RNA will be extracted using the single step guanidinium thiocyanate-phenol/chloroform method (Chomczynski and Sacchi, 1987) following irradiation. Control RNA from nonirradiated cells treated with otherwise identical conditions and RNA from irradiated cells will be size fractionated by 1% agarose formaldehyde electrophoresis. RNA gels will be then transferred to a nylon membrane (Genescreen Plus, New England Nuclear, Boston, MA). Northern blots will be hybridized to 32p labeled P-selectin cDNA probe and GAPDH expression will be quantified to verify equal loading of RNA into lanes followed by autoradiography for 3 days at -85°C with intensifying screens. RNA levels will be quantified by densitometry (Sherman *et al.,* 1991).

The inventors contemplate that P-selectin gene expression will be endothelial cell type specific. The inventors also contemplate that the response will be dose dependent, but that apoptosis will be associated with diminished expression at higher doses.

The inventors' data indicate that P-selectin is translocated to the luminal surface of blood vessels within 10-30 min. P-selectin remains at the blood-tissue interface of the vascular endothelium for approximately 1 h. After that time point, P-selectin is no longer present on immunofluorescent staining of endothelial *cells in vitro* or in irradiated vascular endothelium *in vivo.* The inventors performed ELISA assays to determine whether the P-selectin was released in the medium *in vitro* or into the serum *in vivo.* These studies demonstrated no detection of P-selectin outside of the vascular endothelium. Previous studies have shown that P-selectin is internalized following the transient exocytosis (Green *et al.,* 1994). Furthermore, studies have shown that apoptosis occur in vascular endothelial cells following irradiation. The inventors contemplate that the proteolytic enzymes participating in the apoptosis also induce proteolytic degradation of P-selectin following internalization. The inventors will study P-selectin expression in vascular endothelium over time and determine whether inhibition of proteolytic cleavage eliminates degradation.

The inventors have found that the disappearance of P-selectin in irradiated endothelial cells is dose dependent. It is more rapid and profound in cells treated with 10 Gy as compared with cells treated with 2 Gy. For that reason the inventors will compare intercellular P-selectin levels in cells exposed to 2 Gy and 10 Gy. Cells will then be treated with protease inhibitors including inhibitors of apoptosis mediated proteolytic cleavage such as zVAD-fmk, calpane, and crmA. Protein will be extracted and separated on SDS-PAGE and transferred to Western blots. Antibodies to P-selectin will be used for Western immunoblots and P-selectin immunopercipitation using techniques that the inventors have previously described (Hallahan et *al.,* 1991a, Rubin *et al.,* 1991, Brachman *et al.,* 1991). The inventors will determine the half-life of P-selectin following irradiation by use of ³⁵S-methionine labeling and immunoprecipitation.

The inventors contemplate that P-selectin will be degraded by proteases following doses of 5 Gy and greater. The inventors contemplate that less P-selectin degradation will occur in the range of 1-2 Gy. P-selectin may be internalized and recirculated as previous described. The inventors contemplate that protease inhibitors that are active in preventing apoptosis will also attenuate P-selectin degradation following irradiation.

The intercellular signaling represents a novel paradigm for the study of the mechanisms of the radiation response. Moreover, these studies will utilize the vascular endothelium as a model for two reasons: Vascular injury is a principal component in the pathogenesis of radiation-mediated tissue injury and organs in which the endothelium plays a crucial functional role are among the most radiation sensitive organs. The inventors contemplate that intercellular signaling contributes to the biological response to ionizing radiation.

The heterodimer CD11/CD18 (β2 integrin) on neutrophils is constitutively present on the plasma membrane and subcellular granules (Springer, 1990). CD11/CD18 on subcellular granules is translocated to the cell surface of neutrophils following leukocyte binding to selectins. Following neutrophil activation, this integrin achieves a more firm adhesion between the leukocyte and vascular endothelium and facilitates transendothelial migration (Springer, 1990). The inventors' data indicate that leukocytes accumulate in irradiated blood vessels within a few hours of irradiation. In addition, the inventors have found that selectins are upregulated in irradiated vascular endothelium. The inventors contemplate that radiation-mediate upregulation of P-selectin on the luminal surface of blood vessels activates circulating leukocytes to achieve more firm adhesion. The inventors will utilize immunofluorescence of CD11/CD18 on sections of irradiated blood vessels.

C57BL6 mice will be irradiated as described above. The tissues that show P-selectin translocation to the vascular lumen will be studied in this study. For example, the inventors have shown that P-selectin is translocated to the vascular lumen in the irradiated blood vessels within the thorax (Hallahan and Virudachalam, 1997a). Tissues will be fixed, paraffin embedded and sectioned as the inventors have described (Hallahan and Virudachalam, 1997a, Hallahan and Virudachalam, 1997a, Hallahan *et al.,* 1995b). Tissue sections will then be incubated with antibody to CD11/CD18 immunofluorescence microscopy and NIH image software will be utilized to quantify fluorescence as the inventors have described (Hallahan and Virudachalam, 1997a, Hallahan and Virudachalam, 1997a, Hallahan *et al.,* 1995b). The inventors will determine whether the surface expression of CD11/CD18 is upregulated in leukocytes within irradiated blood vessels.

The inventors contemplate that P-selectin will activate circulating neutrophils resulting in increased cell membrane expression of CD11/CD18. The contemplate expect that this is a mechanism of increased leukocyte margination within irradiated blood vessels. This will be verified by use of the P-selectin knockout mouse and by blocking antibodies to P-selectin.

Monocyte tethering by P-selectin induces the expression of monocyte chemotactic protein-1 to TNF secretion (Weyrich *et al.,* 1995) and tissue factor (Celi *et al.,* 1994). P-selectin-mediated monocyte production of MCP-1, tissue factor or TNF expression can be attenuated by ± P-selectin blocking antibody (Weyrich *et al.,* 1995, Celi *et al.,* 1994). Moreover, increased monocyte procoagulant activity was blocked by antibodies to tissue factor or the P-selectin counterreceptor LeX (Lo *et al.,* 1995). The inventors contemplate that P-selectin translocation to the vascular lumen will activate circulating leukocytes. The inventors will irradiated the vascular endothelium and assay for the induction of tissue factor, MCP-1, and TNF.

Monocytes will be isolated from peripheral blood by countercurrent elutriation as previously described (Elstad *et al.,* 1988). Monocytes will be added to irradiated endothelial cell cultures for 8 h. TNF, tissue factor (Celi *et al.,* 1994) and MCP-1 production will be quantified in the medium and cells by use of ELISA assays and northern blot analysis.

In a separate study, immunofluorescence will be used to determine whether monocytes in the irradiated vasculature demonstrate MCP, TF or TNF expression. The role of P-selectin in this induction will be determined by the use of the P-selectin knockout mouse (Mayadas *et al.,* 1993). Antibodies to each of the inducible factors will be incubated with tissue sections as described (Franco, 1997; Cell, 1994; Hallahan, 1997; Hallahan, 1997; Mauceri, 1996).

The inventors contemplate that radiation-induced P-selectin will activate monocytes to produce cytokines, based on the inventors' observations that P-selectin is translocated and can activate monocytes in culture (Weyrich *et al.,* 1995).

The inventors' data indicate that ionizing radiation induces the translocation of P-selectin within the vascular endothelium both *in vivo* and *in vitro.* The inventors have also found that leukocyte margination occurs in irradiated blood vessels. Previous studies have shown that the P-selectin knockout mouse has an attenuated inflammatory response. P-selectin binds to its counterreceptor on leukocytes to initiate a signaling cascade resulting in inflammatory cell activation (Weyrich *et al.,* 1995, Celi *et al.,* 1994, Pouyani and Seed, 1995). The inventors contemplate that ionizing radiation-mediated intercellular signaling from the vascular endothelium to circulating leukocytes will be attenuated when the P-selectin gene is deleted from the mouse model. The inventors will irradiate the P-selectin knockout mouse and study leukocyte activation within the irradiated blood vessels.

The inventors have obtained the P-selectin knockout mouse from Jackson Laboratories, and have bred them in the inventors' transgenic mouse core laboratory. When the mice reach the age of 5-6 wk, they will be irradiated as the inventors have previously described (Hallahan and Virudachalam, 1997a). The tissues that are identified as models will be excised and analyzed for leukocyte activation. The inventors have found that the vascular endothelium in the irradiated lung shows P-selectin translocation following thoracic irradiation (Hallahan and Virudachalam, 1997a). Therefore, the lung will serve as one model tissue and will be studied by immunohistochemistry using techniques that the inventors have previously described (Hallahan and Virudachalam, 1997a, Hallahan and Virudachalam, 1997a). A time course of tissue fixation will be 1, 3, 5, and 10 h. The inventors will stain tissues for the presence of leukocytes by use of CD45 antibody as the inventors have described (Hallahan and Virudachalam, 1997a).

The inventors contemplate that the P-selectin knockout mouse will have an attenuated inflammatory response with a reduction in the number of inflammatory cells, reduced CD11/CD18 translocation and diminished expression of P-selectin/inducible genes.

The inventors contemplate that blocking von Willebrand factor binding to its counterreceptor will attenuate platelet activation by the irradiated vascular endothelium.

Previous studies have demonstrated the feasibility of inhibiting vWF activity by use of either blocking antibody or dominant negative (Minamoto; Lankhof, 1996). The dominant negative genetic constructs encodes a mutated form of vWF that binds receptor but does not activate it (Lankhof, 1996). The inventors contemplate that the dominant negative genetic construct and the blocking antibody to von Willebrand factor will inhibit platelet activation by irradiated endothelial cells. The inventors will utilize blocking antibodies (Minamoto) and dominant negative genetic constructs (Lankhof, 1996) to von Willebrand factor added to endothelial cell cultures following irradiation. The inventors will also utilize blocking antibodies to the vWF receptor on platelets (Minamoto).

HUVEC cells will be grown to near confluence and irradiated. vWF blocking antibody will be added to cell cultures with rocking. In a separate study, the dominant negative genetic construct Platelets will be isolated as described (platelet isolation and bracket) and added to irradiated endothelial cell cultures.

Platelet activation will be determined by the following assays: platelet aggregates and free platelets will be counted on a hemocytometer and the ratio of aggregates to free platelets will be calculated, and platelet granule secretion

The inventors contemplate that blocking von Willebrand factor binding to its counterreceptor will attenuate platelet activation by the irradiated vascular endothelium. The inventors will utilize an *in vivo* model identified from the experiments described above in this example, which demonstrates von Willebrand factor translocation to the vascular lumen. The significance of this study is that von Willebrand Factor is associated with microangiopathy following total body irradiation for bone marrow transplantation (Ziegler, 1996).

von Willebrand factor activates platelets by binding to the GP-lb receptor. This receptor is mutated in the Bernard-Soulier syndrome (White, Blood, 1984). Following binding to GP-lb receptor, signal transduction is activated within platelets. Protein kinase A activates filamin which in turn activates Raf-kinase. Phosphoinositol 3-kinase then leads to Src-kinase activation (Clemetson, 1995). This signaling cascade can be blocked by recently described inhibitors, ticlopidine, and clopidogrel (Caen and Rosa, 1995). The inventors can therefore study the mechanisms of radiation-mediated platelet activation by use of these inhibitors of protein kinase A (ticlopidin, and propeditrol). The inventors will determine whether platelets are activated by quantifying the degree of platelet aggregation.

Vascular endothelial cells will be grown and irradiated as described in above in this example. Platelets will be added to irradiated endothelial cell cultures. At various times after coincubation, platelets will be removed from cell culture and the number of single and aggregated platelets will be quantified by hemocymoter and flow cytometry. In separate studies, the role of PKA will be studied by use of the PKA inhibitors ticlopidine, clopidodrel, which will be added to platelets prior to incubation with endothelial cells.

The inventors contemplate that irradiated endothelial cells with activate nonirradiated platelets through intercellular signal transduction. The classic signaling pathway that follows von Willebrand factor binding to GP-1, includes activation of PKA, raf kinase, and IP3-kinase.

Platelets have recently been shown to role along the vascular endothelium which is dependent upon P-selectin translocation to the luminal surface of blood vessels (Frenette, PNAS, 1995). The P-selectin knockout mouse show a 40% prolongation in bleeding time and attenuation of hemorrhagic necrosis resulting from the Shwartzman-like reaction (Subramaniam, 1996). The inventors contemplate that intercellular signaling from P-selectin contributes to platelet activation. The inventors will use of the P-selectin knockout mouse and P-selectin blocking antibodies injected prior to irradiation.

Wild-type C57BL6 mice and the P-selectin knockout mouse (C57BL6 derived) have been obtained from Jackson Laboratories and bred in the University of Chicago transgenic mouse core laboratory. Mice will be irradiated as described above in this example. Platelet aggregation within blood vessels will be studied by use of immunohistochemistry with platelet markers, using techniques that the inventors have previously shown (Hallahan and Virudachalam, 1997a).

P-selectin knockout eliminates P-selectin expression in all tissues including the endothelium and platelets. Therefore, this animal model is not specific for the intercellular signaling between platelets and leukocytes, but also affects leukocytes and endothelial interaction. P-selectin counterreceptor (PSGL-1) is present on monocytes and neutrophils. P-selectin is present on platelets and participates in platelet-leukocyte interactions (Furie, 1995 #89). Platelet activation may initiate leukocyte interaction with the irradiated vascular endothelium. P-selectin induces tissue factor production in monocytes (Celi *et al.,* 1994). The inventors will study the P-selectin blocking antibody in irradiated endothelial cells *in vitro.*

The inventors expect that knockout P-selectin gene or P-selectin blocking antibodies will attenuated platelet aggregation in irradiated blood vessels. The inventors will determine whether other platelet activating signals from the endothelium such as PAF and thrombin factor participate in radiation mediated platelet aggregation using the methods described above in this example.

Interleukin-8 is localized to Weibel Palade bodies within endothelial cells. This chemokine translocates to the luminal surface of endothelial cells following exocytosis. IL-8 then binds to receptors on monocytes and neutrophils to activate diapadesis and gene expression. The inventors contemplate that IL-8 translocation to the cell membrane in irradiated endothelial cells will contribute to intercellular signaling from irradiated endothelial cells to non-irradiated leukocytes. The inventors will utilize the IL-8 blocking antibody added to irradiated endothelial cells.

To determine whether Interleukin 8 is translocated to the cell membrane of vascular endothelium following irradiation, the inventors will utilize both *in vivo in vitro* models. Endothelial cells will be grown and irradiated as described above in this example. Immunofluorescence staining for IL-8 will be performed using techniques that the inventors have previously described (Hallahan, 1997a; Hallahan, 1997b). Interleukin 8 remains tethered to the cell membrane and acts as a chemokine for leukocytes. Immunofluorescence will verify whether IL-8 translocates to the cell membrane. In a separate study, blocking antibody will be added to endothelial cell cultures prior to the addition of untreated leukocytes as described above.

The inventors contemplate that IL-8 will participate in radiation mediated intercellular signal transduction. The inventors expect that blocking antibodies to IL-8 will attenuate activation of leukocytes by the irradiated vascular endothelium.

The IL-8 receptor is located on leukocyte cell membranes. During leukocyte rolling over the vascular endothelial surface, the IL-8 receptor contacts this tether chemokine, resulting in activation of leukocytes. To study this model of intercellular signaling, an IL-8 receptor knockout mouse has been developed (IL-8 RKO). The inventors contemplate that mice deficient in the IL-8 receptor will have diminished activation of circulating leukocytes within irradiated blood vessels. The IL-8 receptor knockout mice will be irradiated and leukocyte activation will be studied.

The IL-8 receptor knockout mouse will be bred in the inventors' transgenic core laboratory as the inventors have previously described (Hallahan and Virudachalam, 1997a). Tissues will be excised and fixed at various time intervals after irradiation (1, 3, 5, and 10 h after irradiation). The number of leukocytes adhering to the vascular endothelium will be quantified by staining for the leukocyte common antigen (LCA, CD45) and counting the number of inflammatory cells as the inventors have previously described (Hallahan and Virudachalam, 1997a). The inventors will then utilize immunohistochemistry and immunofluorescence to determine whether IL-8 mediated leukocyte activation is attenuated in the receptor knockout mouse.

The inventors contemplate that IL-8 translocation following irradiation of the vascular endothelium will contribute to the radiation response. In this regard, the inventors contemplate that knockout of the receptor for IL-8 will prevent radiation-mediated activation of leukocytes. Upon demonstration that the IL-8 receptor participates in intercellular signaling, the inventors will utilize IL-8 blocking antibodies to prevent radiation-mediated tissue injury.

IL-8 functions as a chemokine by binding to leukocyte receptors and activating diapedisis and enhancing adhesion. The IL-8 blocking antibody has been shown to attenuate inflammatory cell activation *in vivo* (Harada). IL-8 is a component of WP bodies and may participate in intercellular signal transduction following irradiation of the vascular endothelium. The inventors will use the IL-8 blocking antibody injected into mice immediately after irradiation.

Tissues will be excised and fixed at various time intervals after irradiation (1, 3, 5, and 10 h after, irradiation). The number of leukocytes adhering to the vascular endothelium will be quantified by staining for the leukocyte common antigen (LCA, CD45) and counting the number of inflammatory cells as the inventors have previously described (Hallahan and Virudachalam, 1997a). The inventors will then utilize immunohistochemistry and immunofluorescence to determine IL-8 mediated leukocyte activation is attenuation.

Previous studies have shown that it is feasible to use the IL-8 blocking antibody *in vivo* to attenuate inflammatory cell activation (Harada). The inventors expect that the IL-8 antibody will attenuate intercellular signaling from the irradiated endothelium to circulating leukocytes.

### REFERENCES

Abbassi *et al.,* "E-selectin supports neutrophil rolling *in vitro* under conditions of flow," *J. Clin.* Invest., 92(6):2719-30, 1993.

Ades *et al.,* "HMEC-1: establishment of an immortalized human microvascular endothelial cell line," *J. Invest. Dermatol.,* 99:683-690, 1992.

Alao *et al., J. Pharm. Pharmacol.,* 46(2) :135-7, 1994.

Allen and Choun, "Large unilamellar liposomes with low uptake into the reticuloendothelial system," *FEBS Lett.,* 223:42-46, 1987.

Alon *et al., J. Cell Biol.,* 127(5) :1485-95, 1994.

Amirkhosravi, "Platelets in the expression of monocyte tissue factor," *Thrombosis Haemostasis,* 75:87, 1996.

Barkalow, Goodman, Gerritsen, Mayadas, "Brain endothelium lack one of two pathways of P-selectin-mediated neutrophil adhesion," *Blood,* 88:4585, 1996.

Barker *et al.,* "Clinical experience of inflammatory breast carcinoma of the breast with or without chemotherapy," *Cancer,* 45:625-9, 1980.

Behrends *et al.,* "ICAM," *J. Invest. Dermat.*, 1994.

Berg *et al.,* "A carbohydrate domain common to both sialyl Le(a) and sialyl Le(X) is recognized by the endothelial cell leukocyte adhesion molecule ELAM-1," *J. Biol. Chem.,* 266(23):14869-72, 1991.

Betageri *et al., J. Pharm. Pharmacol.,* 45(1):48-53, 1993.

Bevilacqua *et al.,* "Endothelial leukocyte adhesion molecule 1: an inducible receptor for neutrophils related to complement regulatory proteins and lectins," *Science,* 243:1160-1165, 1989.

Bevilacqua, "Endothelial-leukocyte adhesion molecules," *Annu. Rev. Immunol.,* 11:767-804, 1993.

Bicknell, "Vascular targeting and the inhibition of angiogenesis," *Ann. Oncol.,* 4:45-50, 1994.

Bloom and Endow, "Motor proteins 1: kinesins," *Protein Profile,* 2:1105, 1995.

Bochner *et al., J. Immunol.,* 152(2):774-82, 1994.

Bode *et al., Science,* 229(4715):765-7, 1985.

Borgstrom, Hughes, Hansell, Wolitsky, Sriramarao, "Leukocyte adhesion in angiogenic blood vessels. Role of E-selectin, P-selectin, and beta2 integrin in lymphotoxin-mediated leukocyte recruitment in tumor microvessels," *J. Clin. Invest.,* 99(9):2246-2253, 1997.

Boukerche, "A monoclonal antibody directed against a granule membrane glycoprotein (GMP-140/PADGEM, P-selectin, CD62P) inhibits ristocetin-induced platelet aggregation," *Br. J. Haematol.,* 92:442, 1996.

Brach *et al.,* "Ionizing radiation stimulates N.F-kB binding activity in human myeloid leukemia cells," *J. Clin. Invest.,* 88:691-695, 1991.

Brady, "A novel ATPase with fast axonal transport," *Nature,* 317:73, 1985.

Braslawsky *et al., Cancer Res.,* 50(20):6608-14, 1990.

Brooks, "Cell adhesion molecules in angiogenesis," *Cancer and Metastasis Reviews,* 15(2):187-194, 1996.

Brunner *et al.,* "Xenograft model of progressive human proliferative breast disease," *J. Natl. Cancer Institute,* 85(21):1725-32, 1993.

Byers *et al., Cancer Res.,* 49:6153-6160, 1989.

Caen and Rosa, "Platelet-vessel wall interaction: from the bedside to molecules," *Thrombosis Haemostasis,* 74:18, 1995.

Campbell, *In: Monoclonal Antibody Technology, Laboratory Techniques in Biochemistry and Molecular Biology,* Vol. 13, Burden and Von Knippenberg (Eds.), pp 75-83, Amsterdam, Elseview, 1984.

Caplen *et al.,* "Liposome-mediated *CFTR* gene transfer to the nasal epithelium of patients with cystic fibrosis," *Nature Med.,* 1(1):39-46, 1995.

Casey *et al.,* "Growth suppression of human breast cancer cells by the introduction of a wild-type p53 gene," *Oncogene,* 6:1791-1797, 1991.

Cavenagh *et al.,* "Acute myeloid leukaemia blast cells bind to human endothelium *in vitro* utilizing E-selectin and vascular cell adhesion molecule-1 (VCAM-1)," *Br. J. Haematol.,* 85(2):285-91, 1993.

Celi, Pellegrini, Lorenze, De Blasi, Ready, Furie, Furie, "P-selectin induces expression of tissue factor on monocytes," *Proc. Natl. Acad. Sci. USA,* 91:8767, 1994.

Chapman *et al.,* "Noninvasive imaging of E-selectin expression by activated endothelium in urate crystal-induced arthritis," *Arthritis Rheum,* 37(12):1752-6, 1994.

Chomczynski and Sacchi, "Single-step method of RNA isolation by acid guanidinium thiocyanate-phenol-chloroform extraction," *Analytical Biochem.,* 162:156-159, 1987.

Clemetson, "Platelet activation: signal transduction via membrane receptors," *Thrombosis Haemostasis,* 74:111, 1995.

Cliff, "The acute inflammatory reaction in the rabbit ear chamber," *J. Exp. Med.,* 124:546-556, 1966.

Collen *et al., Circulation,* 82(5):1744-53, 1990.

Collins *et al.,* "Structure and chromosomal location of the gene for endothelial-leukocyte adhesion molecule 1," *J. Biol. Chem.,* 266:2466-2473, 1991.

Collins, "Endothelial nuclear factor-kappa B and the initiation of the atherosclerotic lesion," *Lab. Invest.,* 68:499-508, 1993.

Collins, "Adhesion molecules in leukocyte emigration," *Sci. Am. Sci. Med.,* 28-37, 1995.

Couvreur, "Polyalkyleyanoacrylates as colloidal drug carriers," *Crit. Rev. Ther. Drug Carrier Syst.,* 5:1-20, 1988.

Datta, Romano, Jacobson, Golan, Serhan, Ewenstein, "Peptido-leukotrienes are potent agonists of von Willebrand factor secretion and P-selectin surface expression in human umbilical vein endothelial cells," *Cir.,* 92:3304, 1995.

Davis and Morris, *Mol. Cell Endocrinol.,* 1:5-6, 1991.

De Boer *et al., Immunology,* 81(3):359-65, 1994.

Deshpande *et al.,* "Copper-67-labeled monoclonal antibody Lym-1, a potential radiopharmaceutical for cancer therapy: labeling and biodistribution in RAJI tumored mice," *J. Nuclear Med.,* 29(2):217-25, 1988.

DeSombre *et al.,* "Comparison of the distribution of bromine-77-bromovinyl steroidal and triphenylethylene estrogens in the immature rat," *J. Nuclear Med.,* 31(9):1534-42, 1990.

Dowell *et al.,* "Effects of a xanthine oxidase/hypoxanthine free radical and reactive oxygen species generating system on endothelial function in New Zealand white rabbit aortic rings," *J. Cardiovascular Pharmacol.,* 22:792-797, 1993.

Dunn *et al.,* "Effects of irradiation on endothelial cell-PMN leukocyte interaction," *J. Appl. Physiol.,* 60:1932-1937, 1986.

Eldor *et al.,* "Arachidonic metabolism and radiation toxicity in cultures of vascular endothelial cells," *Prostaglandis leukotrienes and essential fatty acids,* 36:251-258, 1989a.

Eldor *et al.,* "Perturbation of endothelial functions by ionizing irradiation: effects on prostaglandins, chemoattractants and mitogens," *Semin. Thromb. Hemost.,* 15:215-225, 1989b.

Epenetos *et al., Cancer Res.,* 46:3183-3191, 1986.

Etingin *et al.,* "Identification of a monocyte receptor on herpesvirus-infected endothelial cells," *Proc. Natl. Acad. Sci. USA,* 88:7200-7203, 1991.

Evangelista, Manarini, Rotondo, Martelli, Polischuk, McGregor, de Gaetano, Cerletti, "Platelet/polymorphonuclear leukocyte interaction in dynamic conditions: evidence of adhesion cascade and cross talk between P-selectin and the β 2 integrin CD11b/CD18," Blood, 88:4183, 1996.

Eyden, "Ultrastructural observations on Weibel-Palade bodies suggesting exocytosis," *J. Submicroscopic Cytol. Path.,* 25:145, 1993.

Eyden, *J. Submicro. Cytol. Path.,* 25:145, 1993.

Fajardo and Berthrong, "Vascular lesions following radiation," *Pathol. Annu.,* 23:297-330, 1988.

Fantone and Ward, "Polymorphonuclear leukocyte-mediated cell and tissue injury: oxygen metabolites and their relations to human disease," *Human Pathol.,* 16:973-978, 1985.

Fastenberg, *Am. J Clin. Oncol.,* 8:134, 1985.

Fernadez-Segura, Garcia, Campos, "Topographical distribution of CD 18 integrin on neutrophils as related to shape changes induced by chemotactic peptide," *Cell. Immunol.,* 171:120, 1996.

Finco and Baldwin, "Kappa B site-dependent induction of gene expression by diverse inducers of nuclear factor kappa B requires Raf-1," *J. Biol. Chem.,* 268:17676-17679, 1993.

Fletcher and Shukovsky, "The interplay of radiocurability and tolerance in the irradiation of human cancers," *J. Radiol. Electrol.,* 56:383-400, 1975.

Fliss and Menard, "Rapid neutrophil accumulation and protein oxidation in irradiatedrat lungs," *J. Applied Phys.,* 77:2727, 1994.

Folkman, "Clinical applications of research on angiogenesis," *New Engl. J. Med.,* 333:1757, 1995.

Fraker and Speck, "Protein and cell membrane iodination," *Biochem. Biophys. Res. Com.,* 80:849, 1989.

Franko, Sharplin, Ghahary, Barcellos-Hoff, "Immunohistochemical localization of TGF-B and TNF in the lungs after irradiation," *Rad. Res.,* 147:245-257, 1997.

Frenette, Johnson, Hynes, Wagner, "Platelets roll on stimulated endothelium *in vivo:* an interaction mediated by endothelial P-selectin," *Proc. Natl. Acad. Sci. USA,* 92:7450, 1995.

Fuks *et al.,* "Intravenous bFGF protects the lung against radiation-induced apoptosis *in vivo," Cancer J,* 1:62-72, 1995.

Fukuda, Takaichi, Naritomi, Hashimoto, Nagata, Nozaki, Kikuchi, *Brain Res.,* 696:30, 1995.

Furie and Furie, "The molecular basis of platelet and endothelial cell interaction with neutrophils and monocytes: role of P-selectin and the P-selectin ligand, PSGL-1," *Thrombosis Haemostasis,* 74:224, 1995.

Furie and Furie, *Haemostasis,* 26:60, 1996.

Gabizon and Papahadjopoulos, "Liposomes formulations with prolonged circulation time in blood and enhanced uptake by tumors," *Proc. Natl. Acad. Sci. USA,* 85:6949-6953, 1988.

Gefter *et al., Somatic Cell Genet.,* 3:231-236, 1977.

Ghersa *et al.,* "Labile proteins play a dual role in the control of endothelial leukocyte adhesion molecule-1 (ELAM-1) gene regulation," *J. Biol. Chem.,* 267(27):19226-32, 1992.

Glaser, "Cell Adhesion-Molecular Based Drugs Test the Waters in the Clinical Area," *Generig Engin. News,* 1994.

Goding, *In: Monoclonal Antibodies: Principles and Practice,* 2d ed., Academic Press, Orlando, FL, pp 60-61, 65-66, 71-74, 1986.

Goelz *et al., Cell,* 63:1349-1356, 1990.

Goelz *et al.,* "Differential expression of an E-selectin ligand (SLex) by two Chinese hamster ovary cell lines transfected with the same α(1,3)-fucosyltransferase gene (ELFT)," *J. Biol. Chem.,* 269(2):1033-40, 1994.

Goodson, Valetti, Kreis, "Motors and membrane traffic," *Curr. Opin. Cell. Biol.,* 9:18, 1997.

Gosset *et al.,* "Expression of E-selectin, ICAM-1 and VCAM-1 on bronchial biopsies from allergic and non-allergic asthmatic patients," *Int. Arch. Allergy Immunol.,* 106(1):69-77, 1995.

Graham, Evans, Dahlen, Mahler, Rasey, "Pharmacological alteration of the lung vascular response to radiation," *Intl. J. Rad. Oncol., Biol., Physics,* 19:329-339, 1990.

Green *et al.,* "High Affinity Binding of the Leucocyte Adhesion Molecule L-Selectin to 3'-Sulphated-Le and -Le^{x} Oligosaccharides and the Predominance of Sulphate in this Interaction Demonstrated by Binding Studies with a Series of Lipid-Linked Oligosaccharides," *Biochem. Biophys. Res. Commun.,* 188(1):244-251, 1992.

Griffioen, Damen, Blijham, Groenewegen, "Tumor angiogenesis is accompanied by a decreased inflammatory response of tumor-associated endothelium," *Blood,* 88(2):667-673, 1996.

Grinnell *et al., Glycobiology,* 4(2):221-5, 1994.

Groves *et al.,* "Endothelial leucocyte adhesion molecule-1 (ELAM-1) expression in cutaneous inflammation, *Br. J. Dermatol.,* 124(2):117-23, 1991.

Gustafson *et al.,* "Hydrogen peroxide stimulates phospholipase A2-mediated arachidonic acid release in cultured intestinal epithelial cells (INT 407)," *Scand. J. Gastroenterol,* 26:237-247, 1991.

Gutierrez *et al.,* "Gene therapy for cancer," *Lancet,* 339(8795):715-21, 1992.

Haagensen, *In: Diseases of the Breast,* 2nd ed., WB Saunders, Philadelphia, PA, p 623, 1971.

Hakkert *et al.,* "Neutrophil and monocyte adherence to and migration across monolayers of cytokine-activated endothelial cells: the contribution of CD18, ELAM-1, and VLA-4," *Blood,* 78(10):2721-6, 1991.

Hallahan and Virudachalam, "Intercellular adhesion molecule 1 knockout abrogates the inflammatory response to ionizing radiation," *Proc. Natl. Acad. Sci. USA,* 94:6432, 1996.

Hallahan and Virudachalam, "Ionizing radiation mediates expression of cell adhesion molecules in distinct patterns within the lung," *Cancer Res.,* 57:2096, 1997.

Hallahan, Spriggs, Beckett, Kufe, Weichselbaum, "Increased tumor necrosis factor alpha mRNA after cellular exposure to ionizing radiation," *Proc. Natl. Acad. Sci. USA,* 86:10104-10107, 1989.

Hallahan *et al.,* "Tumor necrosis factor gene expression is mediated by protein kinase C following activation by ionizing radiation," *Cancer Res.,* 51:4565-4569, 1991.

Hallahan *et al.,* "Radiation signalling mediated by Jun activation following dissociation from the Jun inhibitor," *J. Biol. Chem.,* 268:4903-7, 1992.

Hallahan *et al.,* "Membrane-derived second messengers mediate radiation induced TNF gene induction," *Proc. Natl. Acad. Sci.,* 91:4897-4901, 1993a.

Hallahan *et al., In: The Role of Cytokines in Radiation Oncology. Important Advances in Oncology,* DeVita and Rosenberg, (Eds.), Lippincott, Philadelphia, PA, 1993b.

Hallahan, Clark, Kuchibhotla, Gewertz, Collins, "E-selectin gene induction by ionizing radiation is independent of cytokine induction," *Biochem. Biophys. Res. Commun.,* 217:784-795, 1995a.

Hallahan, Mauceri, Seung, Dunphy, Toledano, Hellman, Kufe, Weichselbaum, "Spatial and temporal control of gene therapy by ionizing radiation," *Nature Med.,* 1:786, 1995b.

Hallahan, *Sem. Rad. Oncol.,* 6:250, 1996.

Hallahan, Kuchibholta, Wyble, "Cell adhesion molecules mediate radiation-induced leukocyte adhesion to the vascular endothelium," *Cancer Res.,* 56:5150-5155, 1996.

Hallahan, Kuchibhotla, Wyble, "Sialyl Lewis X mimetics attenuate E-selectin-mediated leukocyte adhesion to irradiated human endothelial cells," *Rad. Res.,* 147:41-47, 1997.

Haller, Kunzendorf, Sacherer, Lindschau, Walz, Distler, Luft, "T cell adhesion to P-selectin induces tyrosine phosphorylation of pp125 focal adhesion kinase and other substrates," *J. Immun.,* 158:1061, 1997.

Hamilton and Sims, "Changes in cytosolic Ca²⁺ associated with von Willebrand factor release in human endothelial cells exposed to histamine. Study of microcarrier cell monolayers using the fluorescent probe indo-1," *J. Clin. Invest.,* 79:600, 1987.

Harris *et al.,* "Gene therapy through signal transduction pathways and angiogenic growth factors as therapeutic targets in breast cancer," *Cancer,* 74:1021-5, 1994.

Hasegawa *et al., Carbohydrate Res.,* 257(1):67-80, 1994.

Henry-Michelland *et al.,* "Attachment of antibiotics to nanoparticles; Preparation, drug-release and antimicrobial activity *in vitro," Int. J. Pharm.,* 35:121-127, 1987.

Hollenbeck and Swanson, *Nature,* 346:864, 1990.

Hollstein *et al.,* "p53 mutations in human cancers," *Science,* 253:49-53, 1991.

Hopewell, Calvo, Jaenke, Reinhold, Robbins, Whitehouse, "Acute and long-term side effects of radiotherapy," *In: Microvasculature and Radiation Damage,* Hinkelbein (Ed.), Springer-Verlag, Berlin, 1993.

Howard, Hudspeth Vale, "Movement of microtubules by single kinesin molecules," *Nature,* 342:154, 1989.

Hsu-Lin, Berman, Furie, August, Furie, "A platelet membrane protein expressed during platelet activation and secretion. Studies using a monoclonal antibody specific for thrombin-activated platelets," *J. Biol. Chem.,* 259:9121, 1984.

Hsu-Lin, Berman, Furie, August, Furie, *J. Biol. Chem.,* 259:9121, 1984.

Hwu *et al.,* "Functional and molecular characterization of tumor-infiltrating lymphocytes transduced with tumor necrosis factor-alpha cDNA for the gene therapy of cancer in humans," *J. Immunol.,* 1993.

Jaenke *et al.,* "Capillary endothelium. Target site of renal radiation injury," *Lab. Invest.,* 68:396-405, 1993.

Jahroudi, Ardekani, Greenberger, "Ionizing irradiation increases transcription of the von Willebrand factor gene in endothelial cells," *Blood,* 88:3801, 1996.

Johnston, Cook, McEver, "Cloning of GMP-140, a granule membrane protein of platelets and endothelium: sequence similarity to proteins involved in cell adhesion and inflammation," *Cell,* 56:1033, 1989.

Jones and Shenk, "Isolation of Adenovirus Type 5 Host Range Deletion Mutants Defective for Transformation of Rat Embryo Cells," *Cell,* 17:683-689, 1979.

Jones *et al.,* "A two step adhesion cascade for T cell/endothelial cell interactions under flow conditions," *J. Clin. Invest.,* 94:2443-2450, 1995.

Jutila *et al.,* "Characterization of a functionally important and evolutionarily well-conserved epitope mapped to the short consensus repeats of E-selectin and L-selectin," *J. Exp. Med.,* 175(6): 1565-73, 1992.

Kamb *et al., Science,* 264:436-440, 1994.

Kameyama *et al.,* 1991.

Kancka *et al.,* "Preparation of bovine serum albumin conjugate and glycyrrhetic acid," *Chem. Pharm. Bull.,* 38:221-4, 1990.

Katagiri, Hayashi, Yamamoto, Tanoue, Kosaki, Yamazaki, "Localization of von Willebrand factor and thrombin-interactive domains on human plateletglycoprotein Ib," *Thrombosis Haemostasis,* 63:122, 1990.

Keelan *et al.,* "Characterization of E-selectin expression *in vivo* with use of a radiolabeled monoclonal antibody," *Am. J. Physiol.,* 266(1 Pt 2):H278-90, 1994a.

Keelan *et al.,* "Imaging vascular endothelial activation: an approach using radiolabeled monoclonal antibodies against the endothelial cell adhesion molecule E-selectin," *J. Nucl. Med.,* 35(2):276-81, 1994b.

Kharbanda *et al.,* "Activation of the src-like tyrosine kinase by ionizing radiation," *J. Biol. Chem.,* 269:20739-20743, 1994.

Kimura, Wu, Dewhirst, "Inhibition of radiation-induced upregulation of leukocyte adhesion to endothelial cells with the platelet activating factor inhibitor, BN52021," *Intl. J. Rad. Oncol., Biol., Physics,* 33:627, 1995.

Kishimoto *et al.,* "Antibodies against human neutrophil LECAM-1 (LAM-1/Leu-8/DREG-56 antigen) and endothelial cell ELAM-1 inhibit a common CD18-independent adhesion pathway *in vitro, " Blood,* 78(3):805-11, 1991.

Klein, "Yt-90 an I-131 radioimmunoglobin therapy of experimental hepatoma," *Cancer* Res., 49:6383, 1989.

Klibanov *et al., Am. J. Physiol.,* 261(4 Suppl):60-5, 1991.

Kohler and Milstein, *Nature,* 256:495-497, 1975.

Kohler and Milstein, *Eur. J. Immunol.,* 6:511-519, 1976.

Kojima *et al.,* "Multi-recognition capability of E-selectin in a dynamic flow system, as evidenced by differential effects of sialidases and anti-carbohydrate antibodies on selectin-mediated cell adhesion at low vs. high wall shear stress: a preliminary note," *Biochem. Biophys. Res. Commun.,* 189(3):1686-94, 1992.

Konno *et al.,* "Antitumor effect of adriamycin entrapped in liposomes conjugated with anti-human alpha-fetoprotein monoclonal antibody," *Cancer Res.,* 47(16):4471-7, 1987.

Koong *et al.,* "Hypoxia causes the activation of nuclear factor kappa B through the phosphorylation of I kappa B alpha on tyrosine residues," *Cancer Res.,* 54:1425-1430, 1994.

Krahenbuhl *et al., J. Clin. Endocrinol. Metab.,* 78(3):581-5, 1994.

Kumar *et al., J. Biol. Chem.* 266:21777-21783, 1991.

Kunich *et al., Molec. Cell. Biol.,* 12:4412, 1992.

Kushimoto, Okajima, Uchiba, Murakami, Okabe, Takatsuki, "Pulmonary vascular injury induced by hemorrhagic shock is mediated by P-selectin in rats," *Throm. Res.,* 82:97, 1996.

Labow, Norton, Rumberger, Lombard-Gillooly, Shuster, Hubbard, Bertko, Knaack, Terry, Harbison, "Characterization of E-selectin-deficient mice: demonstration of overlapping function of the endothelial selectins," *Immunity,* 1:709-720, 1994.

Lafont and Simons, "The role of microtubule-based motors in the exocytic transport of polarized cells," *Semin. Cell Develop. Biol.,* 7:343, 1996.

Larsson and Cerutti, "Translocation and enhancement of phosphotransferase activity of protein kinase C following exposure in mouse epidermal cells to oxidants," *Cancer Res.,* 49:5627-5632, 1989.

Lasek and Brady, *Nature,* 316:645, 1985.

Lawrence and Springer, "Leukocytes roll on a selectin at physiologic flow rates: distinction from and prerequisite for adhesion through integrins," *Cell,* 65:859-873, 1991.

Le Doussal *et al., Int. J Cancer Suppl.,* 7:58-62, 1992.

Lehr, Olofsson, Carew, Vajkoczy, von Andrian, Hubner, Berndt, Steinberg, Messmer, Arfors, "P-selectin mediates the interaction of circulating leukocytes with platelets and microvascular endothelium in response to oxidized lipoprotein *in vivo," Lab. Invest.,* 71(3):380-386, 1994.

Leichner *et al.,* "An overview of imaging techniques and physical aspects of treatment planning in radioimmunotherapy", *Med. Phys.,* 20(2 Pt 2):569-77, 1993.

Lenter *et al. J.* Cell. *Biol.,* 125(2):471-81, 1994.

Lessem *et al.,* "Accumulation of technetium-99m pyrophosphate in experimental infarctions in the rat," *Cardiovascular Res.,* 14(6):352-9, 1980.

Li and Sedivy, "Raf-1 protein kinase activates the NF-kappa B transcription factor by dissociating the cytoplasmic NF-kappa B-I kappa B complex," *Proc. Natl. Acad. Sci. USA,* 90:9247-9251, 1993.

Lo, Lee, Ramos, Lobb, Rosa, Chi-Rosso, Wright, "Endolthelial-leukocyte adhesion molecule 1 stimulates the adhesive activity of leukocyte integrin CR3 (CD 11b/CD18, Mac-1, alpha m beta 2) on human neutrophils," *J. Exper. Med.,* 173:1493, 1991.

Lo, Bevilacqua, Malik, "E-selectin ligands mediate TNF-induced neutrophil sequestration and pulmonary edema in guinea pig lung," *Circ. Res.,* 75:955-960, 1994.

Lorant, Patel, McIntyre, McEver, Prescott, Zimmerman, "Coexpression of GMP-140 and PAF by endothelium stimulated by histamine or thrombin: a juxtacrine system for adhesion and activation of neutrophils," *J. Cell Bio.,* 115:223, 1991.

Lorant, Patel, McIntyre, McEver, Prescott, Zimmerman, *J. Cell Biol.,* 115:223, 1991.

Lowe *et al., Cell.* 63, 475-484, 1990.

Lowe *et al., J. Biol. Chem.* 266, 21777-21783, 1991.

Luders *et al., Cancer Immunol., Immunother.,* 20(1):85-90, 1985.

Luscinskas, Cybulsky, Kiely, Peckins, Davis, Gimbrone, Jr., "Cytokine-activated human endothelial monolayers support enhanced neutrophil transmigration via a mechanism involving both endothelial-leukocyte adhesion molecule-1 and intercellular adhesion molecule-1," *J. Immunol.,* 146:1617-1625, 1991.

Malik and Lo, "Vascular endothelial adhesion molecules and tissue inflammation," *Pharm. Rev.,* 48(2):213-229, 1996.

Malik, "The activity of TNF in experimental animal models. *In: Tumor Necrosis Factor,"* Beutler (Ed), New York, NY, 1992.

Marx, *Science,* 263:319-321, 1994.

Matthay *et al., Cancer Res.,* 49(17):4879-86, 1989.

Matzner *et al.,* "Generation of lipid neutraphil chemoattractants by irradiated BAEC," *J Immunol.,* 140:2681-2685, 1988.

Mauceri, Hanna, Wayne, Hallahan, Hellman, Weichselbaum, "Tumor necrosis factor gene therapy targeted by ionizing radiation selectively damages tumor vasculature," *Cancer Res.,* 56:4311, 1996.

Mayadas, Johnson, Rayburn, Hynes, Wagner, "Leukocyte rolling and extravasation are severely compromised in P selectin-deficient mice," *Cell,* 74:541, 1993.

Mazzone and Ricevuti, *Haematologica,* 80:161, 1995.

McGrory *et al., Virology,* 163:614-7, 1988.

Mehta *et al. Intl J. Rad. Oncol., Biol., Physics,* 19(3):627-31, 1990.

Meyer *et al.,* "H202 and antioxidants have opposite effects on activation of NF-kappa B and AP-1 in intact cells: AP-1 as secondary antioxidant-responsive factor," *Embo. J.,* 12:2005-2015, 1993.

Miller *et al.,* "Selection of a highly tumorigenic breast cancer cell line sensitive to estradiol to *evidence in vivo* the tumor-inhibitory effect of butyrate derivative Monobut-3," *Life Sciences,* 55(12):951-9, 1994.

Miyagami and Nakamura, "Tubular bodies (Weibel-Palade bodies) in endothelial cells of glioblastomas and astrocytomas," *Noshuyo Byori,* 13:107, 1996.

Montefort *et al.,* "Intercellular adhesion molecule-1 (ICAM-1) and endothelial leucocyte adhesion molecule-1 (ELAM-1) expression in the bronchial mucosa of normal and asthmatic subjects," *Eur. Respir. J.,* 5(7):815-23, 1992.

Montgomery *et al.,* "Activation of endothelial-leukocyte adhesion molecule 1 (ELAM-1) gene transcription," *Proc. Natl. Acad. Sci. USA,* 88:6523-6527, 1991.

Moughal *et al.,* "Endothelial cell leukocyte adhesion molecule-1 (ELAM-1) and intercellular adhesion molecule-1 (ICAM-1) expression in gingival tissue during health and experimentally- induced gingivitis," *J. Periodontal Res.,* 27(6):623-30, 1992.

Mulligan *et al.,* "Role of Endothelial Leukocyte Adhesion Molecule 1 (ELAM-1) in Neutrophil-mediated Lung Injury in Rats," *J. Clin. Invest.,* 88:1396-1406, 1991.

Mulligan, Polley, Bayer, Nunn, Paulson, Ward, *J. Clin. Invest.,* 90:1600, 1992.

Mulligan *et al.,* "Protective Effects of Sialylated Oligosaccharides in Immune Complex-induced Acute Lung Injury," *J. Exp. Med.,* 178:623-631, 1993a.

Mulligan, Paulson, De Frees, Zheng, Lowe, Ward, *Nature,* 364:149, 1993b.

Munro *et al.,* "Expression of sialyl-Lewis X, an E-selectin ligand, in inflammation, immune processes, and lymphoid tissues," *Am. J. Pathol.,* 141(6):1397-408, 1992.

Nabel, "Gene transfer and vascular disease" *Cardiovascular Res.,* 28(4):445-55, 1994.

Narasinga Rao *et al.,* "Sialyl Lewis X mimics derived from a Pharmacophore search are selectin inhibitors with anti-inflammatory activity," *J. Biol. Chem.,* 269:19663-19666; 1994.

Narayan and Cliff, "Morphology of irradiated microvasculature," *Am. J Pathol.,* 106:47, 1982.

Navone, Niclas, Hom-Booher, Sparks, Bernstein, McCaffrey, Vale, *J. Cell. Biol.,* 117:1263, 1992.

Nelson *et al.,* "Higher-affinity oligosaccharide ligands for E-selectin," *J. Clin. Invest.,* 91:1157-1166, 1993.

Neumann *et al.,* "Immunohistochemistry of port-wine stains and normal skin with endothelium-specific antibodies PAL-E, anti-I-CAM-1, anti-ELAM-1, and anti-factor VIIirAg," *Arch. Dermatol.,* 130(7):879-83, 1994.

Norton *et al.,* "Characterization of murine E-selectin expression *in vitro* using novel anti-mouse E-selectin monoclonal antibodies," *Biochem. Biophys. Res. Commun.,* 195(1):250-8, 1993.

Numazaki *et al., Tohoku J. Exper. Med.,* 172(2):147-53, 1994.

O'Brien-Ladner, Nelson, Kimler, Wesselius, "Release of interleukin-1 by human alveolar macrophages after *in vitro* irradiation," *Rad. Res.,* 136:37-41, 1993.

Ohno *et al.,* "Gene therapy for vascular smooth muscle cell proliferation after arterial injury," *Science,* 265(5173):781-4, 1994.

Okuno *et al., Nippon Rinsho - Japanese J. Clin. Med.,* 52(7):1823-1827, 1994.

Olofsson *et al.,* "E-selectin mediates leukocyte rolling in interleukin-1-treated rabbit mesentery venules," *Blood,* 84(8):2749-58, 1994.

Pai *et al.,* "Antitumor effects of B3-PE and B3-LysPE40 in a nude mouse model of human breast cancer and the evaluation of B3-PE toxicity in monkeys," *Cancer Res.,* 52(11):3189-93, 1992.

Palabrica, Lobb, Furie, Aronovitz, Benjamin, Hsu, Sajer, Furie, "Leukocyte accumulation promoting fibrin deposition is mediated *in vivo* by P-selectin on adherent platelets," *Nature,* 359(6398):848-51, 1992.

Panes, Anderson, Miyasaka, Granger, "Role of leukocyte-endothelial cell adhesion in radiation-induced microvascular dysfunction in rats," *Gastroenterology,* 108:1761, 1995.

Patel *et al.,* "Oxygen radicals induce human endothelial cells to express GMP-140 and bind neutrophils," *J. Cell Biol.,* 112:749-759, 1991.

Patey, Vazeux, Canioni, Potter, Gallatin, Brousse, "Intercellular adhesion molecule-3 on endothelial cells. Expression in tumors but not in inflammatory responses," *Am. J. Path.,* 148(2):465-472, 1997.

Phillips *et al.,* "ELAM-1 Mediates Cell Adhesion by Recognition of a Carboydrate Ligan, Sialyl-Lex," *Science,* 250:1130-1132, 1990.

Piguet, Vesin, Thomas, "Bombesin down modulates pulmonary fibrosis elicited in mice by bleomycin," *Exper. Lung Res.,* 21:227, 1995.

Pinola *et al., J. Immunol.,* 152 (2) :774-82, 1994.

Pinsky, Naka, Liao, Oz, Wagner, Mayadas, Johnson, Hynes, Heath, Lawson, Stern, "Hypoxia-induced exocytosis of endothelial cell Weibel-Palade bodies. A mechanism for rapid neutrophil recruitment after cardiac preservation," *J. Clin. Invest.,* 97:493, 1996.

Pober and Cotran, "Cytokines and endothelial cell biology," *Physiol. Rev.,* 70:427, 1990.

Porras *et al.,* "Dissociation," *J. Biol. Chem.,* 269:12741, 1994.

Pouyani, and Seed, *Cell,* 83:333, 1995.

Prasad *et al.,* "Activation of Nuclear Factor κB in Human Lymphoblastoid Cells by Low-Dose Ionizing Radiation," *Rad. Res.,* 138:367-372, 1994.

Read *et al.,* "NFκB. An inducible regulatory system in Endothelial Cells," *J. Exp. Med.,* 179:503, 1994.

Reinhold *et al.,* "The vascular system," *Adv. Rad. Biol.,* 14:177-226, 1990.

Roeske *et al.,* "Modeling of dose to tumor and normal tissue from intraperitoneal radioimmunotherapy with alpha and beta emitters," *Int. J Rad. Oncol. Biol. Phys.,* 19:1539-48,1990.

Romberg and Vale, "Chemomechanical cycle of kinesin," *Nature,* 361:168, 1993.

Rot, *Immun. Today,* 13:291, 1992.

Rothwell, Deal, Pinto, Wright, *J. Leuk. Biol.,* 53:372, 1993.

Saluson *et al.,* "Inflammatory sequences in acute pulmonary radiation injury," 82:529-572, 1976.

Sands, *Immunoconjugates and Radiopharmaceuticals,* 1:213-226, 1988.

Schreck *et al.,* "Reactive oxygen intermediates as apparently widely used messengers in the activation of the NF-κB transcription factor and HIV-1," *The EMBO Journal,* 10(8):2247-2258, 1991.

Schreck *et al.,* "Nuclear factor kappa B: an oxidative stress-responsive transcription factor of eukaryotic cells," *Free Radic. Res. Commun.,* 17:221-237, 1992.

Schreiber *et al., Nucl. Acids Res.,* 17:6419, 1989.

Scott *et al., J. Natl. Cancer Instit.,* 79(5) :1163-72, 1987.

Sedmak *et al.,* "Divergent patterns of ELAM-1, ICAM-1, and VCAM-1 expression on cytomegalovirus-infected endothelial cells," *Transplantation,* 58(12):1379-85, 1994.

Senaldi and Piguet, "Platelets play a role in the pathogenesis of the irritant reaction," *J. Invest. Derm.,* 108:248, 1997.

Shwartzman, "A new phenomenon of local skin reactivity to B typhosus culture filtrate," *Proc. Soc. Exper. Bio. Med.,* 25:560, 1997.

Silber *et al.,* "Recruitment of lymphocytes during cutaneous delayed hypersensitivity in nonhuman primates is dependent on E-selectin and vascular cell adhesion molecule 1," *J. Clin. Invest.,* 93(4):1554-63, 1994.

Sinha and Wagner, "Intact microtubules are necessary for complete processing, storage and regulated secretion of von Willebrand factor by endothelial cells," *Eur. J. Cell. Biol.,* 43:377, 1987.

Slauson *et al.,* "Inflammatory sequences in acute pulmonary radiation injury," *Am. J. Path.,* 82:549-572, 1976.

Sligh, Ballantyne, Rich, Hawkins, Smith, Bradley, "Inflammatory and immune responses are impaired in mice deficient in intercellular adhesion molecule 1," *Proc. Natl. Acad. Sci. USA,* 90:8529-8533, 1993.

Smith, Rothlein, Hughes, Mariscalco, Rudloff, Schmalstieg, Anderson, "Recognition of an endothelial determinant for CD18-dependent neutrophil adherence and transendothelial migration," *J. Clin. Invest.,* 82:1746, 1988.

Soma *et al., Endocrine Regulations,* 28 (1):31-34, 1994.

Span *et al.,* "Cytomegalovirus induced PMN adherence in relation to an ELAM-1 antigen present on infected endothelial cell monolayers," *Immunology,* 72(3):355-60, 1991.

Sporn *et al.,* "E-selectin-dependent neutrophil adhesion to Rickettsia rickettsii-infected endothelial cells," *Blood,* 81(9):2406-12, 1993.

Springer, *Nature,* 346:425, 1990.

Springer, "Traffic signals for lymphocyte recirculation and leukocyte emigration: the multistep paradigm," *Cell,* 76:301-314, 1994.

Steinberg *et al.,* "Survival in lung reperfusion injury is improved by an antibody that binds and inhibits L- and E-selectin, "*J*. *Heart Lung Transplant,* 13(2):306-18, 1994.

Steinberg, Quabeck, Rehn, Kraus, Mohnke, Costabel, Kreuzfelder, Molls, Bruch, Schaefer, *et al.,* "Lung effects after total body irradiation of mice and bone marrow transplant patients: comparison of experimental and preliminary clinical data," *Recent Results Cancer Res.* 130:133-143, 1993.

Steinberg, Quabeck, Rehn, Kraus, Mohnke, Costabel, Kreuzfelder, Molls, Bruch, Schaefer *et al., In: Lung effects after TBI of mice and bone marrow transplant patients: comparison of experimental and clinical data,* Springer-Verlag, Berlin, 1993.

Subramaniam, Frenette, Saffaripour, Johnson, Hynes, Wagner, "Defects in hemostasis in P-selectin-deficient mice," *Blood,* 87:1238, 1996.

Sugama and Malik, *Circ. Res.,* 71:1015,1992.

Suzuki, Yamazaki, Tanoue, "Immunocytochemical aspects of platelet membrane glycoproteins and adhesive proteins during activation," *Progress Histochem. Cytochem.,* 30:1, 1996.

Swerlick and Lawley, "Role of microvascular endothelial cells in inflammation," *HDMEC Inflammation,* 100(1):111S-1115S, 1993.

Terada *et al., Carbohydrate Res.,* 259(2):210-18, 1994.

Travis, "The sequence of histological changes in mouse lungs after single doses of x-rays," *Intl. J. Rad. Oncol., Biol., Physics,* 6:345-347, 1980.

Treisman *et al.,* "Upregulation of tumor necrosis factor-alpha production by retrovirally transduced human tumor-infiltrating lymphocytes using trans-retinoic acid," *Cell. Immunol.,* 156(2):448-57, 1994.

Trubetskoy *et al., Biochim. Biophys. Acta,* 1131(3):311-3, 1992.

Tsai *et al., J. Pharmaceutical Sci.,* 81(9):961-3, 1992.

Tsuji, Nagata, Koike, Todoroki, Irimura, "Induction of superoxide anion production from monocytes an neutrophils by activated platelets through the P-selectin-sialyl Lewis X interaction," *J. Leuk. Biol.,* 56:583, 1994.

Tyrrell *et al.,* "Structural requirements for the carbohydrate ligand of E-selectin," *Proc. Natl. Acad. Sci. USA,* 88:10372-10376, 1991.

Uckun *et al.,* "Ionizing radiation stimulates tyrosine-specific protein kinases triggering apoptosis," *Proc. Natl. Acad. Sci. USA,* 89:9005-9009, 1992.

Ulich *et al.,* "Intratracheal administration of endotoxin and cytokines: VIII. LPS induces E-selectin expression; anti-E-selectin and soluble E-selectin inhibit acute inflammation," *Inflammation,* 18(4):389-98, 1994.

Vale, Reese, Sheetz, "Identification of a novel force-generating protein, kinesin, involved in microtubule-based motility," *Cell,* 42:39, 1985.

Veale *et al.,* "Reduced synovial membrane macrophage numbers, ELAM-1 expression, and lining layer hyperplasia in psoriatic arthritis as compared with rheumatoid arthritis," *Arthritis Rheum,* 36(7):893-900, 1993.

Verheij, Dewit, Boomgaard, Brinkman, van Mourik, "Ionizing radiation enhances platelet adhesion to the extracellular matrix of human endothelial cells by an increase in the release of von Willebrand x factor," *Rad. Res.,* 137:202, 1994.

Vischer and Wagner, *Blood,* 82:1184, 1993.

von *Asmuth et al.,* "Evidence for endocytosis of E-selectin in human endothelial cells," *Eur. J. Immunol.,* 22(10):2519-26, 1992.

Wakita *et al.,* "E-selectin and vascular cell adhesion molecule-1 as critical adhesion molecules for infiltration of T lymphocytes and eosinophils in atopic dermatitis," *J. Cutan. Pathol.,* 21(1):33-9, 1994.

Walker, "Inducible DNA repair systems," *Ann. Rev. Biochem.,* 54:425-457, 1985.

Walz *et al.,* "Recognition by ELAM-1 of the Sialyl-Le^{x} Determinant on Myeloid and Tumor Cells," *Science,* 250:1132-1135, 1990

Ward, "DNA damage produced by ionizing radiation in cells: mechanisms of formation and repairability," *Prog. Nucl. Acid Res.,* 3:95, 1988.

Ward, Kemsley, Davies, Holecek, Berend, "The pulmonary response to sublethal thoracic irradiation in the rat," *Rad. Res.,* 136:15-21, 1993.

Weibel and Palade, "New cytoplasmic components in arterial endothelia," *J. Cell Biol.,* 23:101, 1964.

Weichselbaum *et al.,* "Radiation targeting of gene therapy preferentially radiosensitizes tumor cells," *Cancer Res.,* 54:4266-4269, 1994.

Weinberg, "Tumor suppressor gene," *Science,* 254: 1138-1145, 1991.

Weiner *et al., Cancer Res.,* 49:4062-4067, 1989.

Weyrich, McIntyre, McEver, Prescott, Zimmerman, "Monocyte tethering by P-selectin regulates monocyte chemotactic protein-1 and tumor necrosis factor-alpha secretion. Signal integration and NF-kappa B translocation," *J. Clin. Invest.,* 95:2297, 1995.

Whelan *et al.,* "An NF kappa B-like factor is essential but not sufficient for cytokine induction of endothelial leukocyte adhesion molecule 1 (ELAM-1) gene transcription," *Nucl. Acids Res.,* 19:2645-2653, 1991.

Wicki and Clemetson, "Structure and function of platelet membrane glycoproteins Ib and V. Effects of leukocyte elastase and other proteases on platelets response to von Willebrand factor and thrombin," *Eur. J. Biochem.,* 153:1, 1985.

Willard, "Genetic modification of the vessel wall. Comparison of surgical and x catheter-based techniques for delivery of recombinant adenovirus," *Circ.,* 89(5):2190-7, 1994.

Williams and Roberts, "Signal transduction pathways involving the Raf proto-oncogene," *Cancer Metastasis Rev.,* 13:105-116, 1994.

Williams *et al.,* "Targeting of human glioma xenografts utilizing radiolabeled antibodies," *Intl. J. Rad. Oncol. Biol., Phys.,* 18:1367-1375, 1990.

Wu, Ross, Gulledge, Klitzman, Dodge, Dewhirst, "Differences in leucocyte-endothelium interactions between normal and adenocarcinoma bearing tissues in response to radiation," *Br. J. Cancer,* 68:883, 1994.

Yoshida *et al., Glycoconjugate J.,* 10(1):3-15, 1993.

Yuen *et al.,* "Novel Sulfated Ligands for the Cell Adhesion Molecule E-Selectin Revealed by the Neoglycolipid Technology Among O-Linked Oligosaccharides on an Ovarian Cystadenoma Glycoprotein, *Biochemistry,* 31:9126-9131, 1992.

Yuen *et al.,* "A Superior Oligosaccharide Ligand for Human E-Selectin," *J. Biol. Chem.,* 269(3):1595-1598, 1994.

Zeigler, Rosenfeld, Andrews, Nemunaitis, Raymond, Shadduck, Kramer, Gryn, Rintels, Besa, George, "Plasma von Willebrand Factor Antigen (vWF:AG) and thrombomodulin (TM) levels in Adult Thrombotic Thrombocytopenic Purpura/Hemolytic Uremic Syndromes (TTP/HUS) and bone marrow transplant-associated thrombotic microangiopathy (BMT-TM)," *Am. J. Hematol.,* 53(4):213-220, 1996.

Zhang *et al.,* "A secreted mucin carrying sialyl-Lewis a from colon carcinoma cells binds to E-selectin and inhibits HL-60 cell adhesion," *Int. J. Cancer,* 59(6):823-9, 1994.

Ziegler *et al.,* "Pyrrolidine dithiocarbamate inhibits NF-kappa B mobilization and TNF production in human monocytes," *J. Immunol.,* 151:6986-6993, 1993.

## Claims

1. Use of a pharmaceutically acceptable composition comprising a P-selectin binding component, for the manufacture of a medicament for preventing or treating radiation damage in an animal patient exposed to radiation by administering the composition to said patient.

2. The use of Claim 1, wherein the preventing or treating further comprises administering to said patient a selected agent.

3. The use of Claim 1 or 2 wherein said animal patient is a human.

4. The use of any one of the preceding claims, wherein said pharmaceutically acceptable composition may be administered to said animal patient in a topical, an oral or a parenteral formulation.

5. The use of any one of the preceding claims, wherein the preventing or treating further comprises reducing or blocking the inflammatory response.

6. The use of any one of the preceding claims, wherein said P-selectin binding component inhibits the interaction of P-selectin with an inflammatory cell receptor, preferably a leukocyte receptor.

7. The use of any one of the preceding claims wherein said radiation is from radiotherapy.

8. The use of any one of the preceding claims, wherein said administration occurs from 15 minutes to 2 hours after exposure to radiation.

9. The use of any one of Claims 1 to 7 wherein said administration occurs within 30 minutes of exposure to radiation, preferably within 15 minutes of exposure to radiation.

10. The use of any one of the preceding claims, wherein said P-selectin binding component is an antibody or a fragment thereof, an oligosaccharide, a polysaccharide, a glycolipid, a glycoprotein, a cell or a cell ligand.

11. The use of Claim 10, wherein said P-selectin binding component is an antibody, preferably a monoclonal antibody.

12. The use of Claim 10, wherein said P-selectin binding component is a fragment of an antibody, preferably a scFv, a Fv, a Fab', a Fab, or a F(ab')₂.

13. The use of Claim 10, wherein said P-selectin binding component is an oligosaccharide, preferably a PGSL, a glycyrrhizin, a carminic acid, a cylexin, a sialyl Lewis X/A oligosaccharide, a sialyl Lewis X/A mimic or a structural analogue thereof.

14. The use of Claim 13, wherein said sialyl Lewis X/A oligosaccharide is a sialyl Lewis X, a sialyl Lewis A, a sialyl Lewis X/A, a sialyl Lewis pentasaccharide, a sialyl Lewis tetrasaccharide, a sulfated Le pentasaccharide, a sulfated Le tetrasaccharide, an amino substituted sLe^{a} or a dimeric sialyl Lewis compound, preferably a sialyl Lewis^{x}, a sLe^{x} a Neu5Acα2-3Galβ1-4 [Fucα1-3]GlcNAc, a sialyl Lewis^{a}, a sLe^{a}, or a Neu5Acα2-3Galβ1-3 [Fucα1-4]GlcNAc.

15. The use of Claim 1, wherein said polysaccharide is a polylactosamine.

16. The use of Claim 10, wherein said glycoprotein is a Protein C, a 150 kD, a 230 kD or a 130 kD, as measured by polyacrylamide gel electrophoresis.

17. The use of any one of Claims 10 to 16, wherein said cell is a T lymphocyte, a T leukocyte, a PMN, an eosinophil or a NK cell.

18. The use of any one of Claims 10 to 17, wherein said oligosaccharide, polysaccharide or glycolipid are formulated into a liposome preparation, preferably a cationic liposome, a DOTMA/DOPE, a DOTMA or a DORIE.

19. The use of any one of Claims 10 to 17, wherein said oligosaccharide, polysaccharide or glycolipid are formulated into a protein or a polypeptide carrier.

20. The use of Claim 10, wherein said cell ligand is isolated from a leukocyte, a T cell or from a polymorphonuclear neutrophil.

21. Use of a composition comprising a P-selectin binding component operatively associated with or attached to a selected agent, for the manufacture of a medicament for use in a method for delivering a selected agent to the vasculature of an animal subject comprising inducing P-selectin translocation to the lumen of a vascular endothelial cell and administering the composition to said animal subject.

22. The use of Claim 21, wherein said administration occurs from 15 minutes to 2 hours after said inducing P-selectin translocation.

23. The use of Claim 21, wherein said administrating occurs within 30 minutes of said inducing P-selectin translocation.

24. The use of any one of the preceding claims wherein said administrating comprises a single dose.

25. The use of any one of Claims 1 to 23, wherein said administrating comprises a multiple dose.

26. The use of any one of Claims 1 to 23, wherein said administrating comprises continuous administration.

27. Use of a composition comprising a P-selectin binding compound operatively associated with a selected agent, for the manufacture of a medicament for use in a method for delivering a selected agent to the disease-associated vasculature of an animal subject with a disease that has a vascular component, comprising inducing translocation of P-selectin to the lumen of disease-associated vascular endothelial cells and administering the composition to said animal subject.

28. The use of any one of Claims 21 to 27, wherein said animal subject is a human.

29. The use of Claim 27 or Claim 28 as dependent on Claim 27, wherein said disease with a vascular component is a solid tumor, a diabetic retinopathy, a vascular restenosis, an arteriovenous malformation (AVM), a meningioma or a vascular component-associated benign disease.

30. The use of any one of Claims 21 to 29, wherein said P-selectin translocation to said lumen of disease-associated vascular endothelial cells is induced by: an ionizing radiation, preferably a γ-irradiation or an x-ray; a heat; or an oxidant, preferably a H₂O₂ or an O₂.

31. The use of Claim 27 or any claim dependent thereon, further comprising administration to said animal subject in a pharmaceutically acceptable formulation a composition of a second agent operatively associated with a selectin binding agent.

32. The use of 31, wherein said administration is a parental administration, an injection or an insulation into a disease site or a vascularized tumor site.

33. The use of Claim 27 or any claim dependent thereof, wherein said P-selectin binding compound is an antibody or a fragment thereof, an oligosaccharide, a glycolipid, a glycoprotein or a cell.

34. The use of Claim 27, or any claim dependent thereon further comprising obtaining a tumor-infiltrating lymphocyte from said animal subject, treating said tumor-infiltrating lymphocyte with a second agent, and reintroducing said tumor-infiltrating lymphocyte.

35. The use of Claim 31 or 32, wherein said second agent is a recombinant vector that is inserted into a TIL.

36. The use of Claim 31 or 32, wherein said second agent is a thrombolytic agent, preferably a streptokinase or an urokinase.

37. The use of Claim 31 or 32, wherein said second agent is a chemotherapeutic agent, preferably a steroid, an anthracycline, a vinca alkaloid, an antibiotic, an alkylating agent, an epipodophyllotoxin, a neocarzinostatin (NCS), an adriamycin or a dideoxycytidine.

38. The use of Claim 31 or 32, wherein said second agent is a therapeutic polypeptide, preferably an interferon-α (IFN-α), an interferon-βγ (IFN-βγ) an interleukin-12 (IL-12) or a tumor necrosis factor -α (TFN-α).

39. The use of Claim 31 or 32, wherein said second agent is a toxin, preferably a plant-, a fungus- or a bacteria derived toxin.

40. The use of Claim 39, wherein said toxin is a gelonin, a α-sarvin, an aspergillin, a restrictocin, a ribonucleases, a diphtheria toxin, a *Pseudomonas* exotoxin, a bacterial endotoxin, a lipid A, a ricin A chain, a deglycosylated ricin A chain or a recombinant recin A chain.

41. The use of Claim 31 or 32, wherein said second agent is a fluorogenic, a paramagnetic or a radioative ion.

42. The use oif Claim 41, wherein the paramagnetic ion is chromium (III), manganese (II), iron (III), iron (II), cobalt (II), nickel (II), copper (II), neodymium (III), samarium (III), ytterbium (III), gadolinium (III), vanadium (II), terbium (III), dysprosium (III), holmium (III) or erbium (III).

43. The use of Claim 41 wherein the radioactive ion is iodine¹²³, technicium^{99m}, indium¹¹¹, rhenium¹⁸⁸, rhenium¹⁸⁶, copper⁶⁷, iodine¹³¹, yttrium⁹⁰, iodine¹²⁵, astatine²¹¹, gallium⁶⁷, iridium¹⁹², cobalt⁶⁰, radium²²⁶, gold¹⁹⁸, cesium¹³⁷, or phosphorus³².

44. The use of Claim 41, wherein the fluorogenic agents is gadolinium or renographin.

45. The use of Claim 31 or 32, wherein said second agent is a recombinant vector comprising a promoter operatively linked to a polynucleotide encoding a therapeutic polynucleotide or polypeptide.

46. The use of Claim 45, wherein said promoter is an ionizing radiation-inducible promoter, preferably an *Egr-1* promoter, a *los* promoter, a c-jun promoter or a TNF-α promoter.

47. The use of Claim 45, wherein the promoter is a vascular endothelial cell specific promoter, preferably an *Egr-1* gene promoter, an ICAM-1 gene promoter of an E-selectin gene promoter.

48. The use of any one of Claims 45 to 47 wherein said therapeutic polypeptide is a p53, a p16, a Rb, a WT1, an IFN-α- an IFN-βγ, an IL-12, a TNF-α, a thymidine kinase (tk) or a cytosine deaminase.

49. The use of any one of Claims 45 to 48, wherein the recombinant vector is a retrovirus, an AAV, a HSV-1, a HPV, a vaccinia, an adeno-associated virus or an adenovirus.

50. The use of any one of Claims 45 to 49, wherein the disease is a benign hyperproliferative disease.

51. The use of any one of Claims 45 to 49, wherein the disease is a malignancy.

52. The use of any one of Claims 45 to 51, further comprising providing an additional dose of ionizing radiation after delivery of said selected agent.

53. The use of Claim 52, as dependent on Claim 45, wherein said selected agent is a recombinant vector operatively associated with a P-selectin targeting component, wherein said vector comprises an ionizing radiation-inducible promoter operatively linked to a polynucleotide encoding a therapeutic polynucleotide or polypeptide.

54. A use of a composition comprising a P-selectin binding component operatively associated with a detectable marker for the preparation of a reactant for use in a method for determing the radiation exposure of an animal or patient comprising administering an effective amount of the composition to said animal or patient; exposing the animal or patient to a device that detects said detectable marker and determining the level of P-selectin translocation of the lumen of vascular endothelial cells of an irradiated site of the animal or patient, wherein an increase in the P-selectin level, as compared to the level of P-selectin at said site in a non-irradiated animal or patient, is indicative of radiation exposure.

55. The use of Claim 54, wherein said P-selectin targeting component is a glycyrrhizin, a carminic acid, a cylexin, a sialyl Lewis X, a sialyl Lewis A, a sialyl Lewis X/A, a sialyl Lewis X/A mimics or an antibody that binds immunologically to P-selectin.

56. The use of Claim 55, wherein said detectable marker is a nuclear magnetic spin-resonance isotope or a radioactive isotope.

## Patentansprüche

1. Verwendung einer pharmazeutisch annehmbaren Zusammensetzung umfassend eine P-Selektinbindungskomponente, für die Herstellung eines Medikaments für die Prävention oder Behandlung eines Strahlungsschadens in einem Patienten, der gegenüber Strahlung ausgesetzt wird, durch Verabreichen der Zusammensetzung an den Patienten.

2. Verwendung nach Anspruch 1, wobei die Prävention oder die Behandlung des weiteren die Verabreichung eines ausgewählten Mittels an den Patienten umfaßt.

3. Verwendung nach Anspruch 1 oder 2, wobei der Patient ein Mensch ist.

4. Verwendung nach einem der vorangehenden Ansprüche, wobei die pharmazeutisch annehmbare Zusammensetzung an den Patienten in einer topischen, oralen oder parenteralen Formulierung verabreicht werden kann.

5. Verwendung nach einem der vorangehenden Ansprüche, wobei die Prävention oder Behandlung des weiteren die Veringerung oder die Blockierung der inflammatorischen Antwort umfaßt.

6. Verwendung nach einem der vorangehenden Ansprüche, wobei die 4-Selektinbindungskomponente die Bindung von 4-Selektin an einen inflammatorischen Zellrezeptor vorzugsweise einen Leukozytenrezeptor behindert.

7. Verwendung nach einem der vorangehenden Ansprüche, wobei die Bestrahlung aus der Radiotherapie stammt.

8. Verwendung nach einem der vorangehenden Ansprüche, wobei die Verabreichung von 15 Minuten bis 2 Stunden nach der Aussetzung gegenüber der Strahlung stattfindet.

9. Verwendung nach einem der Ansprüche 1 bis 7, wobei die Verabreichung innerhalb von 30 Minuten nach der Aussetzung gegenüber der Strahlung vorzugsweise innerhalb von 15 Minuten nach der Aussetzung gegenüber der Strahlung stattfindet.

10. Verwendung nach einem der vorangehenden Ansprüche, wobei die P-Selektinbindungskomponente ein Antikörper oder ein Fragment davon, ein Oligosaccharid, ein Polysaccharid, ein Glycolipid, ein Glycoprotein, eine Zelle oder ein Zellenligand ist.

11. Verwendung nach Anspruch 10, wobei die P-Selektinbindungskomponente ein Antikörper vorzugsweise ein monoklonaler Antikörper ist.

12. Verwendung nach Anspruch 10, wobei die P-Selektinbindungskomponente ein Fragment eines Antikörpers vorzugsweise ein scFv, ein Fv, eine Fab', ein Fab oder ein F(ab')₂ ist.

13. Verwendung nach Anspruch 10, wobei die P-Selektinbindungskomponente ein Oligosaccharid vorzugsweise ein PGSL, eine Glycyrrhizin, eine Carminsäure, ein Cylexin, ein Sialyl-Lewis-X/A-Oligosaccharid, ein Sialyl-Lewis-X/A-Immitator oder ein strukturelles Analog dazu ist.

14. Verwendung nach Anspruch 13, wobei das Sialyl-Lewis-X/A-Oligosaccharid ein Sialyl-Lewis-X; ein Sialyl-Lewis-A, ein Sialyl-Lewis-X/A, ein Sialyl-Lewis-Pentasaccharid, ein Sialyl-Lewis-Tetrasaccharid, ein sulfiertes Le-Pentasaccharid, ein sulfiertes Le-Tetrasaccharid, ein aminosubstituiertes sLe^{a} oder eine dimere Sialyl-Lewis-Verbindung, vorzugsweise eine Sialyl-Lewis^{x}, ein sLe^{x}, ein Neu5Acα2-3Galβ1-4 [Fucα1-3]GlcNAc, ein Sialyl-Lewis^{a}, ein sLe^{a} oder ein Neu5Acα2-3Galβ1-3 [Fucα1-4]GlcNAc ist.

15. Verwendung nach Anspruch 1, wobei das Polysaccharid ein Polylaktosamin ist.

16. Verwendung nach Anspruch 10, wobei das Glycoprotein ein Protein C, ein 150 kD, ein 230 kD oder ein 130 kD, wie durch Polyacrylamidgelelektrophorese gemessen, ist.

17. Verwendung nach einem der Ansprüche 10 bis 16, wobei die Zelle ein T-Lymphozyt, ein T-Leukozyt, ein PMN, ein Eosinophil oder eine NK-Zelle ist.

18. Verwendung nach einem der Ansprüche 10 bis 17, wobei das Oligosaccharid, das Polysaccharid oder das Glycolipid in einer Liposomzubereitung vorzugsweise in ein kationisches Liposom, ein DOTMA/DOPE, ein DOTMA oder ein DORIE formuliert ist.

19. Verwendung nach einem der Ansprüche 10 bis 17, wobei das Oligosaccharid, das Polysaccharid oder das Glycolipid in ein Protein- oder in einem Polypeptid-Träger formuliert ist.

20. Verwendung nach Anspruch 10, wobei der Zellligand aus einem Leukozyten, einer T-Zelle oder einem polymorphonukleären Neutrophil isoliert ist.

21. Verwendung einer Zusammensetzung umfassend eine P-Selektinbindungskomponente wirksam verbunden mit oder befestigt an einem ausgewählten Mittel für die Herstellung eines Medikaments für die Verwendung in einem Verfahren für die Verabreichung eines ausgewählten Mittels an die Vaskulatur einer Person, umfassend die P-Selektintranslokation zum Lumen der vaskulären Endothelzelle und Verabreichen der Zusammensetzung an die Tierperson.

22. Verwendung nach Anspruch 21, wobei die Verabreichung von 15 Minuten bis 2 Stunden nach der Induktion der P-Selektintranslokation stattfindet.

23. Verwendung nach Anspruch 21, wobei die Verabreichung innerhalb von 30 Minuten noch der Induktion der P-Selektintranslokation stattfindet.

24. Verwendung nach einem der vorangehenden Ansprüche, wobei die Verabreichung eine einzelne Dosis umfaßt.

25. Verwendung nach einem der Ansprüche 1 bis 23, wobei die Verabreichung eine Mehrfachdosis umfaßt.

26. Verwendung nach einem der Ansprüche 1 bis 23, wobei die Verabreichung die fortdauernde Verabreichung umfaßt.

27. Verwendung einer Zusammensetzung umfassend eine P-Selektinbindungskomponente, die wirksam mit einem ausgewählten Mittel verbunden ist, für die Herstellung eines Medikaments für die Verwendung in einem Verfahren für die Verabreichung eines ausgewählten Mittels an die krankheits-assoziierte Vaskulatur einer Person mit einer Erkrankung, die einen vaskulären Bestandteil aufweist, umfassend die Induktion der Translokation des P-Selektins zum Lumen der krankheits-assoziierten vaskularen Endothelzellen und die Verabreichung der Zusammensetzung an die Person.

28. Verwendung nach einem der Ansprüche 1 bis 27, wobei die Person ein Mensch ist.

29. Verwendung nach Anspruch 27 oder 28 soweit von Anspruch 27 abhängig, wobei die Erkrankung mit einer vaskulären Komponente ein solider Tumor, eine diabetische Retinopathie, eine vaskuläre Restenose, eine arteriovenöse Mißbildung (AVM= "Ateriovenous malformation"), ein Meningiom oder eine mit einer vaskulären Komponente assoziierte gutartige Erkrankung ist.

30. Verwendung nach einem der Ansprüche 21 bis 29, wobei die P-Selektintranslokation zum Lumen der krankheits-assoziierten vaskulären Endothelzellen durch eine ionisierende Bestrahlung induziert wird vorzugsweise eine γ-Strahlung oder eine Röntgenstrahlung; Hitze; oder ein Oxidanz vorzugsweise H₂O₂ oder O₂.

31. Verwendung nach Anspruch 27 oder einem davon abhängigen Anspruch des weiteren umfassend die Verabreichung einer pharmazeutisch annehmbaren Formulierung der Zusammensetzung eines zweiten Mittels wirksam assoziiert mit einem Selektinbindungmittel an die Person.

32. Verwendung nach Anspruch 31, wobei die Verabreichung eine parenterale Verabreichung, eine Injektion oder eine Insulation an eine Erkrankungsstelle oder eine vaskularisierte Tumorstelle ist.

33. Verwendung anch Anspruch 27 oder einem davon abhängigen Anspruch, wobei die P-Selektinbindungskomponente ein Antikörper, ein Fragment davon, ein Oligosaccharid, ein Glycolipid ein Glycoprotein oder eine Zelle ist.

34. Verwendung nach Anspruch 27 oder einem davon abhängigen Anspruch des weiteren umfassend, das Erhalten eines Tumor-infiltrierenden Lymphozyts aus der Person, das Behandeln des Tumor-infiltrierenden Lymphozyts mit einem zweiten Mittel und das Wiedereinführen des Tumor-infiltrierenden Lymphozyts.

35. Verwendung nach Anspruch 31 oder 32, wobei das zweite Mittel ein rekombinanter Vektor ist der in einen TIL eingefügt ist.

36. Verwendung nach Anspruch 31 oder 32, wobei das zweite Mittel ein thrombolytisches Mittel, vorzugsweise eine Streptokinase oder eine Urokinase ist.

37. Verwendung nach Anspruch 31 oder 32, wobei das zweite Mittel ein chemotherapeutisches Mittel, vorzugsweise ein Steroid, ein Anthracyclin, ein Vincaalkaloid, ein Antibiotikum, ein alkylierendes Mittel, ein Epipodophylltotoxin, ein Neocarzinostatin (NCS), ein Adriamycin oder ein di-Deoxycytidin ist.

38. Verwendung nach Anspruch 31 oder 32, wobei das zweite Mittel ein therapeutisches Polypeptid, vorzugsweise ein Interferon-α (IFN-α), ein Interferon-βγ (INF-βγ), eine Interleukin-12 (IL-12) oder eine Tumornekrosefaktor-α (TFN-α) ist.

39. Verwendung nach Anspruch 31 oder 32, wobei das zweite Mittel ein Toxin, vorzugsweise ein Pflanzen-, Pilz- oder Bakterien-abgeleitetes Toxin ist.

40. Verwendung nach 39, wobei das Toxin ein Gelonin, ein α-Sarvin, ein Aspergillin, ein Restriktocin, eine Ribonuklease, ein Diphteri-Toxin, ein *Pseudomonas* Exotoxin, ein bakterielles Endotoxin, eine Lipid A, eine Ricin A-Kette, eine Deglycosylierte-Ricin-A-Kette oder eine rekombinante Ricin-A-Kette ist.

41. Verwendung nach Anspruch 31 oder 32, wobei das zweite Mittel ein flurogenes, paramagnetisches oder radioaktives Ion ist.

42. Verwendung nach Anspruch 41, wobei das paramagnetische Ion Chrom (III), Mangan (II), Eisen (III), Eisen (II), Kobalt (II), Nickel (II), Kupfer (II), Neodym (III), Samarium (III), Yttrium (III), Gadolinium (III), Vanadium (II), Terbium (III), Dysprosium (III), Holmium (III) oder Erbium (III) ist.

43. Verwendung nach Anspruch 41, wobei das radioaktive Ion Iod¹²³, Technecium^{99m}, Indium¹¹¹, Rhenium¹⁸⁸, Rhenium¹⁸⁶, Kupfer⁶⁷, Iod¹³¹, Yttrium⁹⁰, Iod¹²⁵, Astat²¹¹, Gallium⁶⁷, Iridium¹⁹², Kobalt⁶⁰, Radium²²⁶, Gold¹⁹⁸, Cäsium¹³⁷ oder Phosphor³² ist.

44. Verwendung nach Anspruch 41, wobei das flurogene Mittel Gadolinium oder Renografm ist.

45. Verwendung nach Anspruch 31 oder 32, wobei das zweite Mittel ein rekombinanter Vektor ist, der einen Promotor umfaßt, welcher operativ mit einem Polynukleotid verknüpft ist, daß für ein therapeutisches Polynukleotid oder Polypeptid kodiert.

46. Verwendung nach Anspruch 45, wobei der Promotor ein durch ionisierende Strahlung induzierbarer Promotor vorzugsweise ein *Egr-1*-Promotor, ein *los*-Promotor, ein c-jun-Promotor oder ein TNF-α-Promotor ist.

47. Verwendung nach Anspruch 45, wobei der Promotor ein vaskulärer Endothelzellspezifischer Promotor bzw. ein *Egr-1-*Gen-Promotor, ein ICAM-1-Gen-Promotor oder E-Selektin-Promotor ist.

48. Verwendung nach einem der Ansprüche 45 bis 46, wobei das therapeutische Polypeptid ein p53, ein p16, ein Rb, ein WT1, ein IFN-α, ein IFN-βγ, ein IL-12, ein TNF-α, eine Thymidinkinase (tk) oder eine Cytosindeaminase ist.

49. Verwendung nach einem der Ansprüche 45 bis 48, wobei der rekombinante Vektor ein Retrovirus, ein AAV, ein HSV-1, ein HPV, ein Vaccinia-, ein Adeno-assoziiertes Virus oder eine Adenovirus ist.

50. Verwendung nach einem der Ansprüche 45 bis 49, wobei die Erkrankung eine gutartige hyperproliferative Erkrankung ist.

51. Verwendung nach einem der Ansprüche 45 bis 49, wobei die Erkrankung ein bösartiger Tumor ist.

52. Verwendung nach einem der Ansprüche 45 bis 51, des weiteren umfassend eine zusätzliche Dosis ionisierender Strahlung nach der Verabreichung des ausgewählten Mittels.

53. Verwendung nach Anspruch 52 in soweit er von Anspruch 45 abhängig ist, wobei das ausgewählte Mittel ein kombinanter Vektor ist, der wirksam mit einer P-Selektinkomponente assoziiert ist, wobei der Vektor einen durch ionisierende Strahlung induzierbaren Promotor umfaßt, der operativ mit einem Polynukleotid verknüpft ist, das für ein therapeutisches Polynukleotid oder Polypeptid kodiert.

54. Verwendung einer Zusammensetzung umfassend eine P-Selektinbindungskomponente, die wirksam mit einem nachweisbaren Marker assoziiert ist, für die Herstellung eines Medikaments für die Verwendung in einem Verfahren zur Bestimmung der Aussetzung eines Tiers oder eines Patienten gegenüber Strahlung, das das Verabreichen einer wirksamen Menge der Zusammensetzung an das Tier oder den Patienten; das Aussetzen des Tiers oder des Patienten gegenüber einer Vorrichtung, die die nachweisbare Markierung nachweist, und das Bestimmen der Menge der P-Selektintranslokation in das Lumen der vaskulären Endothezellen einer bestrahlten Stelle des Tiers oder des Patienten umfaßt , wobei eine Erhöhung der P-Selektinmenge im Vergleich zur Menge des P-Selektins an dieser Stelle in einem nicht bestrahlten Tier oder Patienten, ein Hinweis auf die Aussetzung gegenüber der Strahlung ist.

55. Verwendung nach Anspruch 54, wobei die P-Selektinzielkomponente ein Glycyrrhizin, eine Carminsäure, ein Cylexin, ein Sialyl-Lewis-X, ein Sialyl-Lewis-A, eine Sialyl-Lewis-X/A, ein Sialyl-Lewis-X/A-Immitator oder ein Antikörper, der immunologisch an P-Selektin bindet, ist.

56. Verwendung nach Anspruch 55, wobei die nachweisbare Markierung ein kernmagnetishes-spinresonanz Isotop oder ein radioaktives Isotop ist.

## Revendications

1. Utilisation d'une composition pharmaceutiquement acceptable comportant un composant de liaison de P-sélectine, pour la fabrication d'un médicament destiné à prévenir ou traiter une lésion par rayonnement chez un patient animal exposé à un rayonnement en administrant la composition audit patient.

2. Utilisation selon la revendication 1, dans laquelle la prévention ou le traitement comporte de plus l'administration audit patient d'un agent sélectionné.

3. Utilisation selon la revendication 1 ou 2, dans laquelle ledit patient animal est un être humain.

4. Utilisation selon l'une quelconque des revendications précédentes, dans laquelle ladite composition pharmaceutiquement acceptable peut être administrée audit patient animal dans une formulation topique, orale ou parentérale.

5. Utilisation selon l'une quelconque des revendications précédentes, dans laquelle la prévention ou le traitement comporte de plus la réduction ou le blocage de la réponse inflammatoire.

6. Utilisation selon l'une quelconque des revendications précédentes, dans laquelle ledit composant de liaison de P-sélectine inhibe l'interaction de P-sélectine avec un récepteur de cellule inflammatoire, de préférence un récepteur de leucocyte.

7. Utilisation selon l'une quelconque des revendications précédentes, dans laquelle ledit rayonnement provient d'une radiothérapie.

8. Utilisation selon l'une quelconque des revendications précédentes, dans laquelle l'administration a lieu entre 15 minutes et 2 heures après exposition à un rayonnement.

9. Utilisation selon l'une quelconque des revendications 1 à 7, dans laquelle ladite administration a lieu dans les 30 minutes d'une exposition à un rayonnement, de préférence dans les 15 minutes d'exposition à un rayonnement.

10. Utilisation selon l'une quelconque des revendications précédentes, dans laquelle ledit composant de liaison de P-sélectine est un anticorps ou un fragment de celui-ci, un oligosaccharide, un polysaccharide, un glycolipide, une glycoprotéine, une cellule ou un ligand cellulaire.

11. Utilisation selon la revendication 10, dans laquelle ledit composant de liaison de P-sélectine est un anticorps, de préférence un anticorps monoclonal.

12. Utilisation selon la revendication 10, dans laquelle ledit composant de liaison de P-sélectine est un fragment d'un anticorps, de préférence un scFv, un Fv, un Fab', un Fab, ou un F(ab')₂.

13. Utilisation selon la revendication 10, dans laquelle ledit composant de liaison de P-sélectine est un oligosaccharide, de préférence un PGSL, une glycyrrhizine, un acide carminique, un cylexin, un oligosaccharide de sialyle Lewis X/A, une imitation de sialyle Lewis X/A ou un analogue structurel de ceux-ci.

14. Utilisation selon la revendication 13, dans laquelle ledit oligosaccharide de sialyle Lewis X/A est un sialyle Lewis X, un sialyle Lewis A, un sialyle Lewis X/A, un pentasaccharide de sialyle Lewis, un tétrasaccharide de sialyle Lewis, un pentasaccharide de Le sulfaté, un tétrasaccharide Le sulfaté, un sLe^{a} substitué par un groupe amino ou un composé dimère de sialyle Lewis, de préférence un sialyle Lewis^{x}, un sLe^{x}, un Neu5Acα2-3Galβ1-4[Fucα1-3]GlcNAc, un syalyle Lewis^{a}, un sLe^{a}, ou un Neu5cα2-3Galβ1-3[Fucα1-4]GlcNAc.

15. Utilisation selon la revendication 1, dans laquelle ledit polysaccharide est une polylactosamine.

16. Utilisation selon la revendication 10, dans laquelle ladite glycoprotéine est une Protéine C, 150 kD, 230 kD ou 130 kD, comme mesuré par électrophorèse sur gel de polyacrylamide.

17. Utilisation selon l'une quelconque des revendications 10 à 16, dans laquelle ladite cellule est un lymphocyte T, un leucocyte T, un PMN, un éosinophile ou une cellule NK.

18. Utilisation selon l'une quelconque des revendications 10 à 17, dans laquelle ledit oligosaccharide, polysaccharide ou glycolipide est formulé dans une préparation de liposome, de préférence un liposome cationique, DOTMA/DOPE, DOTMA ou DORIE.

19. Utilisation selon l'une quelconque des revendications 10 à 17, dans laquelle ledit oligosaccharide, polysaccharide ou glycolipide est formulé dans une protéine ou un support polypeptidique.

20. Utilisation selon la revendication 10, dans laquelle ledit ligand cellulaire est isolé d'un leucocyte, d'une cellule T ou d'un neutrophile poly-morphonucléaire.

21. Utilisation d'une composition comportant un composant de liaison de P-sélectine associé de manière opérationnelle à un agent sélectionné, ou fixé à celui-ci, pour la fabrication d'un médicament destiné à être utilisé dans un procédé pour délivrer un agent sélectionné dans la vascularisation d'un sujet animal comportant l'induction d'une translocation de P-sélectine dans le lumen d'une cellule endothéliale vasculaire et l'administration de la composition audit sujet animal.

22. Utilisation selon la revendication 21, dans laquelle ladite administration a lieu entre 15 minutes et 2 heures après ladite induction de translocation de P-sélectine.

23. Utilisation selon la revendication 21, dans laquelle ladite administration a lieu dans les 30 minutes de ladite induction de translocation de P-sélectine.

24. Utilisation selon l'une quelconque des revendications précédentes, dans laquelle ladite administration comporte une dose unique.

25. Utilisation selon l'une quelconque des revendications 1 à 23, dans laquelle ladite administration comporte une dose multiple.

26. Utilisation selon l'une quelconque des revendications 1 à 23, dans laquelle ladite administration comporte une administration continue.

27. Utilisation d'une composition comportant un composé de liaison de P-sélectine associé de manière opérationnelle à un agent sélectionné, pour la fabrication d'un médicament destiné à être utilisé dans un procédé pour délivrer un agent sélectionné dans la vascularisation associée à une maladie d'un sujet animal ayant une maladie qui a un composant vasculaire, comportant l'induction d'une translocation de P-sélectine dans le lumen de cellules endothéliales vasculaires associées à la maladie et l'administration de la composition audit sujet animal.

28. Utilisation selon l'une quelconque des revendications 21 à 27, dans laquelle ledit sujet animal est un être humain.

29. Utilisation selon la revendication 27 ou 28 lorsqu'elle est dépendante de la revendication 27, dans laquelle ladite maladie ayant un composant vasculaire est une tumeur solide, une rétinopathie diabétique, une resténose vasculaire, une malformation artérioveineuse (AVM), un méningiome ou une maladie bénigne associée à un composant vasculaire.

30. Utilisation selon l'une quelconque des revendications 21 à 29, dans laquelle ladite translocation de P-sélectine dans ledit lumen de cellules endothéliales vasculaires associées à une maladie est induite par : un rayonnement ionisant, de préférence une irradiation γ ou un rayon x, une chaleur, ou un oxydant, de préférence H₂O₂ ou O₂.

31. Utilisation selon la revendication 27 ou l'une quelconque des revendications dépendantes de celle-ci, comportant de plus l'administration audit sujet animal dans une formulation pharmaceutiquement acceptable d'une composition constituée d'un second agent associé de manière opérationnelle à un agent de liaison de sélectine.

32. Utilisation selon la revendication 31, dans laquelle ladite administration est une administration parentérale, une injection ou une isolation dans un site malade ou un site de tumeur vascularisé.

33. Utilisation selon la revendication 27 ou l'une quelconque des revendications dépendantes de celle-ci, dans laquelle ledit composé de liaison de P-sélectine est un anticorps ou un fragment de celui-ci, un oligosaccharide, un glycolipide, une glycoprotéine ou une cellule.

34. Utilisation selon la revendication 27, ou l'une quelconque des revendications dépendantes de celle-ci, comportant de plus l'obtention d'un lymphocyte d'infiltration de tumeur à partir d'un sujet animal, le traitement dudit lymphocyte d'infiltration de tumeur à l'aide d'un second agent, et la réintroduction dudit lymphocyte d'infiltration de tumeur.

35. Utilisation selon la revendication 31 ou 32, dans laquelle ledit second agent est un vecteur recombinant qui est inséré dans TIL.

36. Utilisation selon la revendication 31 ou 32, dans laquelle ledit second agent est un agent thrombolytique, de préférence une streptokinase ou une urokinase.

37. Utilisation selon la revendication 31 ou 32, dans laquelle ledit second agent est un agent chimiothérapeutique, de préférence un stéroïde, une anthracycline, un vinca-alcaloïde, un antibiotique, un agent alkylant, une épipodophyllotoxine, une néocarzinostatine (NCS), une adriamycine ou une didésoxycytidine.

38. Utilisation selon la revendication 31 ou 32, dans laquelle ledit second agent est un polypeptide thérapeutique, de préférence un interféron-α (IFN-α), un interféron-βγ (IFN-βγ), une interleukine-12 (IL-12) ou un facteur de nécrose tumorale-α (TFN-α).

39. Utilisation selon la revendication 31 ou 32, dans laquelle ledit second agent est une toxine, de préférence une toxine dérivée d'un végétal, d'un champignon ou d'une bactérie.

40. Utilisation selon la revendication 39, dans laquelle ladite toxine est une gélonine, une α-sarvine, une aspergilline, une restrictocine, une ribonucléase, une toxine diphtérique, une exotoxine de *Pseudomonas,* une endotoxine bactérienne, un lipide A, une chaîne de ricine A, une chaîne de ricine A déglycosylée ou une chaîne de ricine A recombinante.

41. Utilisation selon la revendication 31 ou 32, dans laquelle ledit second agent est un ion fluorogénique, paramagnétique ou radioactif.

42. Utilisation selon la revendication 41, dans laquelle l'ion paramagnétique est le chrome (III), manganèse (II), fer (III), fer (II), cobalt (II), nickel (II), cuivre (II), néodyme (III), samarium (III), ytterbium (III), gadolinium (III), vanadium (II), terbium (III), dysprosium (III), holmium (III) ou erbium (III).

43. Utilisation selon la revendication 41, dans laquelle l'ion radioactif est l'iode¹²³, technicium^{99m}, indium¹¹¹, rhénium¹⁸⁸, rhénium¹⁸⁶, cuivre⁶⁷, iode¹³¹, yttrium⁹⁰, iode¹²⁵, astatine²¹¹, gallium⁶⁷, iridium¹⁹², cobalt⁶⁰, radium²²⁶, or¹⁹⁸, césium¹³⁷, ou phosphore³².

44. Utilisation selon la revendication 41, dans laquelle les agents fluorogéniques sont le gadolinium ou rénographine.

45. Utilisation selon la revendication 31 ou 32, dans laquelle ledit second agent est un vecteur recombinant comportant un promoteur lié de manière opérationnelle à un polynucléotide codant pour un polynucléotide ou un polypeptide thérapeutique.

46. Utilisation selon la revendication 45, dans laquelle ledit promoteur est un promoteur inductible par rayonnement ionisant, de préférence un promoteur *Egr-1,* un promoteur *los,* un promoteur c-jun ou un promoteur TNF-α.

47. Utilisation selon la revendication 45, dans laquelle le promoteur est un promoteur spécifique à des cellules endothéliales vasculaires, de préférence un promoteur du gène *Egr-1,* un promoteur du gène ICAM-1 ou un promoteur du gène de E-sélectine.

48. Utilisation selon l'une quelconque des revendications 45 à 47, dans laquelle ledit polypeptide thérapeutique est p53, p16, Rb, WT1, IFN-α, IFN-βγ, IL-12, TNF-α, thymidine kinase (tk) ou cytosine désaminase.

49. Utilisation selon l'une quelconque des revendications 45 à 48, dans laquelle le vecteur recombinant est un rétrovirus, AAV, HSV-1, HPV, une vaccine, un virus adéno-associé ou un adénovirus.

50. Utilisation selon l'une quelconque des revendications 45 à 49, dans laquelle la maladie est une maladie hyperproliférative bénigne.

51. Utilisation selon l'une quelconque des revendications 45 à 49, dans laquelle la maladie est une malignité.

52. Utilisation selon l'une quelconque des revendications 45 à 51, comportant de plus la fourniture d'une dose supplémentaire de rayonnement ionisant après libération dudit agent sélectionné.

53. Utilisation selon la revendication 52, lorsque dépendante de la revendication 45, dans laquelle ledit agent sélectionné est un vecteur recombinant associé de manière opérationnelle à un composant de ciblage de P-sélectine, ledit vecteur comportant un promoteur inductible par rayonnement ionisant lié de manière opérationnelle à un polynucléotide codant pour un polynucléotide ou polypeptide thérapeutique.

54. Utilisation d'une composition comportant un composant de liaison de P-sélectine associé de manière opérationnelle à un marqueur détectable pour la préparation d'un réactif destiné à être utilisé dans un procédé pour déterminer l'exposition à un rayonnement d'un animal ou patient comportant l'administration d'une quantité efficace de la composition à un animal ou patient, l'exposition de l'animal ou du patient à un dispositif qui détecte ledit marqueur détectable et la détermination du niveau de translocation de P-sélectine du lumen de cellules endothéliales vasculaires d'un site irradié de l'animal ou patient, dans laquelle l'augmentation du niveau de P-sélectine, par comparaison au niveau de P-sélectine au niveau dudit site chez un animal ou patient non-irradié, est représentative d'une exposition à un rayonnement.

55. Utilisation selon la revendication 54, dans laquelle ledit composant de ciblage de P-sélectine est une glycyrrhizine, un acide carminique, un cylexin, un sialyle Lewis X, un syalyle Lewis A, un sialyle Lewis X/A, des imitations de sialyle Lewis X/A ou un anticorps qui se lie de manière immunologique à la P-sélectine.

56. Utilisation selon la revendication 55, dans laquelle ledit marqueur détectable est un isotope détectable par résonance magnétique nucléaire ou un isotope radioactif.
